# FASCICULE DE BREVET EUROPEEN

(11) **EP 3 189 060 B1**
(45) Date de publication et mention de la délivrance du brevet: **20.06.2018**
(21) Numéro de dépôt: 15757283.5
(22) Date de dépôt: 02.09.2015
(51) Int. Cl.: C07D 498/08, C07D 519/00, A61K 31/527, A61P 35/00, C07D 513/08

(54) **DERIVES DE N-ARYL-2-AMINO-4-ARYL-PYRIMIDINES POLYETHERS MACROCYCLIQUES COMME INHIBITEURS DE LA FTL3 AND JAK**
DERIVATE VON MAKROCYCLISCHEN N-ARYL-2-AMINO-4-ARYL-PYRIMIDINPOLYETHERN ALS INHIBITOREN VON FTL3 UND JAK
DERIVATIVES OF MACROCYCLIC N-ARYL-2-AMINO-4-ARYL-PYRIMIDINE POLYETHERS AS INHIBITORS OF FTL3 AND JAK

(30) Priorité: 02.09.2014 FR 1458203
(43) Date de publication de la demande: 12.07.2017
(73) Titulaire: Pierre Fabre Médicament, 92100 Boulogne-Billancourt (FR)
(72) Inventeur: KALOUN, El Bachir, F-31120 Roquettes (FR); GRISONI, Serge, F-31120 Portet Sur Garonne (FR); GOMES, Bruno, F-31470 Saint Lys (FR); SCHMITT, Philippe, F-31560 Nailloux (FR)
(74) Mandataire: Regimbeau
(86) Numéro de dépôt international: PCT/EP2015/070066
(87) Numéro de publication internationale: WO 2016/034634

(56) Documents cités:
- WO-A1-2007/058627
- ANTHONY D. WILLIAM ET AL: "Discovery of the Macrocycle 11-(2-Pyrrolidin-1-yl-ethoxy)-14,19-dioxa- 5,7,26-triaza-tetracyclo[19.3.1.1(2,6).1(8 ,12)]heptacosa-1(25),2(26),3,5,8,10,12(27) ,16,21,23-decaene (SB1518), a Potent Janus Kinase 2/Fms-Like Tyrosine Kinase-3 (JAK2/FLT3) Inhibitor for the Treatment of Myelofibrosis and Lympho", JOURNAL OF MEDICINAL CHEMISTRY, vol. 54, no. 13, 14 juillet 2011 (2011-07-14), pages 4638-4658, XP055163145, ISSN: 0022-2623, DOI: 10.1021/jm200326p
- ANTHONY D. WILLIAM ET AL: "Discovery of Kinase Spectrum Selective Macrocycle (16 E )-14-Methyl-20-oxa-5,7,14,26-tetraazatetra cyclo[19.3.1.1(2,6).1(8,12)]heptacosa-1(25 ),2(26),3,5,8(27),9,11,16,21,23-decaene (SB1317/TG02), a Potent Inhibitor of Cyclin Dependent Kinases (CDKs), Janus Kinase 2 (JAK2), and Fms-like Tyrosine Kinas", JOURNAL OF MEDICINAL CHEMISTRY, vol. 55, no. 1, 12 janvier 2012 (2012-01-12), pages 169-196, XP055071583, ISSN: 0022-2623, DOI: 10.1021/jm201112g
- ANDERS POULSEN ET AL: "Structure-based design of oxygen-linked macrocyclic kinase inhibitors: discovery of SB1518 and SB1578, potent inhibitors of Janus kinase 2 (JAK2) and Fms-like tyrosine kinase-3 (FLT3)", JOURNAL OF COMPUTER-AIDED MOLECULAR DESIGN, KLUWER ACADEMIC PUBLISHERS, DO, vol. 26, no. 4, 22 avril 2012 (2012-04-22) , pages 437-450, XP035053361, ISSN: 1573-4951, DOI: 10.1007/S10822-012-9572-Z

## Description

La présente invention concerne des dérivés de N-aryl-2-amino-4-aryl-pyrimidines polyéthers macrocycliques, ainsi que leur utilisation thérapeutique, notamment dans le traitement du cancer, et leurs procédés de synthèse.

Les mutations des récepteurs à activité tyrosine kinase jouent un rôle crucial dans la pathogénèse de nombreux cancers. Par exemple, le récepteur de FLT3 est fréquemment muté dans la leucémie aiguë myéloïde (dans environ 30% des cas) (Gilliland et al. 2002 Blood 100 : 1532-1542). Les mutations qui ont pour conséquence une augmentation de l'activité kinase du récepteur de FLT3 rendent la cellule tumorale relativement dépendante de ce récepteur pour sa prolifération et sa survie, ce qui fait donc de ce récepteur muté une cible pertinente en oncologie. Trois types de mutations activatrices de FLT3 sont identifiées aujourd'hui dans la leucémie aiguë myéloïde (LAM) : la duplication interne en tandem (FLT3-ITD), qui est détectée dans environ 20% des cas, les mutations ponctuelles du domaine catalytique du récepteur, qui constituent 6-8% des cas, et les mutations ponctuelles des domaines juxtamembranaires et extracellulaires, qui sont rares (2%) (Kayser et al. 2014 Leukemia & Lymphoma 55 : 243-255).

Les nouvelles générations d'inhibiteurs de FLT3 en cours d'évaluation clinique ont montré des résultats encourageants pour le traitement des LAM exprimant une forme mutée de FLT3. Toutefois, la plupart des réponses des patients sont encore insuffisantes car incomplètes et transitoires, entraînant un taux de rechutes qui reste trop élevé. Les causes de ces rechutes / résistances sont multiples. Elles peuvent mettre en jeu des mutations secondaires du récepteur FLT3 ou l'activation de voies de signalisation alternatives qui conduit à une réactivation en aval de la voie du récepteur FLT3. D'autre part, alors que les cellules leucémiques circulant dans le sang du patient peuvent être relativement sensibles aux inhibiteurs de tyrosine kinase, les cellules leucémiques nichées dans la moelle du patient sont plus réfractaires au traitement, suggérant un rôle de la moelle osseuse (micro-environnement) dans la résistance thérapeutique (Weisberg et al. 2012 Leukemia 26 : 2233-2244). Ce micro-environnement stromal des cellules leucémiques, constitué par la moelle osseuse, protègerait les cellules leucémiques des effets des inhibiteurs de tyrosine kinase. La voie de signalisation IL-6/JAK/STAT fait partie des voies majeures qui contribueraient à conférer un avantage de survie aux cellules leucémiques exprimant une forme mutée de FLT3. En outre, il a été montré que la combinaison thérapeutique d'un inhibiteur de JAK et d'un inhibiteur de FLT3 permettait d'augmenter les effets de l'inhibition de FLT3 et de surmonter la résistance induite par le micro-environnement stromal (Weisberg et al. op.cit.). D'une manière générale, la famille des kinases JAK est décrite comme jouant un rôle important dans le contrôle de la prolifération de la survie cellulaire et de l'apoptose. Ces kinases JAK font l'objet d'altérations génétiques associées à de nombreuses pathologies tumorales, incluant les hémopathies malignes. WO2007/058627 décrit des composés macrocycliques et leur utilisation pour le traitement du cancer. La présente invention a permis de manière surprenante d'identifier des composés présentant une double activité à la fois en tant qu'inhibiteur de JAK et de FLT3. Ces composés présentent en outre une remarquable activité.

La présente invention concerne des dérivés de N-aryl-2-amino-4-aryl-pyrimidines polyéthers macrocycliques, ainsi que leur utilisation thérapeutique, notamment dans le traitement du cancer, et leur procédé de synthèse.

La présente invention a donc plus particulièrement pour objet un composé de formule générale **(I)** suivante : 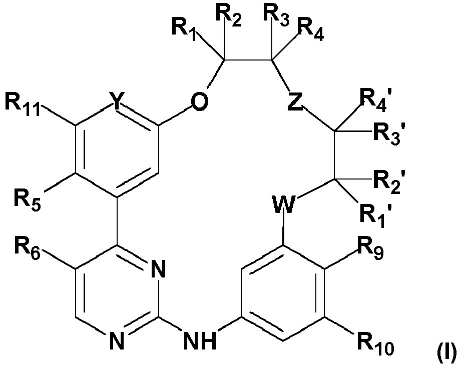 ou un sel et/ou un solvate pharmaceutiquement acceptable de celui-ci, dans laquelle :
- **W** représente un atome d'oxygène ou de soufre,
- **Y** représente un atome d'azote ou un groupe C**Ry** avec **Ry** représentant un atome d'hydrogène, un atome d'halogène, un groupe (C₁-C₆)alkyle, (C₁-C₆)halogénoalkyle, (C₁-C₆)alcoxy, (C₁-C₆)halogénoalcoxy, OH, CN, NO₂, N**R₁₂R₁₃**, CO₂H ou CO₂((C₁-C₆)alkyle),
- **Z** représente un groupe (C**R_{Q1}R_{Q2}**)ₙ**Q**(C**R_{Q3}R_{Q4}**)ₘ, avec **n** et **m** représentant, indépendamment l'un de l'autre, un nombre entier compris entre 0 et 3,
- **Q** représente O, S, S(O) ou S(O)₂,
- **R_{Q1}**, **R_{Q2}**, **R_{Q3}** et **R_{Q4}** représentent, indépendamment les uns des autres, un atome d'hydrogène ou un groupe (C₁-C₆)alkyle,
- **R₁**, **R₂**, **R₁'** et **R₂'** représentent, indépendamment les uns des autres, un atome d'hydrogène ou un groupe (C₁-C₆)alkyle,
- **R₃**, **R₄**, **R₃'** et **R₄'** représentent, indépendamment les uns des autres, un atome d'hydrogène, un groupe (C₁-C₆)alkyle ou OH ou **R₃** et **R₄** et/ou **R₃'** et **R₄'** forment ensemble, avec l'atome de carbone qui les porte, un carbocycle ou hétérocycle monocyclique éventuellement substitué,
- **R₅** et **R₆** représentent, indépendamment l'un de l'autre, un atome d'hydrogène, un atome d'halogène, un groupe (C₁-C₆)alkyle, (C₁-C₆)halogénoalkyle, (C₁-C₆)alcoxy, (C₁-C₆)halogénoalcoxy, (C₁-C₆)thioalcoxy, (C₁-C₆)halogénothioalcoxy, OH, SH, CN, NO₂, ou **NR₇R₈**,
- **R₉** et **R₁₀** représentent, indépendamment l'un de l'autre, un atome d'hydrogène, un atome d'halogène, un groupe (C₁-C₆)alkyle éventuellement substitué, (C₂-C₆)alcényle éventuellement substitué, (C₂-C₆)alcynyle éventuellement substitué, (C₁-C₆)alcoxy éventuellement substitué, (C₁-C₆)thioalcoxy éventuellement substitué, CN, NO₂, N**R₁₄R₁₅**, OH, SH, CO₂**R₅₄**, CON**R₅₅R₅₆**, un carbocycle éventuellement substitué ou un hétérocycle éventuellement substitué,
- **R₁₁** représente un atome d'hydrogène, un atome d'halogène, ou un groupe (C₁-C₆)alkyle, (C₁-C₆)halogénoalkyle, (C₁-C₆)alcoxy ou (C₁-C₆)halogénoalcoxy, et
- **R₇**, **R₈**, **R₁₂**, **R₁₃**, **R₁₄**, **R_{15,} R₅₄**, **R₅₅** et **R₅₆** représentent, indépendamment les uns des autres, un atome d'hydrogène ou un groupe (C₁-C₆)alkyle éventuellement substitué, (C₂-C₆)alcényle éventuellement substitué, ou (C₂-C₆)alcynyle éventuellement substitué, ou **R₇** et **R₈**, **R₁₂** et **R₁₃, R₁₄** et **R₁₅** et/ou **R₅₅** et **R₅₆**, indépendamment les uns des autres, forment avec l'atome d'azote qui les portent un hétérocycle azoté éventuellement substitué.

Les stéréoisomères des composés de formule générale (I) font également partie de la présente invention, ainsi que leurs mélanges, notamment sous forme d'un mélange racémique.

Les tautomères des composés de formule générale (I) font également partie de la présente invention.

Par « stéréoisomère », on entend, au sens de la présente invention, un isomère géométrique (ou isomère de configuration) ou un isomère optique.

Les isomères géométriques résultent de la position différente des substituants sur une double liaison qui peut avoir alors une configuration Z ou E, encore appelée cis ou *trans.*

Les isomères optiques résultent notamment de la position différente dans l'espace des substituants sur un atome de carbone comprenant 4 substituants différents. Cet atome de carbone constitue alors un centre chiral ou asymétrique. Les isomères optiques comprennent les diastéréoisomères et les énantiomères. Les isomères optiques qui sont des images l'un de l'autre dans un miroir mais non superposables sont désignés par « énantiomères ». Les isomères optiques qui ne sont pas des images superposables l'un de l'autre dans un miroir sont désignés par « diastéréoisomères ».

Un mélange contenant des quantités égales de deux formes énantiomères individuelles de chiralité opposée est désigné par « mélange racémique ».

Par « tautomère », on entend, au sens de la présente invention, un isomère de constitution du composé obtenu par prototropie, c'est-à-dire par migration d'un atome d'hydrogène et changement de localisation d'une double liaison. Les différents tautomères d'un composé sont généralement interconvertibles et présents en équilibre en solution, dans des proportions qui peuvent varier selon le solvant utilisé, la température ou encore le pH.

Dans la présente invention, on entend désigner par « pharmaceutiquement acceptable » ce qui est utile dans la préparation d'une composition pharmaceutique qui est généralement sûr, non toxique et ni biologiquement ni autrement non souhaitable et qui est acceptable pour une utilisation vétérinaire de même que pharmaceutique humaine.

On entend désigner par « sel et/ou solvate pharmaceutiquement acceptable » d'un composé, un sel et/ou solvate qui est pharmaceutiquement acceptable, comme défini ici, et qui possède l'activité pharmacologique souhaitée du composé parent.

Les sels pharmaceutiquement acceptables des composés de la présente invention comprennent les sels non toxiques conventionnels des composés de l'invention tels que ceux formés à partir d'acides organiques ou inorganiques ou de bases organiques ou inorganiques. A titre d'exemple on peut citer les sels dérivés d'acides inorganiques comme les acides chlorhydrique, bromhydrique, phosphorique, sulfurique, et ceux dérivés d'acides organiques comme les acides acétique, trifluoroacétique, propionique, succinique, fumarique, malique, tartrique, citrique, ascorbique, maléique, glutamique, benzoïque, salicylique, toluènesulfonique, méthanesulfonique, stéarique, lactique. A titre d'exemple on peut citer les sels dérivés de bases inorganiques comme la soude, la potasse ou l'hydroxyde de calcium et les sels dérivés de bases organiques comme la lysine ou l'arginine.

Ces sels peuvent être synthétisés à partir des composés de l'invention contenant une partie basique ou acide et les acides ou bases correspondants selon les méthodes chimiques conventionnelles.

Les solvates pharmaceutiquement acceptables des composés de la présente invention comprennent les solvates conventionnels tels que ceux formés lors de la dernière étape de préparation des composés de l'invention du fait de la présence de solvants. A titre d'exemple on peut citer les solvates dus à la présence d'eau (hydrates) ou d'éthanol.

Le terme « halogène » représente un fluor, chlore, brome ou iode.

Par groupement « (C₁-C₆)alkyle », on entend, au sens de la présente invention, une chaîne hydrocarbonée saturée, linéaire ou ramifiée, comportant 1 à 6, notamment 1 à 4, atomes de carbone. A titre d'exemple, on peut citer les groupes méthyle, éthyle, propyle, isopropyle, butyle, isobutyle, sec-butyle, *tert*-butyle, pentyle ou encore hexyle.

Par groupement « (C₂-C₆)alcényle », on entend, au sens de la présente invention, une chaîne hydrocarbonée, linéaire ou ramifiée, comportant au moins une double liaison et comportant 2 à 6, notamment 2 à 4, atomes de carbone. A titre d'exemple, on peut citer les groupes éthényle ou allyle.

Par groupement « (C₂-C₆)alcynyle », on entend, au sens de la présente invention, une chaîne hydrocarbonée, linéaire ou ramifiée, comportant au moins une triple liaison et comportant 2 à 6, notamment 2 à 4, atomes de carbone. A titre d'exemple, on peut citer les groupes éthynyle ou propynyle.

Par « (C₁-C₆)halogénoalkyle », on entend, au sens de la présente invention, un groupe (C₁-C₆)alkyle, tel que défini ci-dessus, dans lequel un ou plusieurs atomes d'hydrogène ont été remplacés chacun par un atome d'halogène tel que défini ci-dessus. Il peut s'agir en particulier d'un groupe CF₃.

Par groupement « (C₁-C₆)alcoxy », on entend, au sens de la présente invention, un groupe (C₁-C₆)alkyle tel que défini ci-dessus, lié au reste de la molécule par l'intermédiaire d'un atome d'oxygène. A titre d'exemple, on peut citer les groupes méthoxy, éthoxy, propoxy, isopropoxy, butoxy ou encore *tert*-butoxy.

Par « (C₁-C₆)halogénoalcoxy », on entend, au sens de la présente invention, un groupe (C₁-C₆)halogénoalkyle, tel que défini ci-dessus, lié au reste de la molécule par l'intermédiaire d'un atome d'oxygène. Il peut s'agir notamment d'un groupe OCF₃.

Par groupement « (C₁-C₆)thioalcoxy », on entend, au sens de la présente invention, un groupe (C₁-C₆)alkyle tel que défini ci-dessus, lié au reste de la molécule par l'intermédiaire d'un atome de soufre. A titre d'exemple, on peut citer les groupes thiométhoxy, thioéthoxy, thiopropoxy, thio-isopropoxy, thiobutoxy ou encore thio*-tert-*butoxy.

Par « (C₁-C₆)halogénothioalcoxy », on entend, au sens de la présente invention, un groupe (C₁-C₆)halogénoalkyle, tel que défini ci-dessus, lié au reste de la molécule par l'intermédiaire d'un atome de soufre. Il peut s'agir notamment d'un groupe SCF₃.

Par groupement « (C₁-C₆)alkyl-amino », on entend, au sens de la présente invention, un groupe (C₁-C₆)alkyle, tel que défini ci-dessus, lié au reste de la molécule par l'intermédiaire d'un groupe NH. A titre d'exemple, on peut citer les groupes méthylamino, éthylamino, propylamino, ou encore butylamino.

Par groupement « di((C₁-C₆)alkyl)amino », on entend, au sens de la présente invention, un groupe (C₁-C₆)alkyle, tel que défini ci-dessus, lié au reste de la molécule par l'intermédiaire d'un groupe NAlk avec Alk représentant un groupe (C₁-C₆)alkyle tel que défini ci-dessus. A titre d'exemple, on peut citer les groupes diméthylamino, diéthylamino, méthyléthylamino, etc.

Par « carbocycle », on entend, au sens de la présente invention, un système monocyclique ou polycyclique hydrocarboné, saturé, insaturé ou aromatique, comprenant de 3 à 12 atomes de carbone Le système polycyclique comprend au moins 2, notamment 2 ou 3, cycles accolés ou pontés. Chaque cycle du système monocyclique ou polycyclique comprend avantageusement 3 à 8, notamment 4 à 7, en particulier 5 ou 6, atomes de carbone. A titre d'exemple on peut citer un groupe adamantyle, cyclohexyle, cyclopentyle, cyclobutyle, cyclopropyle, cyclohexényle, phényle, naphtyle.

Par « aryle », on entend, au sens de la présente invention, un groupement hydrocarboné aromatique, comportant de préférence de 6 à 10 atomes de carbone, et comprenant un ou plusieurs cycles accolés, comme par exemple un groupement phényle ou naphtyle. Avantageusement, il s'agit du phényle.

Par « aryl-(C₁-C₆)alkyle », on entend, au sens de la présente invention, un groupe aryle tel que défini ci-dessus, lié au reste de la molécule par l'intermédiaire d'une chaîne (C₁-C₆)alkyle telle que définie ci-dessus. A titre d'exemple, on peut citer le groupe benzyle.

Par « (C₁-C₆)alkyl-aryle », on entend, au sens de la présente invention, un groupe (C₁-C₆)alkyle tel que défini ci-dessus, lié au reste de la molécule par l'intermédiaire d'un groupe aryle tel que défini ci-dessus. A titre d'exemple, on peut citer le groupe tolyle (CH₃Ph).

Par « hétérocycle », on entend, au sens de la présente invention, un monocycle ou bicycle hydrocarboné, saturé, insaturé ou aromatique, de préférence saturé ou insaturé mais non aromatique, contenant de 3 à 12 atomes de carbone, dans lequel 1 à 4, notamment 1 ou 2, atomes de carbone sont chacun remplacés, indépendamment les uns des autres, par un hétéroatome choisi parmi N, O et S, notamment choisi parmi N et O. Le bicycle comprend deux cycles accolés ou pontés. Chaque cycle du monocycle ou du bicycle comprend avantageusement 3 à 8, notamment 4 à 7, en particulier 5 ou 6, atomes de carbone ou hétéroatomes formant le cycle. A titre d'exemple on peut citer les hétérocycles azétidine, oxétane, thiooxétane, pyrrolidine, pyrroline, pyrrole, tétrahydrofurane, dihydrofurane, furane, tétrahydrothiophène, dihydrothiophène, thiophène, pipéridine, dihydropyridine, tétrahydropyridine, pyridine, pyrane, dihydropyrane, tétrahydropyrane, thiopyrane, dihydrothiopyrane, tétrahydrothiopyrane, morpholine, thiomorpholine, pipérazine, homopipérazine (ou diazépane), azépine, pyrazine, pyrimidine, pyridazine, perhydropyrrolo[3,4-c]pyrrole, 2,5-diazabicyclo[4.2.0]octane, 2,5-diazabicyclo[2.2.1]heptane et imidazole. De préférence, l'hétérocycle sera non aromatique et pourra être en particulier un cycle azétidine, oxétane, thiooxétane, pyrrolidine, pyrroline, tétrahydrofurane, dihydrofurane, tétrahydrothiophène, dihydrothiophène, pipéridine, dihydropyridine, tétrahydropyridine, pyrane, dihydropyrane, tétrahydropyrane, thiopyrane, dihydrothiopyrane, tétrahydrothiopyrane, morpholine, thiomorpholine, pipérazine, homopipérazine (ou diazépane), perhydropyrrolo[3,4-c]pyrrole, 2,5-diazabicyclo[4.2.0]octane, 3,8-diazabicyclo [3.2.1]octane et 2,5-diazabicyclo[2.2.1]heptane.

Par « hétérocycle azoté », on entend, au sens de la présente invention, un hétérocycle tel que défini ci-dessus comprenant au moins un atome d'azote, en particulier non aromatique, de préférence saturé. Il pourra s'agir en particulier d'un monocycle ou d'un bicycle dont chaque cycle comprend 5 à 7, de préférence 5 ou 6 chaînons et comprenant éventuellement, en plus de l'atome d'azote, un autre hétéroatome choisi de préférence parmi l'oxygène et l'azote. Il s'agira en particulier d'un groupe pipéridine, pipérazine éventuellement ponté (par ex. pipérazine, 2,5-diazabicyclo[4.2.0]octane, 3,8-diazabicyclo[3.2.1]octane ou 2,5-diazabicyclo[2.2.1]heptane ; notamment pipérazine, 2,5-diazabicyclo[4.2.0]octane, ou 2,5-diazabicyclo[2.2.1]heptane), morpholine, perhydropyrrolo[3,4-c]pyrrole, diazépane (par ex. 1,3-diazépane ou 1,4-diazépane) ou pyrrolidine.

Par cycles « accolés », on entend, au sens de la présente invention, deux cycles liés entre eux par deux atomes de carbone adjacents.

Par cycles « pontés », on entend, au sens de la présente invention, deux cycles liés entre eux par deux atomes de carbone non adjacents.

Par « pipérazine pontée », on entend, au sens de la présente invention, un cycle pipérazine dans laquelle deux atomes de carbone non adjacents sont reliés par une chaîne hydrocarbonée saturée ou insaturée, de préférence saturée, comprenant avantageusement 1 à 5, notamment 1 à 3, de préférence 1 ou 2, atomes de carbone. Il pourra s'agir notamment d'un 2,5-diazabicyclo[4.2.0]octane, d'un 3,8-diazabicyclo[3.2.1]octane ou d'un 2,5-diazabicyclo[2.2.1]heptane.

Par groupe « insaturé », on entend, au sens de la présente invention, un groupe comprenant au moins une liaison C=C ou C=C.

Par cycle « insaturé », on entend, au sens de la présente invention, un cycle comprenant au moins une liaison C=C mais non aromatique.

Par groupe « éventuellement substitué », on entend, au sens de la présente invention, un groupe éventuellement substitué par un ou plusieurs substituants. Ce(s) substituant(s) peu(ven)t être choisi(s) notamment parmi :
- un atome d'halogène,
- un groupe (C₁-C₆)alkyle éventuellement substitué par un ou plusieurs groupes choisis parmi un atome d'halogène, O**R₁₆**, S**R₁₇**, N**R₁₈R₁₉**, un carbocycle et un hétérocycle,
- les groupes oxo (=O), CN, NO₂, O**R₂₀**, S**R₂₁**, N**R₂₂R₂₃**, C(O)**R₂₄**, CO₂**R₂₅**, OC(O)**R₂₆**, S(O)**R₂₇**, SO₂**R₂₈**, N**R₂₉**C(O)**R₃₀**, C(O)N**R₃₁R₃₂**, N**R₃₃**CO₂**R₃₄**, OC(O)N**R₃₅R₃₆**, N**R₃₇**CON**R₃₈R₃₉** et OCO₂**R₄₀**,
- un carbocycle éventuellement substitué par un ou plusieurs groupes choisis parmi un atome d'halogène, un groupe (C₁-C₆)alkyle, oxo (=O), O**R₄₁**, S**R₄₂** et N**R₄₃R₄₄**,
- un hétérocycle éventuellement substitué par un ou plusieurs groupes choisis parmi un atome d'halogène, un groupe (C₁-C₆)alkyle, oxo (=O), O**R₄₅**, S**R₄₆** et N**R₄₇R₄₈**, et
- un groupe -O(CH₂)**ₙ**O- avec **n** représentant un nombre entier compris entre 1 et 5, notamment compris entre 2 et 3 (les deux oxygènes de ce groupe peuvent être attachés au même atome ou à deux atomes distincts, avantageusement, ils seront attachés au même atome, en particulier au même atome de carbone ce qui permettra dans ce cas de former un acétal cyclique),
où :
▪ **R₁₆** à **R₄₈** représentent, indépendamment les uns des autres, un atome d'hydrogène, un groupe (C₁-C₆)alkyle, aryle, aryl-(C₁-C₆)alkyle, hétérocycle ou hétérocycle-(C₁-C₆)alkyle,
   le noyau aryle de ces groupes étant éventuellement substitué par un ou plusieurs groupes choisis parmi un atome d'halogène et un groupe (C₁-C₆)alkyle, et
   le noyau hétérocycle de ces groupes étant éventuellement substitué par un ou plusieurs groupes choisis parmi un atome d'halogène, un groupe (C₁-C₆)alkyle, et oxo(=O), ou
▪ **R₂₂** et **R₂₃**, **R₃₁** et **R₃₂**, **R₃₅** et **R₃₆**, **R₃₈** et **R₃₉**, **R₄₃** et **R₄₄**, et/ou **R₄₇** et **R₄₈** forment ensemble, avec l'atome d'azote qui les porte, un hétérocycle azoté éventuellement substitué par un ou plusieurs groupes choisis parmi un atome d'halogène, un groupe (C₁-C₆)alkyle, et oxo(=O).

**Y** représentera plus particulièrement un atome d'azote ou un groupe C**Ry** avec **Ry** représentant un atome d'hydrogène, un atome d'halogène, un groupe (C₁-C₆)alkyle, (C₁-C₆)halogénoalkyle, (C₁-C₆)alcoxy, (C₁-C₆)halogénoalcoxy, N**R₁₂R₁₃**, CO₂H ou CO₂((C₁-C₆)alkyle), avec **R₁₂** et **R₁₃** représentant, indépendamment les uns des autres, un atome d'hydrogène ou un groupe (C₁-C₆)alkyle éventuellement substitué par un ou plusieurs atomes d'halogène, ou **R₁₂** et **R₁₃** formant avec l'atome d'azote qui les portent un hétérocycle à 5 ou 6 chaînons, de préférence non aromatique, comprenant éventuellement un autre hétéroatome choisi parmi O, N et S, et notamment O et N, ledit hétérocycle étant éventuellement substitué par un groupe (C₁-C₆)alkyle.

**Y** représentera plus particulièrement un atome d'azote ou un groupe C**Ry** avec **Ry** représentant un atome d'hydrogène, un atome d'halogène, un groupe (C₁-C₆)alkyle, (C₁-C₆)halogénoalkyle, (C₁-C₆)alcoxy, (C₁-C₆)halogénoalcoxy ou N**R₁₂R₁₃**, avec **R₁₂** et **R₁₃** représentant, indépendamment les uns des autres, un atome d'hydrogène ou un groupe (C₁-C₆)alkyle éventuellement substitué par un ou plusieurs atomes d'halogène, ou **R₁₂** et **R₁₃** formant avec l'atome d'azote qui les portent un hétérocycle à 5 ou 6 chaînons, de préférence non aromatique, comprenant éventuellement un autre hétéroatome choisi parmi O, N et S, et notamment O et N, ledit hétérocycle étant éventuellement substitué par un groupe (C₁-C₆)alkyle.

**Y** représentera plus particulièrement un atome d'azote ou un groupe C**Ry** avec **Ry** représentant un atome d'hydrogène, un atome d'halogène, un groupe (C₁-C₆)alkyle, (C₁-C₆)alcoxy, ou N**R₁₂R₁₃**, avec **R₁₂** et **R₁₃** représentant, indépendamment les uns des autres, un atome d'hydrogène ou un groupe (C₁-C₆)alkyle.

**Y** représentera en particulier un groupe C**Ry** avec **Ry** tel que défini selon l'une des définitions précédentes. **Ry** représentera notamment un atome d'hydrogène, un atome d'halogène, un groupe (C₁-C₆)alkyle ou (C₁-C₆)halogénoalkyle ; avantageusement un atome d'hydrogène ou un atome d'halogène (par ex. F). **Y** représentera en particulier un groupe CH ou CF.

**Q** représentera plus particulièrement un atome d'oxygène.

**n** et **m** représentent, indépendamment l'un de l'autre, 0, 1, 2 ou 3. Avantageusement, **n** et **m** représentent, indépendamment l'un de l'autre, 0 ou 1. De préférence, **n** et **m** représentent chacun 0.

**R₃**, **R₄**, **R₃'** et **R₄'** représentent notamment, indépendamment les uns des autres, un atome d'hydrogène, un groupe (C₁-C₆)alkyle ou OH, de préférence un atome d'hydrogène ou un groupe (C₁-C₆)alkyle, ou **R₃** et **R₄** et/ou **R₃'** et **R₄'** forment ensemble, avec l'atome de carbone qui les porte, un carbocycle ou hétérocycle monocyclique éventuellement substitué par un ou plusieurs groupes choisis parmi OH, (C₁-C₆)alkyle et oxo (=O).

Le carbocycle monocyclique pourra être en particulier un carbocycle monocyclique en C₃ à C₆, notamment en C₅ ou C₆, notamment saturé, par exemple un cyclopropyle, un cyclobutyle, un cyclopentyle ou un cyclohexyle.

L'hétérocycle monocyclique pourra être en particulier un hétérocycle monocyclique à 3 à 6 chaînons, notamment à 5 ou 6 chaînons, de préférence non aromatique, comprenant avantageusement un atome d'oxygène, par exemple un oxirane, un oxétane, un tétrahydrofurane ou un tétrahydropyrane.

**R₁**, **R₂**, **R₁'**, **R₂'**, **R₃**, **R₄**, **R₃'** et **R₄'** représenteront plus particulièrement un atome d'hydrogène ou un groupe (C₁-C₆)alkyle, de préférence un atome d'hydrogène.

Selon un mode de réalisation particulier, **Z** = O et **R₁** = **R₂** = **R₁'** = **R₂'** = **R₃** = **R₄** = **R₃'** = **R₄'** = H.

**R₇**, **R₈**, **R₁₂** et **R₁₃** représentent avantageusement, indépendamment les uns des autres, un atome d'hydrogène ou un groupe (C₁-C₆)alkyle, (C₂-C₆)alcényle ou (C₂-C₆)alcynyle, ledit groupe étant éventuellement susbtitué par un ou plusieurs groupes choisis parmi un atome d'halogène, CN, O**R₅₇**, N**R₅₈R₅₉**, COO**R₆₀** et CO**NR₆₁R₆₂** avec **R₅₇** à **R₆₂** représentant, indépendamment les uns des autres, un atome d'hydrogène ou un groupe (C₁-C₆)alkyle,
ou **R₇** et **R₈** et/ou **R₁₂** et **R₁₃,** indépendamment les uns des autres, forment avec l'atome d'azote qui les portent un hétérocycle azoté (tel que pipéridine, pipérazine éventuellement ponté (par ex. pipérazine, 2,5-diazabicyclo[4.2.0]octane ou 2,5-diazabicyclo[2.2.1]heptane), morpholine, perhydropyrrolo[3,4-c]pyrrole, diazépane (par ex. 1,3-diazépane ou 1,4-diazépane) ou pyrrolidine) éventuellement substitué par un ou plusieurs groupes choisis parmi les groupes (C₁-C₆)alkyle, (C₂-C₆)alcényle, (C₂-C₆)alcynyle et carbocycle monocyclique saturé ou insaturé en C₃ à C₈.

**R₇**, **R₈**, **R₁₂** et **R₁₃** représentent notamment, indépendamment les uns des autres, un atome d'hydrogène ou un groupe (C₁-C₆)alkyle éventuellement susbtitué par un ou plusieurs groupes choisis parmi un atome d'halogène, CN, O**R₅₇**, N**R₅₈R₅₉**, COO**R₆₀** et CON**R₆₁R₆₂** avec **R₅₇** à **R₆₂** représentant, indépendamment les uns des autres, un atome d'hydrogène ou un groupe (C₁-C₆)alkyle,
ou **R₇** et **R₈** et/ou **R₁₂** et **R₁₃,** indépendamment les uns des autres, forment avec l'atome d'azote qui les portent un hétérocycle azoté choisi parmi les cycles pipéridine, pipérazine, morpholine et pyrrolidine et éventuellement substitué par un ou plusieurs groupes (C₁-C₆)alkyle.

**R₇**, **R₈**, **R₁₂** et **R₁₃** représentent en particulier, indépendamment les uns des autres, un atome d'hydrogène ou un groupe (C₁-C₆)alkyle éventuellement susbtitué par un ou plusieurs groupes choisis parmi un atome d'halogène,
ou **R₇** et **R₈** et/ou **R₁₂** et **R₁₃,** indépendamment les uns des autres, forment avec l'atome d'azote qui les portent un hétérocycle azoté choisi parmi les cycles pipéridine, pipérazine, morpholine et pyrrolidine et éventuellement substitué par un ou plusieurs groupes (C₁-C₆)alkyle.

**R₇**, **R₈**, **R₁₂** et **R₁₃** représenteront notamment, indépendamment les uns des autres, un atome d'hydrogène ou un groupe (C₁-C₆)alkyle.

**R₅** et **R₆** représentent, indépendamment l'un de l'autre, un atome d'hydrogène, un atome d'halogène, un groupe (C₁-C₆)alkyle, (C₁-C₆)halogénoalkyle, (C₁-C₆)alcoxy, (C₁-C₆)halogénoalcoxy, (C₁-C₆)thioalcoxy, (C₁-C₆)halogénothioalcoxy, OH, SH, CN, NO₂, ou **NR₇R₈**, avec avantageusement **R₇** et **R₈** représentant, indépendamment l'un de l'autre, un atome d'hydrogène ou un groupe (C₁-C₆)alkyle éventuellement substitué par un ou plusieurs atomes d'halogène.

**R₅** et **R₆** représentent notamment, indépendamment l'un de l'autre, un atome d'hydrogène, un atome d'halogène, un groupe (C₁-C₆)alkyle, (C₁-C₆)halogénoalkyle, (C₁-C₆)alcoxy ou **NR₇R₈**, avec avantageusement **R₇** et **R₈** représentant, indépendamment l'un de l'autre, un atome d'hydrogène ou un groupe (C₁-C₆)alkyle.

**R₅** et **R₆** représentent en particulier, indépendamment l'un de l'autre, un atome d'hydrogène, un atome d'halogène, un groupe (C₁-C₆)alkyle ou (C₁-C₆)halogénoalkyle.

**R₅** et **R₆** représenteront notamment, indépendamment l'un de l'autre, un atome d'hydrogène ou un groupe (C₁-C₆)alkyle.

**R₁₁** représente avantageusement un atome d'hydrogène, un atome d'halogène, ou un groupe (C₁-C₆)alkyle ou (C₁-C₆)halogénoalkyle; notamment un atome d'hydrogène ou d'halogène. **R₁₁** pourra représenter en particulier H ou F.

**R₉** et **R₁₀** représentent, indépendamment l'un de l'autre, un atome d'hydrogène, un atome d'halogène, un groupe (C₁-C₆)alkyle éventuellement substitué, (C₂-C₆)alcényle éventuellement substitué, (C₂-C₆)alcynyle éventuellement substitué, (C₁-C₆)alcoxy éventuellement substitué, (C₁-C₆)thioalcoxy éventuellement substitué, CN, NO₂, OH, SH, N**R₁₄R₁₅**, CO₂**R₅₄**, CON**R₅₅R₅₆**, un carbocycle éventuellement substitué ou un hétérocycle éventuellement substitué.

**R₉** et **R₁₀** représentent plus particulièrement, indépendamment l'un de l'autre, un atome d'hydrogène, un atome d'halogène, un groupe (C₁-C₆)alkyle éventuellement substitué, (C₂-C₆)alcényle éventuellement substitué, (C₂-C₆)alcynyle éventuellement substitué, (C₁-C₆)alcoxy éventuellement substitué, (C₁-C₆)thioalcoxy éventuellement substitué, N**R₁₄R₁₅**, CO₂**R₅₄**, CON**R₅₅R₅₆**, un carbocycle éventuellement substitué ou un hétérocycle éventuellement substitué, notamment avec **R₁₅** ≠ H et **R₅₆** ≠ H.

**R₉** et **R₁₀** représentent notamment, indépendamment l'un de l'autre, un atome d'hydrogène, un atome d'halogène, un groupe (C₁-C₆)alkyle éventuellement substitué, (C₂-C₆)alcényle éventuellement substitué, (C₂-C₆)alcynyle éventuellement substitué, (C₁-C₆)alcoxy éventuellement substitué, (C₁-C₆)thioalcoxy éventuellement substitué, N**R₁₄R₁₅**, CO₂**R₅₄**, CON**R₅₅R₅₆**, ou un hétérocycle éventuellement substitué, notamment avec **R₁₅** ≠ H et **R₅₆** ≠ H.

**R₉** et **R₁₀** représentent en particulier, indépendamment l'un de l'autre, un atome d'hydrogène, un atome d'halogène, CO₂**R₅₄**, CON**R₅₅R₅₆**, ou un groupe (C₁-C₆)alkyle, (C₂-C₆)alcényle, (C₂-C₆)alcynyle, (C₁-C₆)alcoxy, (C₁-C₆)thioalcoxy, (C₁-C₆)alkyl-amino, di((C₁-C₆)alkyl)amino ou hétérocycle, ledit groupe étant éventuellement substitué, notamment avec **R₅₆** ≠ H et de préférence avec **R₅₅** et **R₅₆** formant avec l'atome d'azote qui les portent un hétérocycle azoté éventuellement substitué.

**R₉** et **R₁₀** représentent plus particulièrement, indépendamment l'un de l'autre, un atome d'hydrogène, un atome d'halogène, CON**R₅₅R₅₆**, ou un groupe (C₁-C₆)alkyle, (C₂-C₆)alcényle, (C₂-C₆)alcynyle, (C₁-C₆)alcoxy, (C₁-C₆)thioalcoxy, (C₁-C₆)alkyl-amino, di((C₁-C₆)alkyl)amino ou hétérocycle, ledit groupe étant éventuellement substitué, notamment avec **R₅₆** ≠ H et de préférence avec **R₅₅** et **R₅₆** formant avec l'atome d'azote qui les portent un hétérocycle azoté éventuellement substitué.

Dans les définitions de **R₉** et **R₁₀** précédentes, **R₁₄** et **R₁₅** représentent, indépendamment l'un de l'autre, un atome d'hydrogène ou un groupe (C₁-C₆)alkyle éventuellement substitué, (C₂-C₆)alcényle éventuellement substitué, ou (C₂-C₆)alcynyle éventuellement substitué, ou **R₁₄** et **R₁₅** eforment avec l'atome d'azote qui les portent un hétérocycle azoté éventuellement substitué. En particulier, **R₁₄** pourra représenter un atome d'hydrogène ou un groupe (C₁-C₆)alkyle et **R₁₅** pourra représenter un atome d'hydrogène ou un groupe (C₁-C₆)alkyle éventuellement substitué, (C₂-C₆)alcényle éventuellement substitué, ou (C₂-C₆)alcynyle éventuellement substitué, ou **R₁₄** et **R₁₅** formeront avec l'atome d'azote qui les portent un hétérocycle azoté éventuellement substitué. Avantageusement, **R₁₄** représentera un atome d'hydrogène ou un groupe (C₁-C₆)alkyle et **R₁₅** représentera un atome d'hydrogène ou un groupe (C₁-C₆)alkyle éventuellement substitué, ou **R₁₄** et **R₁₅** formeront avec l'atome d'azote qui les portent un hétérocycle azoté éventuellement substitué. De préférence, l'hétérocycle azoté éventuellement substitué sera un hétérocycle azoté monocyclique ou bicyclique, de préférence monocyclique, notamment non aromatique, de préférence saturé, dont chaque cycle comprend 5 à 7 chaînons, comprenant éventuellement 1 hétéroatome en plus de l'atome d'azote choisi parmi N et O, tel qu'un cycle pipérazine éventuellement pontée, pipéridine, morpholine, perhydropyrrolo[3,4-c]pyrrole, diazépane (par ex. 1,3-diazépane ou 1,4-diazépane) ou pyrrolidine, l'hétérocycle étant éventuellement substitué par un ou plusieurs groupes choisis parmi un atome d'halogène, un groupe (C₁-C₆)alkyle et oxo(=O).

Préférentiellement, la pipérazine éventuellement ponté sera un cycle pipérazine, 2,5-diazabicyclo[4.2.0]octane, 3,8-diazabicyclo[3.2.1]octane ou 2,5-diazabicyclo[2.2.1]heptane, notamment un cycle pipérazine, 2,5-diazabicyclo[4.2.0]octane ou 2,5-diazabicyclo [2.2.1]heptane.

Dans les définitions de **R₉** et **R₁₀** précédentes, un carbocycle est plus particulièrement un carbocycle monocyclique en C₃ à C₆, notamment en C₅ ou C₆, notamment saturé, par exemple un cyclopropyle, un cyclobutyle, un cyclopentyle, un cyclohexyle ou un cyclohexényle.

Dans les définitions de **R₉** et **R₁₀** précédentes, un hétérocycle est plus particulièrement un hétérocycle monocyclique ou bicyclique, de préférence monocyclique, chaque cycle étant à 5, 6 ou 7 chaînons, notamment à 6 ou 7 chaînons, saturé, insaturé ou aromatique, notamment saturé ou aromatique, de préférence saturé, l'hétérocycle comprenant 1 ou 2 hétéroatomes choisis parmi N, O et S, notamment parmi N et O, et de préférence comprenant au moins un atome d'azote. L'hétérocycle pourra être par exemple un cycle pyrrolidine, pyrroline, pyrrole, tétrahydrofurane, dihydrofurane, furane, tétrahydrothiophène, dihydrothiophène, thiophène, pipéridine, dihydropyridine, tétrahydropyridine, pyridine, pyrane, dihydropyrane, tétrahydropyrane, thiopyrane, dihydrothiopyrane, tétrahydrothiopyrane, morpholine, thiomorpholine, pipérazine, homopipérazine, azépine, pyrazine, pyrimidine, pyridazine, perhydropyrrolo[3,4-c]pyrrole, 2,5-diazabicyclo[4.2.0]octane, 2,5-diazabicyclo[2.2.1]heptane, 3,8-diazabicyclo[3.2.1]octane ou imidazole. L'hétérocycle sera notamment un cycle pyrrolidine, pyrroline, pyrrole, tétrahydrofurane, dihydrofurane, furane, pipéridine, dihydropyridine, tétrahydropyridine, pyridine, pyrane, dihydropyrane, tétrahydropyrane, morpholine, pipérazine, homopipérazine, azépine, pyrazine, pyrimidine, pyridazine, perhydropyrrolo[3,4-c]pyrrole, 2,5-diazabicyclo[4.2.0]octane, 2,5-diazabicyclo[2.2.1]heptane, 3,8-diazabicyclo[3.2.1]octane ou imidazole. L'hétérocycle sera en particulier un cycle pyrrolidine, pyrroline, pyrrole, pipéridine, dihydropyridine, tétrahydropyridine, pyridine, morpholine, pipérazine, homopipérazine, azépine, pyrazine, pyrimidine, pyridazine, perhydropyrrolo[3,4-c]pyrrole, 2,5-diazabicyclo[4.2.0]octane, 2,5-diazabicyclo[2.2.1]heptane, 3,8-diazabicyclo[3.2.1]octane ou imidazole. L'hétérocycle sera plus particulièrement un cycle pyrrolidine, pyrroline, pyrrole, pipéridine, dihydropyridine, tétrahydropyridine, pyridine, morpholine, pipérazine, homopipérazine, azépine, pyrazine, pyrimidine, pyridazine ou imidazole. L'hétérocycle sera notamment un cycle pyrrolidine, pyrrole, pipéridine, pyridine, morpholine, pipérazine, homopipérazine (par ex. 1,3-diazépane ou 1,4-diazépane), azépine, pyrazine, pyrimidine, pyridazine ou imidazole. L'hétérocycle pourra être avantageusement un cycle pipéridine, pyridine, morpholine, pipérazine ou 1,4-diazépane.

Dans les définitions de **R₉**, **R₁₀**, **R₁₄** et **R₁₅** précédentes, un groupe ou un cycle éventuellement substitué est un groupe ou un cycle éventuellement substitué par un ou plusieurs substituants, avantageusement choisis parmi :
- un atome d'halogène,
- un groupe (C₁-C₆)alkyle éventuellement substitué par un ou plusieurs groupes choisis parmi un atome d'halogène, O**R₁₆**, S**R₁₇**, N**R₁₈R₁₉**, un carbocycle et un hétérocycle,
- les groupes oxo (=O), CN, NO₂, O**R₂₀**, S**R₂₁**, N**R₂₂R₂₃**, C(O)**R₂₄**, CO₂**R₂₅**, OC(O)**R₂₆**, S(O)**R₂₇**, SO₂**R₂₈**, N**R₂₉**C(O)**R₃₀**, C(O)N**R₃₁R₃₂**, N**R₃₃**CO₂**R₃₄**, OC(O)N**R₃₅R₃₆**, N**R₃₇**CON**R₃₈R₃₉** et OCO₂**R**_{**40**,}
- un carbocycle éventuellement substitué par un ou plusieurs groupes choisis parmi un atome d'halogène, un groupe (C₁-C₆)alkyle, oxo (=O), O**R₄₁**, S**R₄₂** et N**R₄₃R₄₄**,
- un hétérocycle éventuellement substitué par un ou plusieurs groupes choisis parmi un atome d'halogène, un groupe (C₁-C₆)alkyle, oxo (=O), O**R₄₅**, S**R₄₆** et N**R₄₇R₄₈**, et
- un groupe -O(CH₂)**ₙ**O- avec **n** représentant un nombre entier compris entre 1 et 5, notamment compris entre 2 et 3 (les deux oxygènes de ce groupe peuvent être attachés au même atome ou à deux atomes distincts, avantageusement, ils seront attachés au même atome, en particulier au même atome de carbone ce qui permettra dans ce cas de former un acétal cyclique),
où :
▪ **R₁₆** à **R₄₈** représentent, indépendamment les uns des autres, un atome d'hydrogène, un groupe (C₁-C₆)alkyle, aryle, aryl-(C₁-C₆)alkyle, hétérocycle ou hétérocycle-(C₁-C₆)alkyle,
   le noyau aryle de ces groupes étant éventuellement substitué par un ou plusieurs groupes choisis parmi un atome d'halogène et un groupe (C₁-C₆)alkyle, et
   le noyau hétérocycle de ces groupes étant éventuellement substitué par un ou plusieurs groupes choisis parmi un atome d'halogène, un groupe (C₁-C₆)alkyle, et oxo(=O), ou
▪ **R₂₂** et **R₂₃**, **R₃₁** et **R₃₂**, **R₃₅** et **R₃₆**, **R₃₈** et **R₃₉**, **R₄₃** et **R₄₄**, et/ou **R₄₇** et **R₄₈** forment ensemble, avec l'atome d'azote qui les porte, un hétérocycle azoté éventuellement substitué par un ou plusieurs groupes choisis parmi un atome d'halogène, un groupe (C₁-C₆)alkyle, et oxo(=O).

Dans les définitions de **R₉**, **R₁₀**, **R₁₄** et **R₁₅** précédentes, les groupes ou cycles éventuellement substitués sont en particulier éventuellement substitués par un ou plusieurs substituants choisis parmi :
- un atome d'halogène,
- un groupe (C₁-C₆)alkyle éventuellement substitué par un ou plusieurs groupes choisis parmi un atome d'halogène, O**R₁₆**, N**R₁₈R₁₉**, un carbocycle monocyclique en C₃ à C₆ (notamment saturé) et un hétérocycle monocyclique à 3 à 6 chaînons (notamment saturé),
- les groupes oxo (=O), O**R₂₀**, N**R₂₂R₂₃**, C(O)**R₂₄**, CO₂**R₂₅**, OC(O)**R₂₆**, N**R₂₉**C(O)R**₃₀**, C(O)N**R₃₁R₃₂**, N**R₃₃**CO₂**R₃₄**, OC(O)N**R₃₅**R**₃₆**, N**R₃₇**CON**R₃₈R₃₉** et OCO₂**R₄₀**, et plus particulièrement les groupes oxo (=O), O**R₂₀**, N**R₂₂R₂₃**, CO₂**R₂₅**, C(O)N**R₃₁R₃₂**, N**R₃₃**CO₂**R₃₄**, OC(O)N**R₃₅R₃₆**, N**R₃₇**CON**R₃₈R₃₉** et OCO₂**R₄₀**,
- un carbocycle en C₃ à C₆ éventuellement substitué par un ou plusieurs groupes choisis parmi un atome d'halogène, un groupe (C₁-C₆)alkyle, oxo (=O), O**R₄₁** et N**R₄₃R₄₄**,
- un hétérocycle à 3 à 6 chaînons, notamment à 5 ou 6 chaînons, comprenant 1 ou 2 hétéroatomes choisis parmi N et O, de préférence saturé, éventuellement substitué par un ou plusieurs groupes choisis parmi un atome d'halogène, un groupe (C₁-C₆)alkyle, oxo (=O), O**R₄₅** et N**R₄₇R₄₈**, et
- un groupe -O(CH₂)**ₙ**O- avec **n** représentant un nombre entier égal à 2 ou 3 (les deux oxygènes de ce groupe peuvent être attachés au même atome ou à deux atomes distincts, avantageusement, ils seront attachés au même atome, en particulier au même atome de carbone ce qui permettra dans ce cas de former un acétal cyclique),
   où :

▪ **R₁₆**, **R₁₈** à **R₂₀**, **R₂₂** à **R₂₆**, **R₂₉** à **R₄₀**, **R₄₅** et **R₄₇** à **R₄₈** représentent, indépendamment les uns des autres, un atome d'hydrogène, un groupe (C₁-C₆)alkyle, aryle, aryl-(C₁-C₆)alkyle, hétérocycle ou hétérocycle-(C₁-C₆)alkyle, notamment un atome d'hydrogène, un groupe (C₁-C₆)alkyle, aryle, ou aryl-(C₁-C₆)alkyle, en particulier un atome d'hydrogène, ou un groupe (C₁-C₆)alkyle,
   le noyau aryle de ces groupes étant un groupe phényle et étant éventuellement substitué par un ou plusieurs groupes choisis parmi un atome d'halogène et un groupe (C₁-C₆)alkyle, et
   le noyau hétérocycle de ces groupes étant un hétérocycle à 3 à 6 chaînons, notamment à 5 ou 6 chaînons, comprenant 1 ou 2 hétéroatomes choisis parmi N et O et étant éventuellement substitué par un ou plusieurs groupes choisis parmi un atome d'halogène, un groupe (C₁-C₆)alkyle, et oxo(=O), ou
▪ **R₂₂** et **R₂₃**, **R₃₁** et **R₃₂**, **R₃₅** et **R₃₆**, **R₃₈** et **R₃₉**, **R₄₃** et **R₄₄**, et/ou **R₄₇** et **R₄₈** forment ensemble, avec l'atome d'azote qui les porte, un hétérocycle azoté à 5 ou 6 chaînons, notamment non aromatique, de préférence saturé, comprenant éventuellement 1 hétéroatome en plus de l'atome d'azote choisi parmi N et O, tel qu'un cycle pipérazine, pipéridine, morpholine ou pyrrolidine, l'hétérocycle étant éventuellement substitué par un ou plusieurs groupes choisis parmi un atome d'halogène, un groupe (C₁-C₆)alkyle, et oxo(=O).

Les groupes **R₉** et **R₁₀** pourront en particulier représenter, indépendamment l'un de l'autre :
- un atome d'hydrogène ou d'halogène,
- un groupe CO₂**R₅₄** avec **R₅₄** représentant un atome d'hydrogène ou un groupe (C₁-C₆)alkyle éventuellement substitué par un ou plusieurs substituants choisis parmi un atome d'halogène, O**R₂₀**, N**R₂₂R₂₃**, C(O)**R₂₄**, CO₂**R₂₅**, OC(O)**R₂₆**, N**R₂₉**C(O)R**₃₀**, C(O)N**R₃₁R₃₂**, N**R₃₃**CO₂**R₃₄**, OC(O)N**R₃₅**R**₃₆**, N**R₃₇**CON**R₃₈R₃₉** et OCO₂**R₄₀**; en particulier choisis parmi O**R₂₀**, N**R₂₂R₂₃**, CO₂**R₂₅**, C(O)N**R₃₁R₃₂**, N**R₃₃**CO₂**R₃₄**, OC(O)N**R₃₅**R**₃₆**, N**R₃₇**CON**R₃₈R₃₉** et OCO₂**R₄₀**; notamment choisis parmi N**R₂₂R₂₃**, N**R₃₃**CO₂**R₃₄** et N**R₃₇**CON**R₃₈R₃₉**; en particulier avec **R₅₄** représentant un atome d'hydrogène ou un groupe (C₁-C₆)alkyle, notamment un atome d'hydrogène,
- un groupe (C₁-C₆)alkyle, (C₂-C₆)alcényle, (C₂-C₆)alcynyle, (C₁-C₆)alcoxy, (C₁-C₆)thioalcoxy, (C₁-C₆)alkyl-amino ou di((C₁-C₆)alkyl)amino, ledit groupe étant éventuellement substitué par un ou plusieurs substituants choisis parmi un atome d'halogène, O**R₂₀**, N**R₂₂R₂₃**, C(O)**R₂₄**, CO₂**R₂₅**, OC(O)**R₂₆**, N**R₂₉**C(O)R**₃₀**, C(O)N**R₃₁R₃₂**, N**R₃₃**CO₂**R₃₄**, OC(O)N**R₃₅**R**₃₆**, N**R₃₇**CON**R₃₈R₃₉** et OCO₂**R₄₀**; en particulier choisis parmi O**R₂₀**, N**R₂₂R₂₃**, CO₂**R₂₅**, C(O)N**R₃₁R₃₂**, N**R₃₃**CO₂**R₃₄**, OC(O)N**R₃₅**R**₃₆**, N**R₃₇**CON**R₃₈R₃₉** et OCO₂**R₄₀**; notamment choisis parmi N**R₂₂R₂₃**, N**R₃₃**CO₂**R₃₄** et N**R₃₇**CON**R₃₈R₃₉**, ou
- un hétérocycle ou un groupe CON**R₅₅R₅₆** avec **R₅₅** et **R₅₆** formant avec l'atome d'azote qui les portent un hétérocycle, l'hétérocycle étant à 5, 6 ou 7 chaînons, notamment à 6 ou 7 chaînons, comprenant 1 ou 2 hétéroatomes choisis parmi N et O (et comprenant au moins un atome d'azote dans le cas de N**R₅₅R₅₆**), de préférence saturé ou aromatique, notamment saturé, (l'hétérocycle peut être en particulier un cycle pipérazine éventuellement pontée (par ex. pipérazine, 2,5-diazabicyclo[4.2.0]octane, 3,8-diazabicyclo[3.2.1]octane ou 2,5-diazabicyclo[2.2.1]heptane), pipéridine, perhydropyrrolo[3,4-c]pyrrole, tétrahydropyridine ou pyrrolidine ; notamment un cycle pipérazine, pipéridine, morpholine diazépane (par ex. 1,4-diazépane) ou pyridine) éventuellement substitué par un ou plusieurs substituants choisis parmi :
   - un atome d'halogène,
   - un groupe (C₁-C₆)alkyle éventuellement substitué par un ou plusieurs groupes choisis parmi un atome d'halogène, O**R₁₆**, N**R₁₈R₁₉**, un carbocycle monocyclique en C₃ à C₆ (notamment saturé) et un hétérocycle monocyclique à 3 à 6 chaînons (notamment saturé),
   - les groupes oxo (=O), O**R₂₀**, N**R₂₂R₂₃**, C(O)**R₂₄**, CO₂**R₂₅**, OC(O)**R₂₆**, N**R₂₉**C(O)**R₃₀**, C(O)N**R₃₁R₃₂**, N**R₃₃**CO₂**R₃₄**, OC(O)N**R₃₅**R**₃₆**, N**R₃₇**CON**R₃₈R₃₉** et OCO₂**R₄₀**; et plus particulièrement les groupes oxo (=O), O**R₂₀**, N**R₂₂R₂₃**, CO₂**R₂₅**, C(O)N**R₃₁R₃₂**, N**R₃₃**CO₂**R₃₄**, OC(O)N**R₃₅**R**₃₆**, N**R₃₇**CON**R₃₈R₃₉** et OCO₂**R₄₀**,
   - un carbocycle en C₃ à C₆ éventuellement substitué par un ou plusieurs groupes choisis parmi un atome d'halogène, un groupe (C₁-C₆)alkyle, oxo (=O), O**R₄₁** et N**R₄₃R₄₄**,
   - un hétérocycle à 3 à 6 chaînons, comprenant 1 ou 2 hétéroatomes choisis parmi N et O, notamment saturé ou insaturé, de préférence saturé, éventuellement substitué par un ou plusieurs groupes choisis parmi un atome d'halogène, un groupe (C₁-C₆)alkyle, oxo (=O), O**R₄₅** et N**R₄₇R₄₈**, et
   - un groupe -O(CH₂)**ₙ**O- avec **n** représentant un nombre entier égal à 2 ou 3 (les deux oxygènes de ce groupe peuvent être attachés au même atome ou à deux atomes distincts, avantageusement, ils seront attachés au même atome, en particulier au même atome de carbone ce qui permettra dans ce cas de former un acétal cyclique),
où :
▪ **R₁₆**, **R₁₈** à **R₂₀, R₂₂** à **R₂₆**, **R₂₉** à **R₄₀**, **R₄₅** et **R₄₇** à **R₄₈** représentent, indépendamment les uns des autres, un atome d'hydrogène, un groupe (C₁-C₆)alkyle, aryle, aryl-(C₁-C₆)alkyle, hétérocycle ou hétérocycle-(C₁-C₆)alkyle, notamment un atome d'hydrogène, un groupe (C₁-C₆)alkyle, aryle, ou aryl-(C₁-C₆)alkyle, en particulier un atome d'hydrogène, ou un groupe (C₁-C₆)alkyle,
   le noyau aryle de ces groupes étant un groupe phényle et étant éventuellement substitué par un ou plusieurs groupes choisis parmi un atome d'halogène et un groupe (C₁-C₆)alkyle, et
   le noyau hétérocycle de ces groupes étant un hétérocycle à 3 à 6 chaînons, notamment à 5 ou 6 chaînons, comprenant 1 ou 2 hétéroatomes choisis parmi N et O, et étant éventuellement substitué par un ou plusieurs groupes choisis parmi un atome d'halogène, un groupe (C₁-C₆)alkyle et oxo(=O), ou
▪ **R₂₂** et **R₂₃**, **R₃₁** et **R₃₂**, **R₃₅** et **R₃₆**, **R₃₈** et **R₃₉**, **R₄₃** et **R₄₄**, et/ou **R₄₇** et **R₄₈** forment ensemble, avec l'atome d'azote qui les porte, un hétérocycle azoté à 5 ou 6 chaînons, notamment non aromatique, de préférence saturé, comprenant éventuellement 1 hétéroatome en plus de l'atome d'azote choisi parmi N et O, tel qu'un cycle pipérazine, pipéridine, morpholine ou pyrrolidine, l'hétérocycle étant éventuellement substitué par un ou plusieurs groupes choisis parmi un atome d'halogène, un groupe (C₁-C₆)alkyle, et oxo(=O).

Les groupes **R₉** et **R₁₀** pourront en particulier représenter, indépendamment l'un de l'autre :
- un atome d'hydrogène ou d'halogène,
- un groupe CO₂**R₅₄** avec **R₅₄** représentant un atome d'hydrogène ou un groupe (C₁-C₆)alkyle éventuellement substitué par un ou plusieurs substituants choisis parmi un atome d'halogène, O**R₂₀**, N**R₂₂R₂₃**, C(O)**R₂₄**, CO₂**R₂₅**, OC(O)**R₂₆**, N**R₂₉**C(O)R**₃₀**, C(O)N**R₃₁R₃₂**, N**R₃₃**CO₂**R₃₄**, OC(O)N**R₃₅**R**₃₆**, N**R₃₇**CON**R₃₈R₃₉** et OCO₂**R₄₀**; en particulier choisis parmi O**R₂₀**, N**R₂₂R₂₃**, CO₂**R₂₅**, C(O)N**R₃₁R₃₂**, N**R₃₃**CO₂**R₃₄**, OC(O)N**R₃₅**R**₃₆**, N**R₃₇**CON**R₃₈R₃₉** et OCO₂**R₄₀**; notamment choisis parmi N**R₂₂R₂₃**, N**R₃₃**CO₂**R₃₄** et N**R₃₇**CON**R₃₈R₃₉**; en particulier avec **R₅₄** représentant un atome d'hydrogène ou un groupe (C₁-C₆)alkyle, notamment un atome d'hydrogène,
- un groupe CON**R₅₅R₅₆** avec **R₅₅** et **R₅₆** formant avec l'atome d'azote qui les portent un hétérocycle azoté à 5, 6 ou 7 chaînons, comprenant 1 ou 2 hétéroatomes choisis parmi N et O dont au moins un atome d'azote, notamment non aromatique, de préférence saturé, (l'hétérocycle peut être en particulier un cycle pipérazine, pipéridine, morpholine diazépane (par ex. 1,4-diazépane) ou pyridine) éventuellement substitué par un ou plusieurs substituants choisis parmi un atome d'halogène, (C₁-C₆)alkyle, oxo (=O), O**R₂₀**, N**R₂₂R₂₃**, C(O)**R₂₄**, CO₂**R₂₅**, OC(O)**R₂₆**, N**R₂₉**C(O)R**₃₀**, C(O)N**R₃₁R₃₂**, N**R₃₃**CO₂**R₃₄**, OC(O)N**R₃₅R₃₆**, N**R₃₇**CON**R₃₈R₃₉** et OCO₂**R₄₀**; en particulier choisis parmi un atome d'halogène, (C₁-C₆)alkyle, oxo (=O), O**R₂₀**, N**R₂₂R₂₃**, CO₂**R₂₅**, C(O)N**R₃₁R₃₂**, N**R₃₃**CO₂**R₃₄**, OC(O)N**R₃₅R₃₆**, N**R₃₇**CON**R₃₈R₃₉** et OCO₂**R₄₀**; notamment choisis parmi un atome d'halogène, (C₁-C₆)alkyle, oxo (=O), O**R₂₀**, N**R₂₂R₂₃**, CO₂**R₂₅** et C(O)N**R₃₁R₃₂**,
- un groupe (C₁-C₆)alkyle, (C₂-C₆)alcényle, (C₂-C₆)alcynyle, (C₁-C₆)alcoxy, (C₁-C₆)thioalcoxy, (C₁-C₆)alkyl-amino ou di((C₁-C₆)alkyl)amino, ledit groupe étant éventuellement substitué par un ou plusieurs substituants choisis parmi un atome d'halogène, O**R₂₀**, N**R₂₂R₂₃**, C(O)**R₂₄**, CO₂**R₂₅**, OC(O)**R₂₆**, N**R₂₉**C(O)R**₃₀**, C(O)N**R₃₁R₃₂**, N**R₃₃**CO₂**R₃₄**, OC(O)N**R₃₅**R**₃₆**, N**R₃₇**CON**R₃₈R₃₉** et OCO₂**R₄₀**; en particulier choisis parmi O**R₂₀**, N**R₂₂R₂₃**, CO₂**R₂₅**, C(O)N**R₃₁R₃₂**, N**R₃₃**CO₂**R₃₄**, OC(O)N**R₃₅**R**₃₆**, N**R₃₇**CON**R₃₈R₃₉** et OCO₂**R₄₀**; notamment choisis parmi N**R₂₂R₂₃**, N**R₃₃**CO₂**R₃₄** et N**R₃₇**CON**R₃₈R₃₉**, ou
- un hétérocycle à 5, 6 ou 7 chaînons, notamment à 6 ou 7 chaînons, comprenant 1 ou 2 hétéroatomes choisis parmi N et O et comprenant avantageusement au moins un atome d'azote, de préférence saturé ou aromatique, notamment saturé, (l'hétérocycle peut être en particulier un cycle pipérazine éventuellement pontée (par ex. pipérazine, 2,5-diazabicyclo[4.2.0]octane, 3,8-diazabicyclo[3.2.1]octane ou 2,5-diazabicyclo[2.2.1]heptane), pipéridine, perhydropyrrolo[3,4-c]pyrrole, tétrahydropyridine ou pyrrolidine ; notamment un cycle pipérazine, pipéridine, morpholine diazépane (par ex. 1,4-diazépane) ou pyridine) éventuellement substitué par un ou plusieurs substituants choisis parmi :
   - un atome d'halogène,
   - un groupe (C₁-C₆)alkyle éventuellement substitué par un ou plusieurs groupes choisis parmi un atome d'halogène, O**R₁₆**, N**R₁₈R₁₉**, un carbocycle monocyclique en C₃ à C₆ (notamment saturé) et un hétérocycle monocyclique à 3 à 6 chaînons (notamment saturé) comprenant 1 ou 2 hétéroatomes choisis parmi N et O,
   - les groupes oxo (=O), O**R₂₀**, N**R₂₂R₂₃**, C(O)**R₂₄**, CO₂**R₂₅**, OC(O)**R₂₆**, N**R₂₉**C(O)R**₃₀**, C(O)N**R₃₁R₃₂**, N**R₃₃**CO₂**R₃₄**, OC(O)N**R₃₅R₃₆**, N**R₃₇**CON**R₃₈R₃₉** et OCO₂**R₄₀**; et plus particulièrement les groupes oxo (=O), O**R₂₀**, N**R₂₂R₂₃**, CO₂**R₂₅**, C(O)N**R₃₁R₃₂**, N**R₃₃**CO₂**R₃₄**, OC(O)N**R₃₅**R**₃₆**, N**R₃₇**CON**R₃₈R₃₉** et OCO₂**R₄₀**,
   - un carbocycle en C₃ à C₆ éventuellement substitué par un ou plusieurs groupes choisis parmi un atome d'halogène, un groupe (C₁-C₆)alkyle, oxo (=O), O**R₄₁** et N**R₄₃R₄₄**,
   - un hétérocycle à 3 à 6 chaînons, comprenant 1 ou 2 hétéroatomes choisis parmi N et O, notamment saturé ou insaturé, de préférence saturé, éventuellement substitué par un ou plusieurs groupes choisis parmi un atome d'halogène, un groupe (C₁-C₆)alkyle, oxo (=O), O**R₄₅** et N**R₄₇R₄₈**, et
   - un groupe -O(CH₂)**ₙ**O- avec n représentant un nombre entier égal à 2 ou 3 (les deux oxygènes de ce groupe peuvent être attachés au même atome ou à deux atomes distincts, avantageusement, ils seront attachés au même atome, en particulier au même atome de carbone ce qui permettra dans ce cas de former un acétal cyclique),
où :
▪ **R₁₆**, **R₁₈** à R₂₀, **R₂₂** à **R₂₆**, **R₂₉** à **R₄₀**, **R₄₅** et **R₄₇** à **R₄₈** représentent, indépendamment les uns des autres, un atome d'hydrogène, un groupe (C₁-C₆)alkyle, aryle, aryl-(C₁-C₆)alkyle, hétérocycle ou hétérocycle-(C₁-C₆)alkyle, notamment un atome d'hydrogène, un groupe (C₁-C₆)alkyle, aryle, ou aryl-(C₁-C₆)alkyle, en particulier un atome d'hydrogène, ou un groupe (C₁-C₆)alkyle,
   le noyau aryle de ces groupes étant un groupe phényle et étant éventuellement substitué par un ou plusieurs groupes choisis parmi un atome d'halogène et un groupe (C₁-C₆)alkyle, et
   le noyau hétérocycle de ces groupes étant un hétérocycle à 3 à 6 chaînons, notamment à 5 ou 6 chaînons, comprenant 1 ou 2 hétéroatomes choisis parmi N et O, et étant éventuellement substitué par un ou plusieurs groupes choisis parmi un atome d'halogène, un groupe (C₁-C₆)alkyle et oxo(=O), ou
▪ **R₂₂** et **R₂₃, R₃₁** et **R₃₂, R₃₅** et **R₃₆, R₃₈** et **R₃₉, R₄₃** et **R₄₄,** et/ou **R₄₇** et **R₄₈** forment ensemble, avec l'atome d'azote qui les porte, un hétérocycle azoté à 5 ou 6 chaînons, notamment non aromatique, de préférence saturé, comprenant éventuellement 1 hétéroatome en plus de l'atome d'azote choisi parmi N et O, tel qu'un cycle pipérazine, pipéridine, morpholine ou pyrrolidine, l'hétérocycle étant éventuellement substitué par un ou plusieurs groupes choisis parmi un atome d'halogène, un groupe (C₁-C₆)alkyle, et oxo(=O).

Les groupes **R₉** et **R₁₀** pourront plus particulièrement représenter, indépendamment l'un de l'autre :
- un atome d'hydrogène ou d'halogène (par ex. Br ou I),
- un groupe CO₂**R₅₄** avec **R₅₄** représentant un atome d'hydrogène ou un groupe (C₁-C₆)alkyle, notamment un atome d'hydrogène,
- un groupe CON**R₅₅R₅₆** avec **R₅₅** et **R₅₆** formant avec l'atome d'azote qui les portent un hétérocycle azoté à 5, 6 ou 7 chaînons, comprenant 1 ou 2 hétéroatomes choisis parmi N et O dont au moins un atome d'azote, notamment non aromatique, de préférence saturé, (l'hétérocycle peut être en particulier un cycle pipérazine, pipéridine, morpholine diazépane (par ex. 1,4-diazépane) ou pyridine) éventuellement substitué par un ou plusieurs substituants choisis parmi un atome d'halogène, (C₁-C₆)alkyle, oxo (=O), O**R₂₀**, N**R₂₂R₂₃**, CO₂**R₂₅**, C(O)N**R₃₁R₃₂**, N**R₃₃**CO₂**R₃₄**, OC(O)N**R₃₅R₃₆**, N**R₃₇**CON**R₃₈R₃₉** et OCO₂**R₄₀** ; notamment choisis parmi un atome d'halogène, (C₁-C₆)alkyle, oxo (=O), O**R₂₀**, N**R₂₂R₂₃**, CO₂**R₂₅** et C(O)N**R₃₁R₃₂**,
- un groupe -**Z₁**-(CH₂)ₘ-**R₄₉** dans lequel **Z₁** représente une liaison simple, CH₂-CH₂, CH=CH, C≡C, O, S ou N**R₅₀**, notamment une liaison simple, CH₂-CH₂, C≡C, O ou N**R₅₀**; **m** représente un nombre entier compris entre 1 et 6, notamment compris entre 1 et 4 ; **R₅₀** représente un atome d'hydrogène ou un groupe (C₁-C₆)alkyle ; et **R₄₉** représente un atome d'halogène, O**R₂₀**, N**R₂₂R₂₃**, C(O)**R₂₄**, CO₂**R₂₅,** OC(O)**R₂₆**, N**R₂₉**C(O)**R₃₀**, C(O)N**R₃₁R₃₂**, N**R₃₃**CO₂**R₃₄**, OC(O)N**R₃₅R₃₆**, N**R₃₇**CON**R₃₈R₃₉** ou OCO₂**R₄₀**; en particulier O**R₂₀**, N**R₂₂R₂₃**, CO₂**R₂₅**, C(O)N**R₃₁R₃₂**, N**R₃₃**CO₂**R₃₄**, OC(O)N**R₃₅R₃₆**, N**R₃₇**CON**R₃₈R₃₉** ou OCO₂**R₄₀** ; notamment N**R₂₂R₂₃**, N**R₃₃**CO₂**R₃₄**, ou N**R₃₇**CON**R₃₈R₃₉**, ou
- un hétérocycle à 5, 6 ou 7 chaînons, notamment à 6 ou 7 chaînons, comprenant 1 ou 2 hétéroatomes choisis parmi N et O et comprenant avantageusement au moins un atome d'azote, de préférence saturé ou aromatique, notamment saturé, (l'hétérocycle peut être en particulier un cycle pipérazine éventuellement pontée (par ex. pipérazine, 2,5-diazabicyclo[4.2.0]octane, 3,8-diazabicyclo[3.2.1]octane ou 2,5-diazabicyclo[2.2.1]heptane), pipéridine, perhydropyrrolo[3,4-c]pyrrole, tétrahydropyridine ou pyrrolidine ; notamment un cycle pipérazine, pipéridine, morpholine diazépane (par ex. 1,4-diazépane) ou pyridine) éventuellement substitué par un ou plusieurs substituants choisis parmi :
   - un atome d'halogène,
   - un groupe (C₁-C₆)alkyle éventuellement substitué par un ou plusieurs groupes choisis parmi un atome d'halogène, O**R₁₆**, N**R₁₈R₁₉**, un carbocycle monocyclique en C₃ à C₆ (notamment saturé) et un hétérocycle monocyclique à 3 à 6 chaînons (notamment saturé) comprenant 1 ou 2 hétéroatomes choisis parmi N et O,
   - les groupes oxo (=O), O**R₂₀**, N**R₂₂R₂₃**, C(O)**R₂₄**, CO₂**R₂₅**, OC(O)**R₂₆**, N**R₂₉**C(O)**R₃₀,** C(O)N**R₃₁R₃₂**, N**R₃₃**CO₂**R₃₄**, OC(O)N**R₃₅R₃₆**, N**R₃₇**CON**R₃₈R₃₉** ou OCO₂**R₄₀**; et plus particulièrement les groupes oxo (=O), O**R₂₀,** N**R₂₂R₂₃**, CO₂**R₂₅**, C(O)N**R₃₁R₃₂,** N**R₃₃**CO₂**R₃₄**, OC(O)N**R₃₅R₃₆**, N**R₃₇**CON**R₃₈R₃₉** ou OCO₂**R₄₀**,
   - un carbocycle en C₃ à C₆ éventuellement substitué par un ou plusieurs groupes choisis parmi un atome d'halogène, un groupe (C₁-C₆)alkyle, oxo (=O), O**R₄₁** et N**R₄₃R₄₄**,
   - un hétérocycle à 3 à 6 chaînons, comprenant 1 ou 2 hétéroatomes choisis parmi N et O, notamment saturé ou insaturé, notamment non aromatique, de préférence saturé, éventuellement substitué par un ou plusieurs groupes choisis parmi un atome d'halogène, un groupe (C₁-C₆)alkyle, oxo (=O), O**R₄₅** et N**R₄₇R₄₈**, et
   - un groupe -O(CH₂)ₙO- avec **n** représentant un nombre entier égal à 2 ou 3 (les deux oxygènes de ce groupe peuvent être attachés au même atome ou à deux atomes distincts, avantageusement, ils seront attachés au même atome, en particulier au même atome de carbone ce qui permettra dans ce cas de former un acétal cyclique),
   où :
   ▪ **R₁₆, R₁₈** à **R₂₀, R₂₂** à **R₂₆, R₂₉** à **R₄₀, R₄₅** et **R₄₇** à **R₄₈** représentent, indépendamment les uns des autres, un atome d'hydrogène, un groupe (C₁-C₆)alkyle, aryle, ou aryl-(C₁-C₆)alkyle, notamment un atome d'hydrogène ou un groupe (C₁-C₆)alkyle,
      le noyau aryle de ces groupes étant un groupe phényle et étant éventuellement substitué par un ou plusieurs groupes choisis parmi un atome d'halogène et un groupe (C₁-C₆)alkyle, ou
   ▪ **R₂₂** et **R₂₃, R₃₁** et **R₃₂, R₃₅** et **R₃₆, R₃₈** et **R₃₉, R₄₃** et **R₄₄,** et/ou **R₄₇** et **R₄₈** forment ensemble, avec l'atome d'azote qui les porte, un hétérocycle azoté à 5 ou 6 chaînons, notamment non aromatique, de préférence saturé, comprenant éventuellement 1 hétéroatome en plus de l'atome d'azote choisi parmi N et O, tel qu'un cycle pipérazine, pipéridine, morpholine ou pyrrolidine, l'hétérocycle étant éventuellement substitué par un ou plusieurs groupes choisis parmi un atome d'halogène, un groupe (C₁-C₆)alkyle, et oxo(=O).

Selon un mode de réalisation particulier de l'invention, au moins l'un de **R₉** et **R₁₀** ne représente pas un atome d'hydrogène. Selon un autre mode de réalisation particulier de l'invention, au moins l'un de **R₉** et **R₁₀** ne représente ni un atome d'hydrogène, ni un atome d'halogène.

Selon encore un autre mode de réalisation de l'invention, l'un de **R₉** et **R₁₀** représente un atome d'hydrogène et l'autre ne représente pas un atome d'hydrogène, et notamment ne représente ni un atome d'hydrogène, ni un atome d'halogène.

Selon un mode de réalisation particulier, **Z** = O, **Y** représente un groupe C**Ry** avec **Ry** tel que défini selon l'une des définitions précédentes et en particulier avec **Y** = CH ou CF et **R₁ = R₂ = R₃ = R₄ = R₁' = R₂' = R₃' = R₄'** = H.

Les composés de formule **(I)** correspondent alors à des composés de formule **(Ia)** suivante : 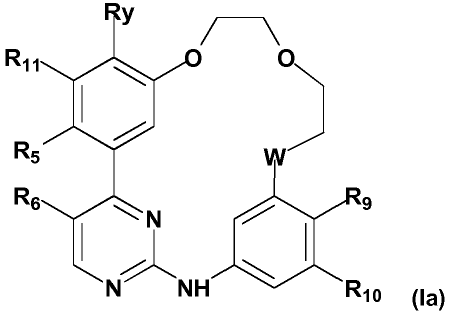 et leurs sels et/ou solvates pharmaceutiquement acceptables, dans laquelle :
- **Ry** est tel que défini précédemment et avantageusement représente un atome d'hydrogène, un atome d'halogène, un groupe (C₁-C₆)alkyle ou (C₁-C₆)halogénoalkyle ; notamment un atome d'hydrogène ou un atome d'halogène (par ex. F),
- **R₅** et **R₆** sont tels que définis selon l'une des définitions précédentes et de préférence représentent, indépendamment l'un de l'autre, un atome d'hydrogène ou un groupe (C₁-C₆)alkyle,
- **R₁₁** est tel que défini précédemment et avantageusement représente un atome d'hydrogène ou d'halogène (par ex. F), et
- **R₉** et **R₁₀** sont tels que définis selon l'un quelconque des modes de réalisation précédents.

Les composés de la présente invention pourront être choisis notamment parmi les composés **1** à **46** décrits dans les exemples ci-après, sous forme de leur base libre ou d'un de leurs sels et/ou solvates pharmaceutiquement acceptables, notamment sous forme de leur chlorhydrate.

La présente invention a également pour objet un composé de formule **(I)** tel que défini ci-dessus, pour son utilisation en tant que médicament, notamment destiné au traitement du cancer.

La présente invention concerne également l'utilisation d'un composé de formule **(I)** tel que défini ci-dessus, pour la fabrication d'un médicament, notamment destiné au traitement du cancer. Egalement décrite est une méthode de traitement du cancer, comprenant l'administration à une personne en ayant besoin d'une dose efficace d'un composé de formule **(I)** tel que défini ci-dessus.

Le cancer pourra être plus particulièrement dans ce cas un cancer du côlon, un cancer du sein, un cancer du rein, un cancer du foie, un cancer du pancréas, un cancer de la prostate, un glioblastome, un cancer du poumon, un neuroblastome, une tumeur myofibroblastique inflammatoire, un lymphome, une leucémie, un syndrome myélo-displasique, une myélo-fibrose, un cancer de l'ovaire, un cancer de la tête et du cou.

La présente invention concerne également une composition pharmaceutique comprenant au moins un composé de formule **(I)** tel que défini ci-dessus, et au moins un excipient pharmaceutiquement acceptable.

Les compositions pharmaceutiques selon l'invention peuvent être formulées notamment pour une administration orale ou par injection, lesdites compositions étant destinées aux mammifères, y compris l'homme.

L'ingrédient actif peut être administré sous formes unitaires d'administration, en mélange avec des supports pharmaceutiques classiques, aux animaux ou aux êtres humains.

Les formes unitaires appropriées d'administration par voie orale comprennent les comprimés, les gélules, les poudres, les granules et les solutions ou suspensions orales.

Lorsque l'on prépare une composition solide sous forme de comprimés, on mélange l'ingrédient actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant l'ingrédient actif avec un diluant et en versant le mélange obtenu dans des gélules molles ou dures.

Une préparation sous forme de sirop ou d'élixir peut contenir l'ingrédient actif conjointement avec un édulcorant, un antiseptique, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, de même qu'avec des correcteurs du goût ou des édulcorants.

Pour une administration par injection, on utilise des suspensions aqueuses, des solutions salines isotoniques ou des solutions stériles et injectables qui contiennent des agents de dispersion et/ou des agents mouillants pharmacologiquement compatibles.

Le principe actif peut être formulé également sous forme de microcapsules, éventuellement avec un ou plusieurs supports additifs.

Les composés de l'invention en tant que principes actifs peuvent être utilisés à des doses comprises entre 0,01 mg et 1000 mg par jour, donnés en une seule dose une fois par jour ou administrés en plusieurs doses tout au long de la journée, par exemple deux fois par jour en doses égales. La dose administrée par jour est avantageusement comprise entre 5 mg et 500 mg, encore plus avantageusement entre 10 mg et 200 mg. Il peut être nécessaire d'utiliser des doses sortant de ces gammes ce dont l'homme du métier peut se rendre compte lui-même.

Les compositions pharmaceutiques selon l'invention peuvent comprendre en outre au moins un autre principe actif, tel qu'un agent anticancéreux.

La présente invention a également pour objet une composition pharmaceutique comprenant :
(i) au moins un composé de formule **(I)** tel que défini ci-dessus, et
(ii) au moins un autre principe actif, tel qu'un agent anticancéreux,
en tant que produit de combinaison, pour une utilisation simultanée, séparée ou étalée dans le temps.

La présente invention concerne également une composition pharmaceutique telle que définie ci-dessus pour son utilisation en tant que médicament, notamment destiné au traitement du cancer.

La présente invention concerne également une méthode de traitement du cancer, comprenant l'administration à une personne en ayant besoin d'une dose efficace d'une composition pharmaceutique telle que définie ci-dessus.

La présente invention a également pour objet des procédés de préparation des composés de formule (I) selon l'invention.

La présente invention concerne ainsi un premier procédé de préparation d'un composé de formule **(I)** comprenant la réaction de couplage entre :
un composé de formule **(II)** suivante : 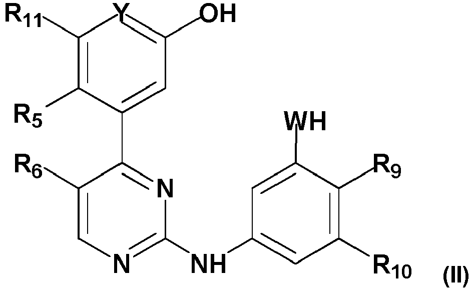 pour laquelle **W, Y, R₅, R₆, R₉, R₁₀ et R₁₁** sont tels que définis précédemment, et un composé de formule **(III)** suivante : 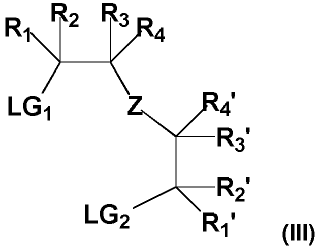 pour laquelle **Z, R₁, R₂, R₃, R₄, R₁', R₂', R₃'** et **R₄'** sont tels que définis précédemment et **LG₁** et **LG₂** représentent chacun, indépendamment l'un de l'autre, un groupe partant.

Par « groupe partant », on entend, au sens de la présente invention, un groupement chimique qui peut être facilement déplacé par un nucléophile lors d'une réaction de substitution nucléophile, le nucléophile étant dans le cas présent un alcool ou un thiol. Un tel groupe partant peut être plus particulièrement un atome d'halogène tel qu'un atome de chlore ou de brome ou un sulfonate. Le sulfonate peut être en particulier un groupe -OSO₂-**R₅₁** avec **R₅₁** représentant un groupe (C₁-C₆)alkyle, aryle, aryl-(C₁-C₆)alkyle ou (C₁-C₆)alkyl-aryle, ledit groupe étant éventuellement substitué par un ou plusieurs atomes de d'halogène tels que des atomes de fluor. Le sulfonate peut être en particulier un mésylate (-OS(O₂)-CH₃), un triflate (-OS(O)₂-CF₃) ou encore un tosylate (-OS(O)₂-(*p*-Me-C₆H₄)).

Les groupes et **LG₁** et **LG₂** représenteront plus particulièrement un atome d'halogène tel qu'un brome.

La réaction de couplage (ou de macrocyclisation) sera réalisée avantageusement en présence d'une base telle que le carbonate de potassium ou de sodium. Le diméthylformamide pourra être utilisé comme solvant de réaction.

Un tel procédé est plus particulièrelment utilisé lorsque **W** = O.

Un tel procédé est illustré plus en détails sur les **schémas 1, 2** et **3** suivants et dans la partie expérimentale ci-après.

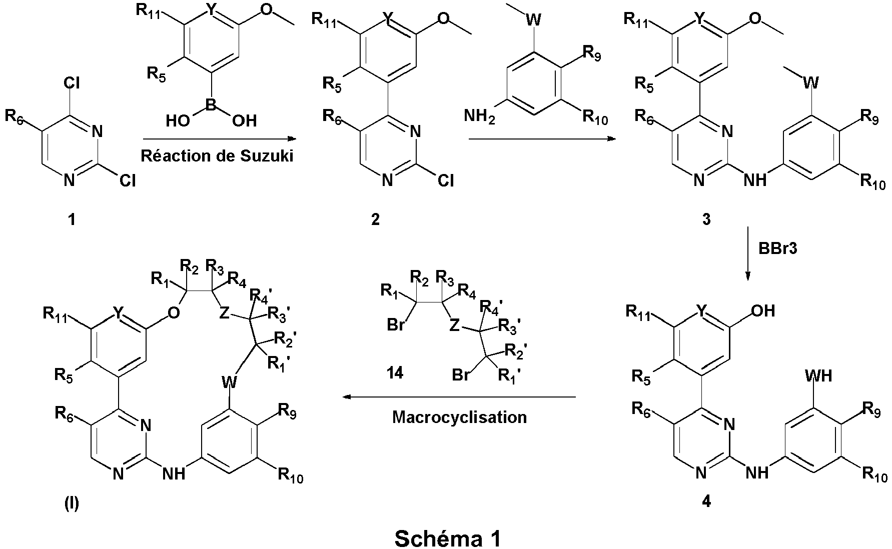

La première étape est une réaction de couplage catalytique telle qu'une réaction de Suzuki entre un boronate, sous une forme acide (tel qu'illustrée) ou ester, et un dérivé de 2,4-dichloropyrimidine éventuellement substitué. Dans ce cas, ces composés sont mis en présence d'un catalyseur au palladium tel que le Pd(dppf)Cl₂ ou le Pd(PPh₃)₄, d'une base organique telle que la triéthylamine ou un alcoolate (en particulier (C₁-C₆)alkyl-OM avec M = Na, K ou Li), ou inorganique telle que le carbonate de sodium, de potassium ou de césium. Le toluène, le benzène, le tétrahydrofurane, le dioxane ou des mélanges de ceux-ci pourront être utilisés comme solvants. Les températures de réaction préférées sont comprises entre 20°C et 100°C.

La transformation des composés **2** en composés **3** peut être réalisée par une réaction de substitution nucléophile aromatique. Dans ce cas, les nucléophiles utilisés sont des anilines fonctionnalisées qui sont mises en réaction en présence d'un acide telle que l'acide chlorhydrique, de préférence dans un solvant polaire tel que le n-butanol. Le cas échéant, ces réactions peuvent être conduites dans un réacteur à micro-ondes, notamment dans un solvant polaire tel que la N-méthyl-2-pyrrolidinone. Les températures de réaction préférées sont comprises entre 20°C et 150°C.

Les composés **3** peuvent être transformés en composés **4** par une réaction de déméthylation en présence de BBr₃, notamment dans un solvant anhydre tel que le diclorométhane, de préférence à une température comprise entre -78°C et 100°C. Les composés **4** sont transformés en composés de formule générale **(I)** par une réaction de macrocyclisation en présence de composés dibromés **14.**

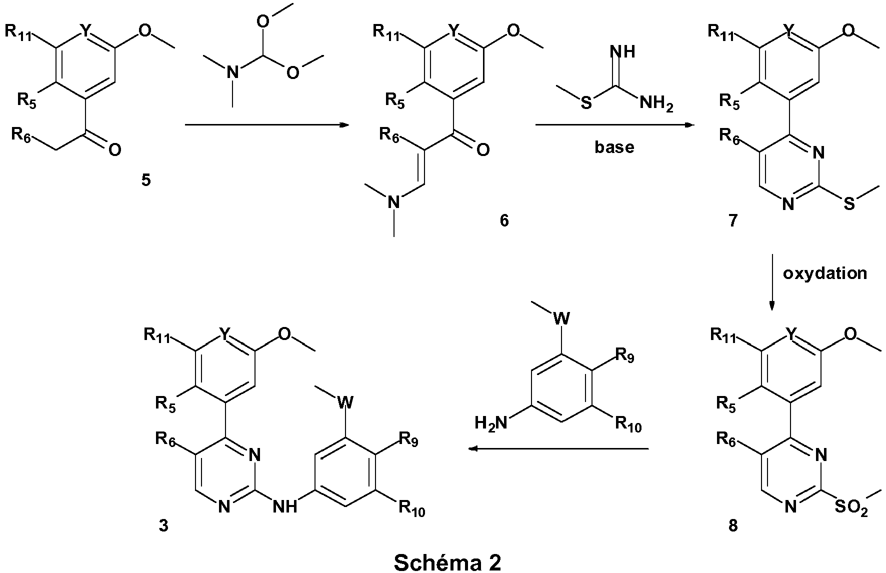

Le **schéma 2** présente une deuxième voie de synthèse des composés **3.**

La réaction de l'acétophenone **5** diversement substituée avec le N,N-diméthylformamide diméthyl acétal permet de donner accès aux composés **6.** Ces réactions sont typiquement effectuées sans solvant ou dans un solvant polaire anhydre tel que le diméthylformamide, notamment à une température comprise entre 0°C et 170°C.

La deuxième étape de cyclisation entre le composé **6** et l'hémisulfate de S-méthylisothiourée est typiquement conduite en présence d'une base organique telle que la triéthylamine, l'acétate de potassium ou de sodium ou un alcoolate, ou inorganique telle que le carbonate de sodium, de potassium ou de césium. Cette réaction pourra être réalisée à une température comprise entre 20°C et 200°C, notamment dans un solvant polaire, tel que le N,N-diméthylformamide, ou sans solvant dans un réacteur à micro-ondes.

Les composés **7** obtenus sont soumis à une oxydation de leur fonction thiométhoxy, typiquement par l'utilisation de m-CPBA, d'oxone ou de tout autre agent oxydant équivalent, pour conduire à la formation de la sulfone correspondante **8.**

Ces composés **8** sont ensuite engagés dans une réaction de substitution nucléophile en présence d'une aniline pour former les composés **3.**

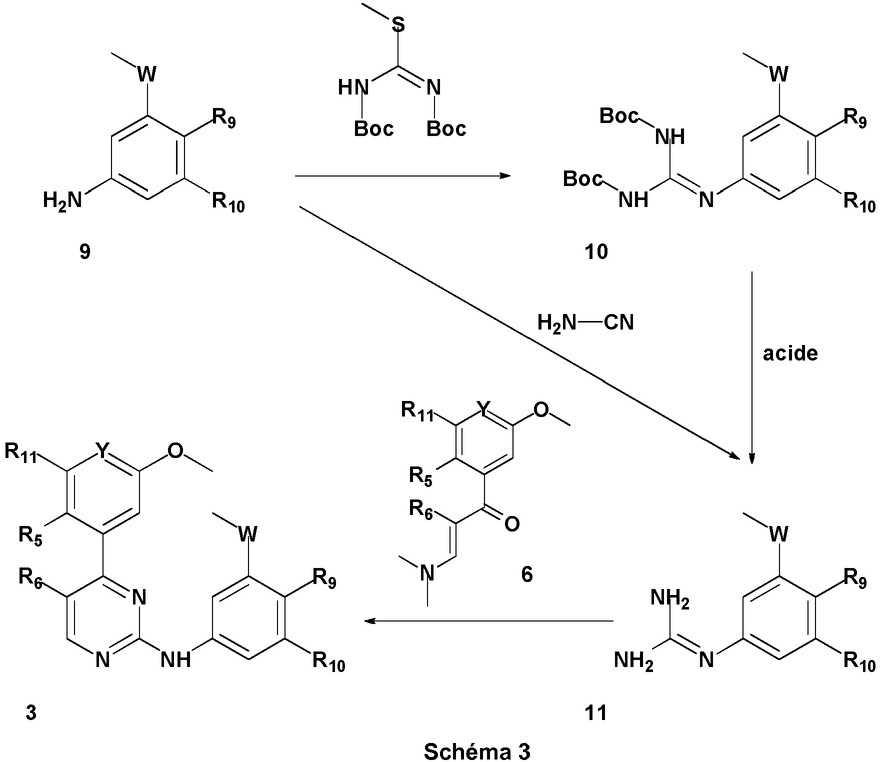

Le **schéma 3** présente une troisième voie de synthèse des composés **3.**

Les anilines **9** sont transformées en dérivés guanidines protégés **10** par l'une des méthodes habituelles bien connues de l'homme de l'art, dérivés **10** qui sont déprotégés en conditions acides pour donner les guanidines déprotégées **11,** par exemple par l'utilisation d'acide trifluoroacétique, notamment pendant des périodes variant typiquement de 1 à 3 jours, notamment à des températures variant entre 0°C et 40°C.

Les anilines **9** peuvent également être transformées directement en guanidines déprotégées **11** par réaction avec du cyanamide, en présence d'un acide tel que HCl, H₂SO₄ ou HCOOH, notamment en absence de solvant ou en présence d'un solvant tel que le toluène ou l'éthanol, notamment à une température comprise entre 20°C et 100°C. Cette réaction peut être réalisée par les méthodes et techniques bien connues de l'homme de l'art.

Les guanidines **11** ainsi obtenues sont soumises à une réaction de condensation avec les composés **6** pour former les composés **3** correspondants.

La présente invention concerne également un deuxième procédé de préparation d'un composé de formule **(I)** comprenant la réaction de cyclisation d'un composé de formule **(VIa)** ou **(VIb)** suivante : 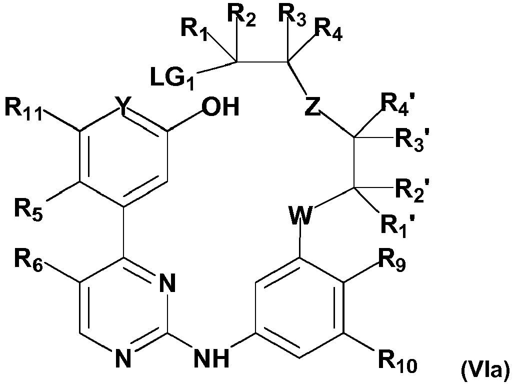 ou 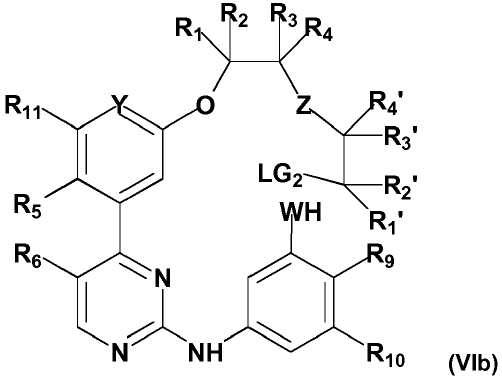 pour lesquelles **W, Y, Z, R₁** à **R₆, R₉** à **R₁₁** et **R₁'** à **R₄'** sont tels que définis précédemment, et **LG₁** et **LG₂** représentent chacun, indépendamment l'un de l'autre, un groupe partant, tel qu'un atome d'halogène et en particulier un atome de brome.

La réaction de cyclisation sera réalisée avantageusement en présence d'une base telle que l'hydroxyde de potassium ou de sodium. Le tétrahydrofurane pourra être utilisé comme solvant de réaction.

Un tel procédé est plus particulièrement utilisé lorsque **W** = S.

Un tel procédé est illustré plus en détails sur le **schéma 4** suivant et dans la partie expérimentale ci-après.

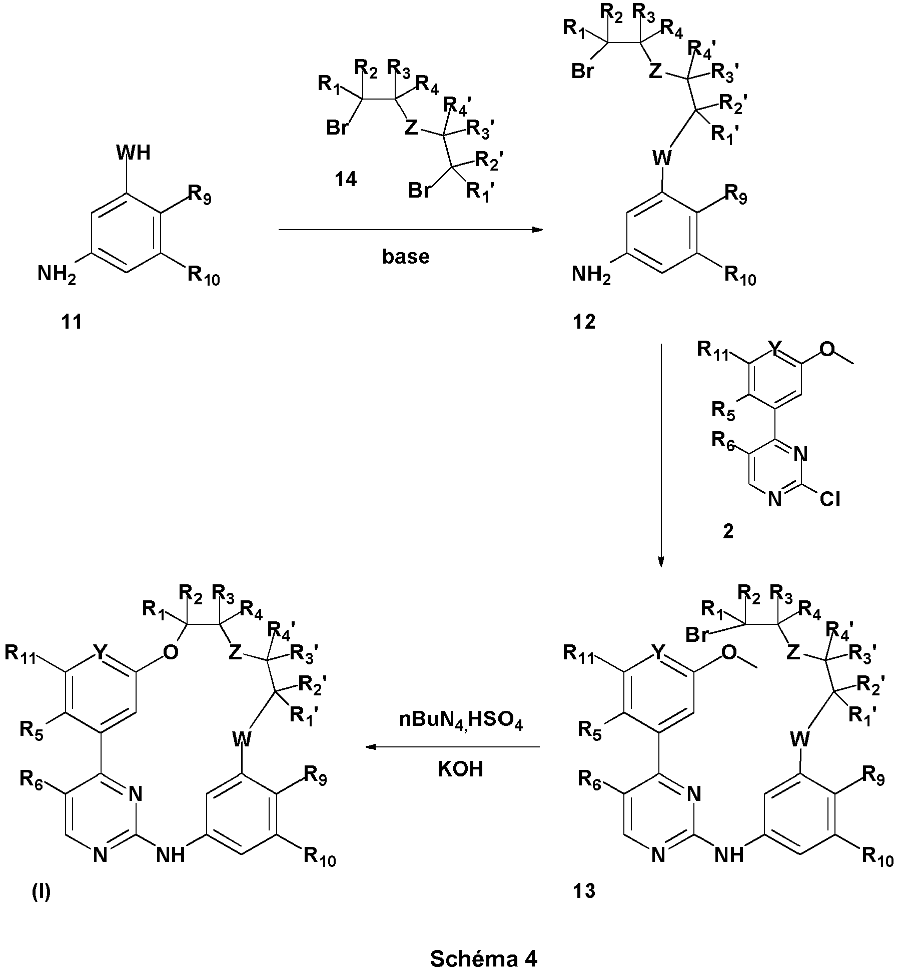

Le composé **11** est transformé en composé **12** par réaction avec le dérivé dibromé **14.** Cette réaction peut être réalisée en présence d'une base organique ou inorganique telle que par exemple Et₃N, iPr₂NEt, NaH, pyridine, Cs₂CO₃, K₂CO₃ ou Na₂CO₃, éventuellement en présence d'un sel comme catalyseur qui peut être Kl, Bu₄NI, Cul Lil, AgBF₄, AgClO₄, Ag₂CO₃, KF, Bu₄NF ou CsF ou bien éventuellement en présence d'agent de transfert de phase tel que nBu₄N, HSO₄. La réaction poura être réalisée dans un solvant anhydre polaire tel que le tétrahydrofurane, le diméthylformamide, le diméthylsulfoxyde, l'acétone ou un mélange de ceux-ci, notamment à une température comprise entre -20°C et 140°C. La réaction peut également être réalisée dans un « tube scellé ou à vis » chauffé par énergie thermique ou énergie micro-onde, à des températures comprises entre 80°C et 180°C.

Les composés **12** peuvent être transformés en composés **13** par une réaction de substitution nucléophile aromatique. La réaction peut être réalisée en présence d'un acide telle que l'acide chlorhydrique, notamment dans un solvant polaire tel que le n-butanol. Le cas échéant, ces réactions peuvent être conduites dans un réacteur à micro-ondes, par exemple dans un solvant polaire tel que la N-méthyl-2-pyrrolidinone. Typiquement, les conditions opératoires préférées impliquent des températures comprises entre 20°C et 150°C.

Le composé **13** est ensuite cyclisé pour donner un composé de formule générale **(I)** selon des conditions réactionnelles similaires à la transformation du composé **11** en composé **12.**

La présente invention concerne également un troisième procédé de préparation d'un composé de formule **(I)** pour lequel **R₉** et/ou **R₁₀** représente un groupe (C₁-C₆)alcoxy éventuellement substitué, (C₁-C₆)thioalcoxy éventuellement substitué ou N**R₁₄R₁₅** ou un hétérocycle éventuellement substitué comprenant un hétéroatome directement lié au noyau phényle, comprenant le couplage entre un composé de formule **(IVa)** ou **(IVb)** suivante : 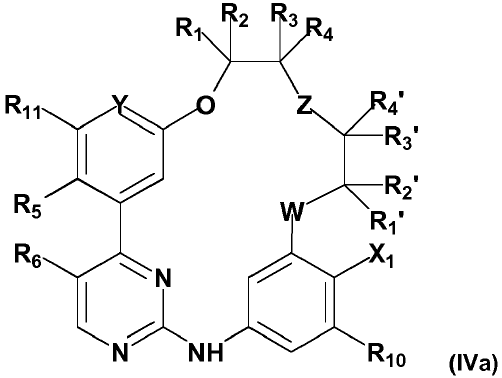 ou 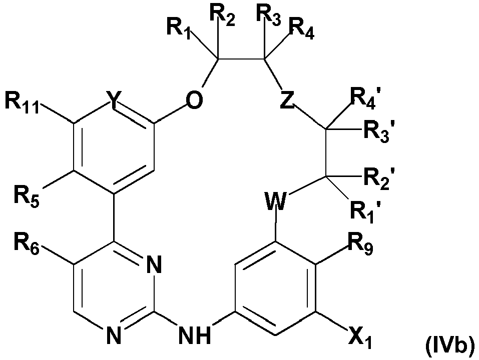 pour lesquelles **W, Y, Z, R₁** à **R₆, R₉** à **R₁₁ et R₁'** à **R₄'** sont tels que définis précédemment et **X₁** représente un atome d'halogène tel que Br, Cl ou I, notamment Br, et respectivement un composé de formule **R₉**H ou **R₁₀**H pour lesquels **R₉** et **R₁₀** sont tels que définis précédemment.

Cette réaction peut être réalisée en présence d'une base organique ou inorganique, telle que Et₃N, iPr₂NEt, NaH, pyridine, Cs₂CO₃, Na₂CO₃ ou K₂CO₃, éventuellement en présence d'un sel comme catalyseur tel que Kl, Cul, BU₄NI, Lil, AgBF₄, AgClO₄, Ag₂CO₃, KF, Bu₄NF ou CsF. Le solvant utilisé sera de préférence un solvant anhydre polaire tel que le tétrahydrofurane, le diméthylformamide, le diméthylsulfoxyde, l'acétone ou un mélange de ceux-ci. La réaction pourra avantageusement être réalisée à une température comprise entre -20°C et 140°C. Le choix des conditions expérimentales et des réactifs pour réaliser cette réaction est bien évidemment dépendant de la nature des nucléophiles **R₉**H et **R₁₀**H et sera réalisé selon les méthodes et techniques bien connues de l'homme de métier.

La réaction peut également être réalisée dans un « tube scellé ou à vis » chauffé par énergie thermique ou énergie micro-onde, notamment à des températures comprises entre 80°C et 180°C selon la référence (J. Org. Chem. 2009, 74, 5075-5078).

Cette réaction peut aussi être réalisée par un couplage catalytique tel que décrit dans la référence (Org. Lett. 2002, 17, 2885-2888). Cette réaction est réalisée en présence d'une quantité catalytique d'un complexe au palladium tel que le (dppf)₂PdCl₂.CH₂Cl₂. La réaction de couplage est conduite avantageusement à des températures comprises entre 25°C et 100°C. Le solvant utilisé sera de préférence un solvant polaire aprotique tel que le tétrahydrofurane ou le dioxane.

La présente invention concerne également un quatrième procédé de préparation d'un composé de formule **(I),** pour lequel **R₉** et/ou **R₁₀** représente un groupe (C₁-C₆)alkyle éventuellement substitué, (C₂-C₆)alcényle éventuellement substitué ou (C₂-C₆)alcynyle éventuellement substitué, un carbocycle éventuellement substitué ou un hétérocycle éventuellement substitué lié au noyau phényle au moyen d'un atome de carbone, comprenant le couplage entre un composé de formule **(Va)** ou **(Vb)** suivante : 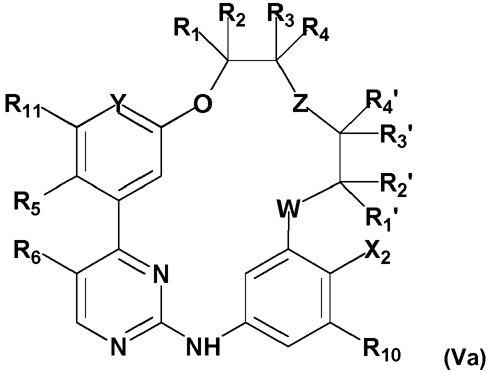 ou 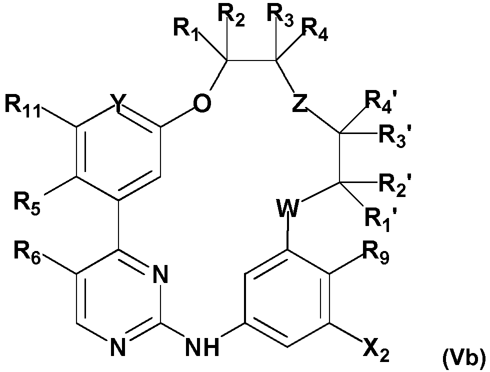 pour lesquelles **W, Y, Z, R₁** à **R₆, R₉** à **R₁₁** et **R₁'** à **R₄'** sont tels que définis précédemment et **X₂** représente Br, Cl, I ou OTf (OSO₂CF₃),
et respectivement un composé de formule **R₉-**B**R₅₂R₅₃** ou **R₁₀-** B**R₅₂R₅₃** pour lesquels **R₉** et **R₁₀** sont tels que définis ci-dessus et **R₅₂** et **R₅₃** représentent, indépendamment l'un de l'autre, un groupe OH, (C₁-C₆)alkyle ou (C₁-C₆)alcoxy ou **R₅₂** et **R₅₃** forment ensemble une chaîne **-X₃-** ou -O-**X₃**-O- pour laquelle **X₃** représente un groupe divalent hydrocarboné comprenant 2 à 15, notamment 2 à 10, atomes de carbone.

Les conditions réactionnelles d'un tel couplage sont bien connues de l'homme du métier puisqu'il s'agit d'un couplage de Suzuki.

Cette réaction est avantageusement réalisée en présence d'un catalyseur à base de palladium, par exemple l'acétate de palladium, le tétrakistriphénylphosphine palladium (0) ou le tris(dibenzylidèneacétone) dipalladium (0). Une phosphine telle que la triphénylphosphine ou la tricyclohexylphosphine pourra également être présente.

Une base organique ou inorganique pourra être présente, telle que un alcoolate (en particulier (C₁-C₆)alkyl-OM avec M = Na, K ou Li), NMP (N-méthyl-morpholine), Et₃N, iPr₂NEt, K₃PO₄, NaH, Cs₂CO₃, Na₂CO₃ ou K₂CO₃.

Un solvant polaire pourra être utilisé tel que le tétrahydrofurane, le diméthylformamide, l'acétonitrile, l'acétone, la méthyléthylcétone, l'éthanol, l'éther diméthylique, le dioxane, l'eau ou un mélange de ceux-ci. La réaction pourra être avantageusement réalisée à une température comrpise entre 20°C et 140°C.

Le groupe -B**R₅₂R₅₃** pourra être par exemple un groupe -B(OH)₂, -B((C₁-C₆)alkyl)₂, -B(O(C₁-C₆)alkyl)₂ (par ex. -B(OiPr)₂), 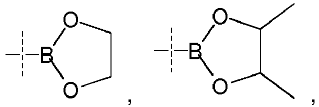 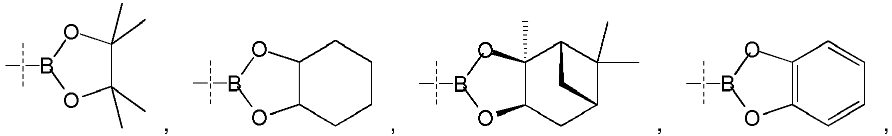 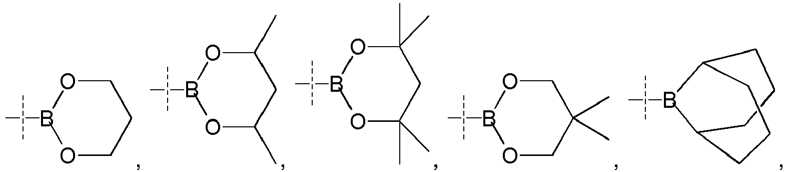 etc.

La présente invention concerne aussi un cinquième procédé de préparation d'un composé de formule **(I)** pour lequel **R₉** et/ou **R₁₀** représente -**Z₁**-(CH₂)**ₘ**-**R₄₉** avec **Z₁** représentant CH₂-CH₂, CH=CH ou C=C, comprenant les étapes suivantes :
(1) couplage de Sonogashira entre un composé de formule **(Va)** ou **(Vb)** tel que défini précédemment
   et un composé de formule HC≡C-(CH₂)**ₘ-R₄₉** pour lequel **m** et **R₄₉** sont tels que définis précédemment,
   pour donner un composé de formule **(I)** pour lequel **R₉** ou **R₁₀** représente -C≡C-(CH₂)**ₘ-R₄₉**, et
(2) éventuellement réduction de la fonction alcyne du composé de formule **(I)** obtenu à l'étape précédente pour donner un composé de formule **(I)** pour lequel **R₉** ou **R₁₀** représente -CH=CH-(CH₂)**ₘ-R₄₉** ou -(CH₂)_{**m**+2}-**R₄₉**.

### Etape (1):

Le couplage de Sonogashira est une réaction bien connue de l'homme du métier qui saura en déterminer les conditions réactionnelles. Elle est décrite notamment dans l'article de Sonogashira et al. dans Tetrahedron Lett. 1975, 16, 4467-4470.

Ce couplage implique une réaction entre un dérivé acétylénique et un halogénure ou un triflate d'aryle catalysée par des complexes de palladium et de cuivre.

Une telle réaction est typiquement effectuée sous atmosphère inerte, en présence d'une quantité catalytique d'un complexe de palladium (par exemple PdCl₂(PPh₃)₂ ou Pd(PPh₃)₄), d'une quantité catalytique d'un sel de cuivre (par exemple Cul), et d'une base qui peut être organique, telle que la triéthylamine ou la DIPEA (diisopropyléthylamine), ou inorganique, telle que le carbonate de sodium, de potassium ou de césium. Les conditions opérationnelles incluent généralement des températures de réaction comprises entre 20°C et 45°C, notamment dans des solvants incluant le diméthylformamide, le tétrahydrofurane, le dioxane, l'éther diéthylique ou un mélange de ceux-ci.

### Etape (2):

La réaction de réduction de la triple liaison de la fonction alcyne C≡C pour donner une double liaison CH=CH ou une liaison simple CH₂-CH₂ est bien connue de l'homme du métier qui saura en déterminer les conditions réactionnelles.

Cette réduction pourra être effectuée par exemple par l'hydrogène en présence d'un catalyseur par exemple de type palladium sur charbon, notamment dans un solvant courant de type éthanol, pour obtenir une liaison simple CH₂-CH₂.

La présente invention concerne enfin un sixième procédé de préparation d'un composé de formule **(I)** pour lequel **R₉** et/ou **R₁₀** représente un groupe CO₂**R₅₄** ou CON**R₅₅R₅₆**, qui comprend au moins l'une des étapes suivantes :
(a) pour obtenir un composé de formule **(I)** pour lequel **R₉** et/ou **R₁₀** représente un groupe CO₂H, la réaction d'un composé de formule **(I)** pour lequel **R₉** et/ou **R₁₀** représente un atome d'halogène avec du CO₂ ;
(b) pour obtenir un composé de formule **(I)** pour lequel **R₉** et/ou **R₁₀** représente un groupe CO₂**R₅₄** avec **R₅₄** ≠ H, la réaction de substitution d'un composé de formule **(I)** pour lequel **R₉** et/ou **R₁₀** représente un groupe CO₂H, éventuellement obtenu selon l'étape (a), éventuellement sous une forme activée, avec un alcool de formule **R₅₄**OH ;
(c) pour obtenir un composé de formule **(I)** pour lequel **R₉** et/ou **R₁₀** représente un groupe CON**R₅₅R₅₆**, la réaction de substitution d'un composé de formule **(I)** pour lequel **R₉** et/ou **R₁₀** représente un groupe CO₂H, éventuellement obtenu selon l'étape (a), éventuellement sous une forme activée, avec une amine de formule HN**R₅₅R₅₆**.

### Etape (a):

Cette réaction sera avantageusement réalisée en présence d'une base telle que ((C₁-C₆)alkyl)Li (par ex. BuLi). Le tétrahydrofurane pourra être utilisé comme solvant de réaction. La réaction sera réalisée de préférence à une température inférieure à 0°C, notamment inférieure à -50°C, par ex. à environ -78°C.

### Etapes (b) et (c) :

Par « forme activée » du groupe CO₂H (acide carboxylique), on entend, au sens de la présente invention, un groupe acide carboxylique dans lequel le motif OH de la fonction COOH a été remplacé par un groupe partant activé (LG) permettant le couplage du groupe CO₂H sous une forme activée avec une fonction hydroxyle OH ou amino NH par formation d'une liaison ester (C(O)-O) ou amide (C(O)-N) et libération du composé LG-H. Les formes activées peuvent être des esters activés, des amides activés, des anhydrides ou des halogénures d'acyles tels que des chlorures d'acyles. Les esters activés incluent des dérivés formés par la réaction du groupe acide carboxylique avec le N-hydroxybenzotriazole ou le N-hydroxysuccinimide.

Les réactions des étapes (b) et (c) sont bien connues de l'homme du métier.

La réaction de susbtitution avec l'alcool sera réalisée de préférence avec un composé de formule **(I)** portant une fonction acide carboxylique CO₂H sous forme activée, notamment sous forme d'un chlorure d'acyle.

La réaction de substitution avec l'amine pourra être réalisée avec un composé de formule **(I)** portant une fonction acide carboxylique CO₂H sous forme activée, notamment sous forme d'un chlorure d'acyle, ou portant une fonction acide carboxylique CO₂H non activée dans des conditions de couplage peptidique bien connues de l'homme du métier.

Le couplage peptidique pourra ainsi être réalisé en présence d'un agent de couplage, tel que le diisopropylcarbodiimide (DIC), le dicyclohexylcarbodiimide (DCC), le chlorhydrate de 1-(3-diméthylaminopropyl)-3-éthylcarbodiimide (EDC), le carbonyldiimidazole (CDI), le 2-(1H-benzotriazole-1-yl)-1,1,3,3-tetraméthyluronium hexafluorophosphate (HBTU), le 2-(IH-benzotriazole-1-yl)-1,1,3,3-tetraméthyluronium tetrafluoroborate (TBTU), le O-(7-azobenzotriazol-1-yl)-1,1,3,3-tétraméthyluronium hexafluorophosphate (HATU), le (benzotriazol-1-yloxy)tripyrrolodinophosphonium hexafluorophosphate (PyBOP) ou l'anhydride propylphosphonique, éventuellement associé à un auxiliaire de couplage tel que le N-hydroxy succinimide (NHS), le N-hydroxy benzotriazole (HOBt), le 3,4-dihydro-3-hydroxy-4-oxo-1,2,3-benzotriazole (HOOBt), le l-hydroxy-7-azabenzotriazole (HAt), le N-hydroxysylfosuccinimide (sulfo NHS), la diméthylaminopyridine (DMAP), la diisopropyléthylamine (DIEA) ou la N-méthylmorpholine (NMM). Notamment, il pourra être réalisé en présence d'anhydride propylphosphonique et d'une base telle que la DIEA. Cette réaction pourra également être réalisée sous micro-ondes.

Les six procédés généraux décrits ci-dessus pourront être complétés, le cas échéant, par toutes manipulations standards décrites dans la littérature, connues de l'homme de métier ou bien encore exemplifiées dans la partie expérimentale, notamment par des réactions de fonctionnalisation et/ou de protection/déprotection additionnelles.

Une étape ou des étapes additionnelles de salification et/ou de solvatation pourront être réalisées à la fin de ces trois procédés afin d'obtenir un sel et/ou solvate pharmaceutiquement acceptable du composé de formule **(I).**

L'étape de salification pourra être réalisée dans des conditions bien connues de l'homme du métier, en présence d'un acide ou d'une base pharmaceutiquement acceptable.

Lorsque le composé de formule **(I)** se trouve sous une forme solvatée, cette solvatation a généralement lieu dans la dernière étape du procédé, le solvant de la forme solvatée étant dans ce cas le solvant du milieu réactionnel.

Le composé de formule **(I)** obtenu par l'un de ces six procédés mentionnés ci-dessus pourra être séparé du milieu réactionnel par des méthodes bien connues de l'homme du métier, comme par exemple par extraction, évaporation du solvant ou encore par précipitation et filtration.

Le composé de formule **(I)** pourra être par ailleurs purifié si nécessaire par des techniques bien connues de l'homme du métier, comme par recristallisation si le composé est cristallin, par distillation, par chromatographie sur colonne sur gel de silice ou encore par chromatographie liquide haute performance (HPLC).

L'invention est illustrée par les exemples non limitatifs qui suivent.

### EXEMPLES

### 1. Synthèse des composés selon l'invention

Les abréviations suivantes ont été utilisées :
- DMSO: : Diméthylsulfoxyde
- IE: : Impact électronique
- LCMS: : Chromatographie liquide couplée à un spectromètre de masse
- RMN: : Résonance magnétique nucléaire

### Composé 1 :

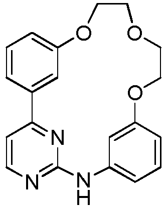

### Etape 1 : 3-(2-chloropyrimidin-4-yl)phénol (intermédiaire 1)

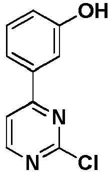

A 5 g (33,6 mmol) de 2,4-dichloropyrimidine dans 250 mL de tétrahydrofurane (THF) anhydre est additionné 4,41g (32 mmol) d'acide (3-hydroxyphényl)boronique. Le milieu réactionnel est agité à température ambiante durant 10 minutes puis est additionné à température ambiante 8,47 g (80 mmol) de carbonate de sodium dissous dans 20 mL d'eau puis 462 mg (1,59 mmol) de tétrakis(triphénylphosphine)palladium(0). Le milieu réactionnel est agité à 90°C pendant 16 heures. De l'acétate d'éthyle est additionné et la phase organique est lavée avec une solution saturée de chlorure de sodium, séchée sur sulfate de magnésium et filtrée. Le filtrat est évaporé et le résidu purifié par chromatographie sur colonne de silice à l'aide du Companion® (éluant : cyclohexane/acétate d'éthyle 0 à 10%) pour conduire à 1,54 g (23%) de 3-(2-chloropyrimidin-4-yl)phénol sous forme d'un solide blanc.
LCMS (IE, m/z) : (M+1) 207,62
RMN 1H : dH ppm (400 MHz, DMSO) : 9,82 (1H, s, OH), 8,79-8,80 (1H, d, CHarom), 8,06-8,08 (1H, d, CHarom), 7,61-7,62 (2H, m, CHarom), 7,35-7,39 (1H, d, CHarom), 6,99-7,01 (1H, t, CHarom)

### Etape 2 : 3-(2-((3-hydroxyphényl)amino)pyrimidin-4-yl)phénol (intermédiaire 2)

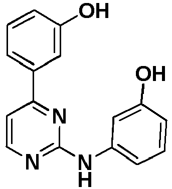

Dans un réacteur micro-ondes sont mélangés 150 mg (0,726 mmol) ed 3-(2-chloropyrimidin-4-yl)phénol, 103 mg (0,944 mmol) de 3-aminophénol et 0,5 mL de N-méthyl-2-pyrrolidinone. Le mélange réactionnel est porté à 150°C pendant 15 minutes. Après retour à température ambiante, ce dernier est hydrolysé par addition d'eau et extrait à l'acétate d'éthyle. La phase organique est séchée sur sulfate de sodium, filtrée et concentrée. Le résidu obtenu est purifié par chromatographie sur gel de silice (éluant : dichlorométhane/méthanol : 95/5) pour conduire à 129 mg (63%) de 3-(2-((3-hydroxyphényl)amino)pyrimidin-4-yl)phénol sous forme d'un solide jaune.
LCMS (IE, m/z) : (M+1) 280,29
RMN 1H : dH ppm (400 MHz, DMSO) : 9,68 (1H, s, OH), 9,50 (1H, s, NH), 9,24 (1H, s, OH), 8,50-8,51 (1H, d, CHarom), 7,54-7,58 (2H, m, CHarom), 7,28-7,35 (4H, m, CHarom), 7,07-7,09 (1H, m, CHarom), 6,93-6,95 (1H, m, CHarom), 6,37-6,39 (1H, d, CHarom)

### Etape 3 : Composé 1

A une solution sous agitation de 47,7 mg (0,171 mmol) de 3-(2-((3-hydroxyphényl)amino)pyrimidin-4-yl)phénol dans 12,5 mL de *N*,*N*-diméthylformamide est ajouté 54,3 mg (0,393 mmol) de carbonate de potassium puis 21,47 µl (0,171 mmol) de 1-bromo-2-(2-bromoéthoxy)éthane dans 0,2 mL de *N*,*N*-diméthylformamide pendant une heure. Le mélange réactionnel est agité à 80°C pendant 5 heures. Après retour à température ambiante, le solvant est évaporé, de l'eau est additionnée et le solide formé est filtré et séché sous vide pour conduire à 5,3 mg (9%) de composé 1 sous forme d'une poudre beige.
LCMS (IE, m/z) : (M+1) 350,38
RMN 1H : dH ppm (400 MHz, DMSO) : 9,83 (1H, s, NH), 8,68 (1H, m, CHarom), 8,58-8,59 (1H, d, CHarom), 8,29 (1H, s, CHarom), 7,73-7,75 (1H, m, CHarom), 7,42-7,50 (2H, m, CHarom), 7,10-7,21 (2H, m, CHarom), 6,84-6,85 (1H, m, CHarom), 6,53-6,55 (1H, m, CHarom), 4,31-4,35 (2H, t, CH₂), 4,04 (2H, m, CH₂), 3,93 (4H, m, CH₂).

### Composé 2 :

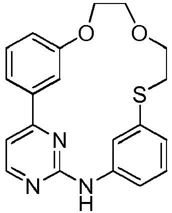

### Etape 1 : 3-((2-(2-bromoéthoxy)éthyl)thio)aniline (intermédiaire 3)

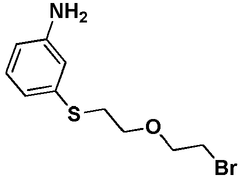

A une solution sous agitation de 3 g (23,9 mmol) de 3-aminobenzènethiol dans 30 mL de *N*,*N*-diméthylformamide est ajouté 3,97 g (28 mmol) de carbonate de potassium puis 5,56 g (23,9 mmol) de 1-bromo-2-(2-bromoéthoxy)éthane. Le mélange réactionnel est agité à 25°C pendant 4 heures. Ce dernier est hydrolysé par addition d'eau et extrait à l'acétate d'éthyle. La phase organique est séchée sur sulfate de sodium, filtrée et concentrée. Le résidu obtenu est purifié par chromatographie sur gel de silice (éluant : acétate d'éthyle/cyclohexane : 70/30) pour conduire à 1,4 g (21%) de 3-((2-(2-bromoéthoxy)éthyl)thio)aniline sous forme d'une huile.
LCMS (IE, m/z): (M+1) 277,19
RMN 1H: dH ppm (400 MHz, DMSO) : 7,01-7,05 (1H, s, CHarom), 6,69-6,72 (1H, m, CHarom), 6,61-6,64 (1H, d, CHarom), 6,51-6,53 (1H, d, CHarom), 3,68-3,75 (2H, m, CH₂), 3,54-3,68 (4H, m, CH₂), 3,07-3,09 (2H, m, CH₂).

### Étape 2 : 3-(2-((3-((2-(2-bromoéthoxy)éthyl)thio)phényl)amino)pyrimidin-4-yl)phénol (intermédiaire 4)

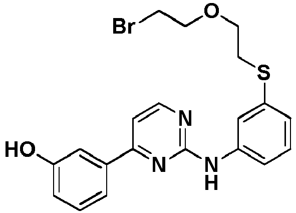

Dans un réacteur micro-ondes sont mélangés 134 mg (0,484 mmol) de 3-((2-(2-bromoéthoxy)éthyl)thio)aniline et 0,1 g (0,484 mmol) de 3-(2-chloropyrimidin-4-yl)phénol dans 0,5 mL de N-méthyl-2-pyrrolidinone. Le mélange réactionnel est porté à 150°C pendant 15 minutes. Après retour à température ambiante, ce dernier est hydrolysé par addition d'eau et extrait à l'acétate d'éthyle. La phase organique est séchée sur sulfate de sodium, filtrée et concentrée. Le résidu obtenu est purifié par chromatographie sur gel de silice (éluant : acétate d'éthyle/cyclohexane : 70/30) pour conduire à 27 mg (12%) de 3-(2-((3-((2-(2-bromoéthoxy)éthyl)thio)phényl) amino)pyrimidin-4-yl)phénol sous forme d'un solide jaune.
LCMS (IE, m/z) : (M+1) 447,36
RMN 1H: dH ppm (400 MHz, DMSO) : 9,70 (1H, s, NH), 9,70 (1H, s, CHarom), 8,54-8,55 (1H, d, CHarom), 7,91 (1H, s, CHarom), 7,67 (1H, d, CHarom), 7,54-7,58 (2H, m, CHarom), 7,33-7,34 (2H, m, CHarom), 7,26 (1H, t, CHarom), 6,94-6,96 (2H, m, CHarom), 3,66-3,68 (6H, m, CH₂), 3,14-3,17 (2H, m, CH₂).

### Etape 3 : Composé 2

Dans un ballon de 50 mL et sous azote, sont mélangés 27 mg (0,060 mmol) de 3-(2-((3-((2-(2-bromoéthoxy)éthyl)thio)phényl)amino)pyrimidin-4-yl)phénol, 5,09 mg (0,091 mmol) d'hydroxyde de potassium et 1,027 mg (3,02 µmol) d'hydrogénosulfate de tétrabutylammonium dans 1 mL de tétrahydrofurane. Le mélange réactionnel est porté à 80°C pendant 2 heures. Après retour à température ambiante, la réaction est hydrolysée par addition d'eau et extraite à l'acétate d'éthyle. La phase organique est séchée sur sulfate de sodium, filtrée et concentrée. Le résidu obtenu est purifié par chromatographie sur gel de silice (éluant : acétate d'éthyle/cyclohexane : 50/50) pour conduire à 7 mg (31%) de composé **2** sous forme d'un solide jaune.
LCMS (IE, m/z) : (M+1) 366,44
RMN 1H: dH ppm (400 MHz, DMSO) : 9,77 (1H, s, NH), 8,53-8,57 (2H, m, CHarom), 8,03 (1H, s, CHarom), 7,64-7,66 (1H, s, CHarom), 7,41-7,47 (2H, m, CHarom), 7,22-7,26 (1H, m, CHarom), 7,16-7,18 (1H, m, CHarom), 7,04-7,08 (2H, d, CHarom), 4,25-4,28 (2H, t, CH₂), 3,75-3,77 (2H, t, CH₂), 3,69-3,72 (2H, t, CH₂), 3,17-3,21 (2H, t, CH₂).

### Composé 3 :

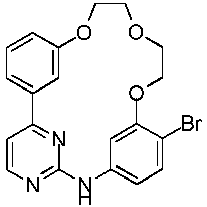

### Etape 1 : (E)-3-(diméthylamino)-1-(3-méthoxyphényl)prop-2-èn-1-one (intermédiaire 5)

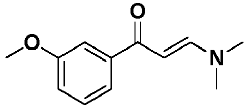

Dans un réacteur micro-ondes sont mélangés 2 g (13,32 mmol) de 1-(3-méthoxyphényl)éthanone et 11,50 mL (87 mmol) de N,N-diméthylformamide diméthyl acétal. Le mélange réactionnel est porté à 200°C pendant 10 minutes. Après retour à température ambiante, le résidu est concentré pour conduire à 2,250 g (82%) de (E)-3-(diméthylamino)-1-(3-méthoxyphényl)prop-2-èn-1-one sous la forme d'une huile jaune.
LCMS (IE, m/z) : (M+1) 206,25
RMN 1H : dH ppm (400 MHz, DMSO) : 7,69-7,72 (1H, d, CHarom), 7,44-7,46 (2H, m, CHarom et CH vinyl), 7,28-7,32 (1H, t, CHarom), 6,98-7,01 (1H, dd, CHarom), 5,67-5,70 (1H, d, CH vinyl), 3,84(3H, s, CH₃), 3,12 (3H, s, CH₃), 2,90 (3H, s, CH₃).

### Etape 2 : 4-(3-méthoxyphényl)-2-(méthylthio)pyrimidine (intermédiaire 6)

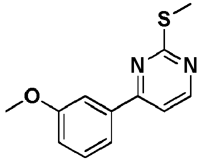

A 2,70 g (13,15 mmol) de (E)-3-(diméthylamino)-1-(3-méthoxyphényl)prop-2-èn-1-one dans 20 mL N, N-diméthylformamide est ajouté 5,49 g (19,73 mmol) d'hémisulfate de S-méthylisothiourée puis 3,23 g (32,9 mmol) d'acétate de potassium. Le mélange réactionnel est porté à 85°C pendant toute la nuit. Après retour à température ambiante, le solvant est évaporé, la réaction est hydrolysée par addition d'eau, basifiée et extraite à l'acétate d'éthyle. La phase organique est séchée sur sulfate de sodium, filtrée et concentrée pour conduire à une huile qui est utilisée telle quelle pour l'étape suivante.
LCMS (IE, m/z) : (M+1) 233,30

### Etape 3 : 4-(3-méthoxyphényl)-2-(méthylsulfonyl)pyrimidine (intermédiaire 7)

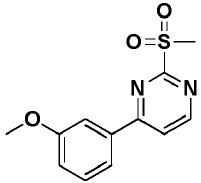

A 2,091 g (9 mmol) de 4-(3-méthoxyphényl)-2-(méthylthio)pyrimidine dans 30 mL de dichlorométhane est ajouté 4,66 g (27,0 mmol) d'acide métachloroperbenzoïque à 0°C par petites portions. Le mélange réactionnel est agité à 25°C pendant 3 heures. Le solvant est évaporé, la réaction est hydrolysée par addition d'eau, basifiée et extraite à l'acétate d'éthyle. La phase organique est séchée sur sulfate de sodium, filtrée et concentrée pour conduire à 0,600 g (25,2%) de 4-(3-méthoxyphényl)-2-(méthylsulfonyl)pyrimidine sous la forme d'un solide jaune.
LCMS (IE, m/z) : (M+1) 265,30
RMN 1H : dH ppm (400 MHz, DMSO) : 9,11-9,12 (1H, d, CHarom), 8,42-8,43 (1H, m, CHarom), 7,88-7,90 (1H, d, CHarom), 7,81-7,82 (1H, t, CHarom), 7,51-7,55 (1H, t, CHarom), 7,21-7,24 (1H, dd, CHarom), 3,87 (3H, s, CH₃), 3,50 (3H, s, CH₃).

### Etape 4 : N-(4-bromo-3-méthoxyphényl)-4-(3-méthoxyphényl)pyrimidin-2-amine (intermédiaire 8)

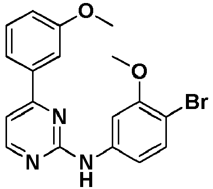

Dans un réacteur micro-ondes sont mélangés 150 mg (0,57 mmol) de 4-(3-méthoxyphényl)-2-(méthylsulfonyl)pyrimidine, 149 mg (0,73 mmol) de 4-bromo-3-méthoxyaniline et 83 mg (0,73 mmol) de tert-butylate de potassium dans 3 mL de N,N-diméthylformamide. Le mélange réactionnel est porté à 120°C pendant 60 minutes. Après retour à température ambiante, le résidu est concentré pour conduire à 165 mg (43%) de N-(4-bromo-3-méthoxyphényl)-4-(3-méthoxyphényl)pyrimidin-2-amine sous la forme d'un solide jaune.
LCMS (IE, m/z) : (M+1) 387,24
RMN 1H : dH ppm (400 MHz, DMSO) : 9,86 (1H, s, NH), 8,59-8,60 (1H, d, CHarom), 7,87 (1H, s, CHarom), 7,73-7,87 (2H, m, CHarom), 7,46-7,50 (3H, m, CHarom), 7,35-7,37 (1H, d, CHarom), 7,13-7,16 (1H, d, CHarom), 3,87 (3H, s, CH3), 3,86 (3H, s, CH3).

### Etape 5 : 2-bromo-5-((4-(3-hydroxyphényl)pyrimidin-2-yl)amino)phénol (intermédiaire 9)

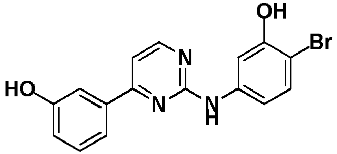

A une solution de 5,67 g (14,68 mmol) de N-(4-bromo-3-méthoxyphényl)-4-(3-méthoxyphényl)pyrimidin-2-amine dans 160 mL de dichlorométhane sont additionnés 6,94 mL de tribromo-borane à -78°C. Le milieu réactionnel est alors agité à 45°C pendant 5h puis toute la nuit à température ambiante. 20 mL de méthanol sont additionnés au mélange réactionnel à 0°C qui est ensuite chauffé à 35°C pendant 25 minutes. Le solide formé est filtré puis lavé deux fois avec 20 mL d'éther pour conduire à 6,45 g (100%) de 2-bromo-5-((4-(3-hydroxyphényl)pyrimidin-2-yl)amino)phénol sous la forme d'une poudre jaune.
LC-MS (IE, m/z): (M+1) 440,10
RMN 1H : dH ppm (400 MHz, DMSO) : 9,72 (1H, s, NH), 8,53-8,54 (1H, d, CHarom), 7,53-7,60 (3H, m, CHarom), 7,26-7,38 (4H, m, CHarom), 6,94-6,96 (1H, d, CHarom).

### Etape 6 : Composé 3

A une solution sous agitation de 0,93 g (2,11 mmol) de 2-bromo-5-((4-(3-hydroxyphényl)pyrimidin-2-yl)amino)phénol dans 100 mL de *N*,*N*-diméthylformamide est ajouté 1,46 g (10,59 mmol) de carbonate de potassium puis 0,49 g (2,11 mmol) de 1-bromo-2-(2-bromoéthoxy)éthane dans 50 mL de *N*,*N*-diméthylformamide pendant une heure. Le mélange réactionnel est agité à 75°C pendant 20 heures. Après retour à température ambiante, le solvant est évaporé, de l'eau est additionnée et le solide formé est filtré et séché sous vide pour conduire à 0,85 g (94%) de composé 3 sous forme d'une poudre beige.
LCMS (IE, m/z) : (M+1) 429,27
RMN 1H : dH ppm (400 MHz, DMSO) : 9,97 (1H, s, NH), 8,69 (1H, s, CHarom), 8,59-8;60 (1H, m, CHarom), 8,01 (1H, s, CHarom), 7,63-7,65 (1H, d, CHarom), 7,43-7,48 (3H, m, CHarom), 7,19-7,21 (1H, d, CHarom), 6.83-6,85 (1H, d, CHarom), 4,24-4,25 (4H, m, CH₂), 3,81-3,85 (4H, m, CH₂).

### Composé 4 :

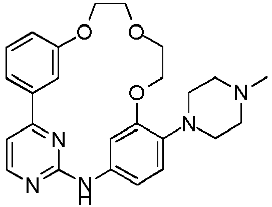

Dans un ballon de 50 mL sont mélangés 80 mg (0,168 mmol) de 2-(dicyclohexylphosphino)-2',4',6'-triisopropylbiphényle, 308 mg (0,336 mmol) de (dppf)₂PdCl₂.CH₂Cl₂, 1,8 g (4,2 mmol) de composé **3** et 5,89 g (58 mmol) de 1-méthylpipérazine sous argon. 23 mL de tétrahydrofurane et 33 mL (33 mmol) de bis(triméthylsilyl)amidure de lithium (LiHMDS) sont ajoutés à température ambiante. Le milieu réactionnel est porté à 85°C durant 5 heures. Après retour à température ambiante, la réaction est hydrolysée par addition lente d'eau à 0°C et le milieu est extrait à l'acétate d'éthyle. La phase organique est séchée sur sulfate de sodium, filtrée et concentrée. Le résidu obtenu est purifié par chromatographie sur gel de silice (éluant : acétate d'éthyle/méthanol/ammoniaque : 94/4/2) pour conduire à 692 mg (37%) de composé **4** sous forme d'un solide jaune.
LCMS (IE, m/z) : (M+1) 448,53
RMN 1H : dH ppm (400 MHz, DMSO) : 9,63(1H, s, NH), 8,52-8,54 (1H, m, CHarom), 8,47 (1H, m, CHarom), 8,11 (1H, s, CHarom), 7,63-7,65 (1H, d, CHarom), 7,44-7,46 (1H, m, CHarom), 7,35-7,36 (1H, d, CHarom), 7,10-7,20 (1H, d, CHarom), 6,51-6,87 (2H, m, CHarom), 4,21-4,27 (4H, m, CH₂), 3,84 (4H, m, CH₂), 3,67 (4H, m, CH₂), 2,46 (4H, m, CH₂), 2,22 (3H, m, CH₃).

### Composé 5 :

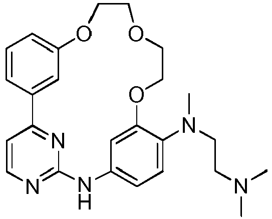

Le composé **5** a été préparé selon le protocole décrit pour la préparation du composé **3** à partir de 150 mg de composé **3** et de 107 mg de l'amine N1,N1,N2-triméthyléthane-1,2-diamine pour conduire à 8,2 mg (5%) du composé **5.**
LCMS (IE, m/z) : (M+1) 450,54
RMN 1H : dH ppm (400 MHz, DMSO) : 9,60 (1H, s, NH), 8,52-8,53 (1H, d, CHarom), 8,44 (1H, s, CHarom), 8,11 (1H, s, CHarom), 7,63-7,65 (1H, d, CHarom), 7,44-7,47 (1H, m, CHarom), 7,34-7,35 (1H, d, CHarom), 7,18-7,20 (1H, d, CHarom), 6,78-6,87 (2H, m, CHarom), 4,19-4,26 (4H, m, CH₂), 3,79-3,87 (4H, m, CH₂), 3,03-3,07 (2H, m, CH₂), 2,69 (3H, s, CH₂), 2,34-2,38 (2H, m, CH₂), 2,19 (6H, s, CH₂).

### Composé 6 :

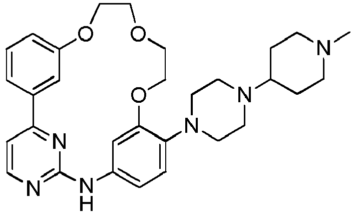

Le composé **6** a été préparé selon le protocole décrit pour la préparation du composé **3** à partir de 150 mg de composé **3** et de 193 mg de l'amine 1-(1-méthylpipéridin-4-yl)pipérazine pour conduire à 45 mg (23%) du composé **6.**
LCMS (IE, m/z) : (M+1) 431,66
RMN 1H : dH ppm (400 MHz, DMSO) : 9,62 (1H, s, NH), 8,52-8,53 (1H, d, CHarom), 8,46 (1H, s, CHarom), 8,10 (1H, s, CHarom), 7,63-7,65 (1H, d, CHarom), 7,43-7,47 (1H, m, CHarom), 7,34-7,35 (1H, d, CHarom), 7,17-7,19 (1H, d, CHarom), 6,84 (2H, m, CHarom), 4,20-4,26 (4H, m, CH₂), 3,81-3,84 (4H, m, CH₂), 2,93 (4H, m, CH₂), 2,77-2,80 (2H, m, CH₂), 2,60 (4H, m, CH₂), 2,13 (4H, m, CH₂), 1,73-1,86 (4H, m, CH₂), 1,40-1,43 (2H, m, CH₂).

### Composé 7 :

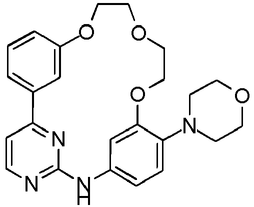

Le composé **7** a été préparé selon le protocole décrit pour la préparation du composé **3** à partir de 150 mg de composé **3** et de 92 mg de l'amine morpholine pour conduire à 25 mg (16%) du composé **7.**
LCMS (IE, m/z): (M+1) 435,48
RMN 1H : dH ppm (400 MHz, DMSO) : 9,97 (1H, s, NH), 8,48-8,54 (2H, m, CHarom), 8,10 (1H, s, CHarom), 7,63-7,65 (1H, d, CHarom), 7,45 (1H, t, CHarom), 7,35-7,36 (1H, d, CHarom), 7,17-7,19 (1H, d, CHarom), 6,84 (2H, m, CHarom), 4,25 (4H, m, CH₂), 3,84 (4H, m, CH₂), 3,72-3,80 (4H, m, CH₂), 2,93 (4H, m, CH₂).

### Composé 8 :

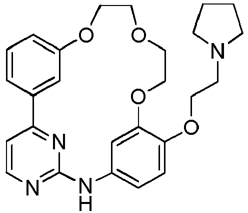

Dans un réacteur micro-ondes sont mélangés 150 mg (3,5 mmol) de composé **3,** 66,7 mg (0,36 mmol) d'iodure cuivreux et 0,4 mL d'(hydroxyéthyl)pyrrolidine (5,13 mmol). Le mélange réactionnel est porté à 200°C pendant 30 minutes. Après retour à température ambiante, la réaction est hydrolysée par addition d'eau et extraite à l'acétate d'éthyle. La phase organique est séchée sur sulfate de sodium, filtrée et concentrée. Le résidu obtenu est purifié par chromatographie sur gel de silice (éluant : acétate d'éthyle/méthanol/ammoniaque : 94/4/2) pour conduire à 24 mg (15%) de composé **8** sous forme d'un solide jaune.
LCMS (IE, m/z) : (M+1) 463,54
RMN 1H : dH ppm (400 MHz, DMSO) : 9,64 (1H, s, NH), 8,52-8,54 (1H, d, CHarom), 8,49 (1H, s, CHarom), 8,09 (1H, s, CHarom), 7,63-7,65 (1H, d, CHarom), 7,43-7,47 (1H, m, CHarom), 7,35-7,36 (1H, d, CHarom), 7,16-7,18 (1H, d, CHarom), 6,94-6,97 (1H, m, CHarom), 6,82-6,84 (1H, m, CHarom), 4,20-4,27 (4H, m, CH₂), 4,03-4,04 (2H, m, CH₂), 3,80 (4H, m, CH₂), 2,68 (2H, s, CH₂), 2,50 (4H, m, CH₂), 1,68 (4H, s, CH₂).

### Composé 9 :

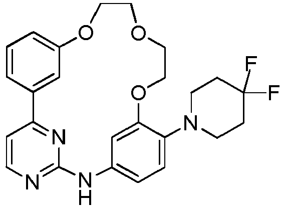

Le composé **9** a été préparé selon le protocole décrit pour la préparation du composé **3** à partir de 200 mg de composé **3** et de 294 mg de l'amine 4,4-difluoropipéridine pour conduire à 26 mg (12%) du composé **9.**
LCMS (IE, m/z): (M+1) 469,50
RMN 1H : dH ppm (400 MHz, DMSO) : 9,68 (1H, s, NH), 8,53-8,55 (1H, d, CHarom), 8,50 (1H, s, CHarom), 8,11 (1H, s, CHarom), 7,64-7,66 (1H, d, CHarom), 7,44-7,48 (1H, m, CHarom), 7,36-7,38 (1H, d, CHarom), 7,18-7,20 (1H, d, CHarom), 6,91-6,93 (1H, m, CHarom), 6,81-6,83 (1H, m, CHarom), 4,24-4,25 (4H, m, CH₂), 3,80-3,88 (4H, m, CH₂), 3,06 (4H, m, CH₂), 2,10 (4H, m, CH₂), 2,50 (4H, m, CH₂).

### Composé 10 :

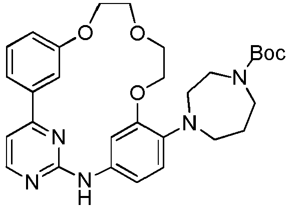

Le composé **10** a été préparé selon le protocole décrit pour la préparation du composé **3** à partir de 400 mg de composé **3** et de 561 mg de l'amine 1,4-diazépane-1-carboxylate de tert-butyle pour conduire à 90 mg (18%) du composé **10.**
LCMS (IE, m/z) : (M+1) 548,64

### Composé 11 :

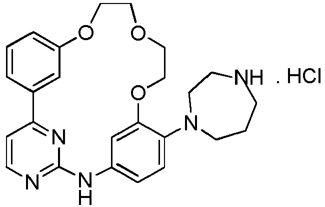

A 90 mg (0,16 mmpl) de composé **10** sont additionnés, au goutte à goutte, 10 mL d'une solution d'acide chlorhydrique dans l'isopropanol (5N). La solution est agitée à 20°C pendant 8 h. Après évaporation à sec, le solide formé est trituré dans l'éther puis recristallisé dans l'isopropanol pour obtenir 80 mg (92%) du composé **11** sous la forme d'une poudre jaune.
LCMS (IE, m/z) : (M+1) 448,53
RMN 1H : dH ppm (400 MHz, DMSO) : 9,71 (1H, s, NH), 8,88 (1H, s, CHarom), 8,53-8,55 (1H, d, CHarom), 8,49 (1H, s, CHarom), 8,10 (1H, s, CHarom), 7,63-7,66 (1H, d, CHarom), 7,44-7,48 (1H, t, CHarom), 7,37-7,38 (1H, d, CHarom), 7,18-7,20 (1H, dd, CHarom), 6,78-6,80 (1H, d, CHarom), 4,25 (4H, m, CH₂), 3,25-3,26 (6H, m, CH₂), 3,06 (4H, m, CH₂), 2,50 (4H, m, CH₂).

### Composé 12 :

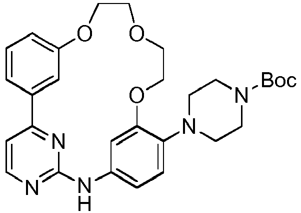

Le composé **12** a été préparé selon le protocole décrit pour la préparation du composé **3** à partir de 600 mg de composé **3** et de 2,08 g de l'amine pipérazine-1-carboxylate de tert-butyle pour conduire à 312 mg (42%) du composé **12.**
LCMS (IE, m/z) : (M+1) 534,62
RMN 1H : dH ppm (400 MHz, DMSO) : 9,65 (1H, s, NH), 8,48-8,53 (2H, m, CHarom), 8,10-8,11 (1H, d, CHarom), 7,62-7,64 (1H, d, CHarom), 7,43-7,47 (1H, t, CHarom), 7,35-7,36 (1H, d, CHarom), 7,18-7,20 (1H, d, CHarom), 6,82-6,84 (2H, m, CHarom), 4,21-4,26 (4H, m, CH₂), 3,80-3,86 (4H, m, CH₂), 3,44 (4H, m, CH₂), 2,28 (4H, m, CH₂), 1,42 (9H, s, CH₃).

### Composé 13 :

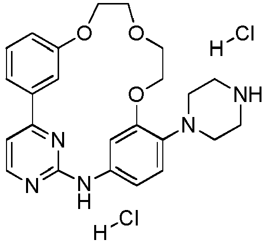

A 312 mg (0,58 mmol) de composé **12** sont additionnés, au goutte à goutte, 3 mL d'une solution d'acide chlorhydrique dans l'isopropanol (5N). La solution est agitée à 45°C pendant 1h. Après évaporation à sec, le solide formé est trituré dans l'éther puis recristallisé dans l'isopropanol pour obtenir 296 mg (95%) du composé **13** sous la forme d'une poudre jaune.
LCMS (IE, m/z) : (M+1) 434,21
RMN 1H : dH ppm (400 MHz, DMSO) : 9,98 (1H, s, NH), 9,45 (1H, s, NH), 8,57-8,58 (1H, d, CHarom), 8,51-8,52 (1H, m, CHarom), 8,09 (1H, t, CHarom), 7,66-7,68 (1H, d, CHarom), 7,43-7,48 (2H, m, CHarom), 7,19-7,21 (1H, dd, CHarom), 7,11-7,12 (1H, d, CHarom), 6,85-6,88 (1H, d, CHarom), 4,24-4,27 (4H, m, CH₂), 3,88-3,91 (2H, m, CH₂), 3,78-3,81 (2H, m, CH₂), 3,31 (8H, m, CH₂).

### Composé 14 :

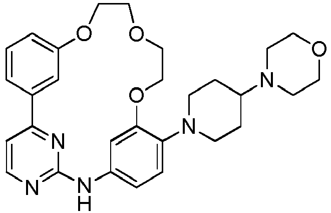

Le composé **14** a été préparé selon le protocole décrit pour la préparation du composé **3** à partir de 400 mg de composé **3** et de 159 mg de l'amine 4-(pipéridine-4-yl)morpholine pour conduire à 58 mg (12%) du composé **14.**
LCMS (IE, m/z): (M+1) 518,61
RMN 1H : dH ppm (400 MHz, DMSO) : 9,60 (1H, s, NH), 8,51-8,52 (1H, d, CHarom), 8,45-8,46 (1H, d, CHarom), 8,10 (1H, s, CHarom), 7,62-7,64 (1H, d, CHarom), 7,43-7,47 (1H, t, CHarom), 7,33-7,35 (1H, d, CHarom), 7,18-7,20 (1H, d, CHarom), 6,81-6,83 (2H, m, CHarom), 4,21-4,26 (4H, m, CH₂), 3,79-3,85 (4H, m, CH₂), 3,85 (4H, m, CH₂), 3,33 (8H, m, CH₂), 2,17-2,24 (1H, t, CH₂), 1,83-1,86 (2H, d, CH₂), 1,47-1,56 (2H, d, CH₂).

### Composé 15 :

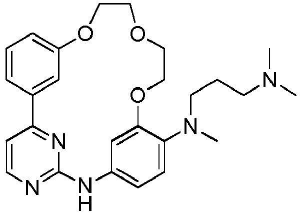

Le composé **15** a été préparé selon le protocole décrit pour la préparation du composé **3** à partir de 400 mg de composé **3** et de 109 mg de l'amine N1,N1,N3-triméthyl-propane-1,3-diamine pour conduire à 53 mg (12%) du composé **15.**
LCMS (IE, m/z) : (M+1) 464,57
RMN 1H : dH ppm (400 MHz, DMSO) : 9,58 (1H, s, NH), 8,51-8,52 (1H, d, CHarom), 8,43 (1H, s, CHarom), 8,10 (1H, s, CHarom), 7,62-7,64 (1H, d, CHarom), 7,42-7,46 (1H, t, CHarom), 7,33-7,34 (1H, d, CHarom), 7,16-7,18 (1H, d, CHarom), 6,78-6,86 (2H, m, CHarom), 4,18-4,25 (4H, m, CH₂), 3,80-3,86 (4H, m, CH₂), 2,93-2,96 (2H, m, CH₂), 2,21-2,22 (2H, t, CH₂), 2,09 (9H, s, CH₃), 1,55-1,58 (2H, m, CH₃).

### Composé 16 :

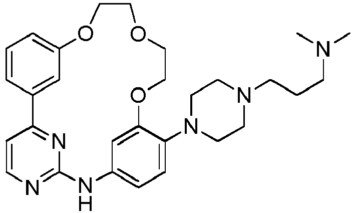

Le composé **16** a été préparé selon le protocole décrit pour la préparation du composé **3** à partir de 400 mg de composé **3** et de 160 mg de l'amine 4-[3-(N,N-diméthylamino)-propyl]-piperazine pour conduire à 90 mg (19%) du composé **16.**
LCMS (IE, m/z): (M+1) 519,65
RMN 1H : dH ppm (400 MHz, DMSO) : 9,61 (1H, s, NH), 8,51-8,52 (1H, d, CHarom), 8,46-8,47 (1H, d, CHarom), 8,09-8,10 (1H, t, CHarom), 7,62-7,64 (1H, d, CHarom), 7,42-7,46 (1H, t, CHarom), 7,33-7,35 (1H, d, CHarom), 7,16-7,18 (1H, dd, CHarom), 6,82-6,85 (2H, m, CHarom), 4,20-4,26 (4H, m, CH₂), 3,79-3,83 (4H, m, CH₂), 2,29 (4H, m, CH₂), 2,30-2,33 (2H, t, CH₂), 2,49 (4H, m, CH₂), 2,19-2,23 (2H, d, CH₂), 2,11 (6H, s, CH₃), 1,54-1,60 (2H, m, CH₂).

### Composé 17 :

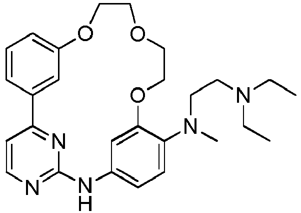

Le composé **17** a été préparé selon le protocole décrit pour la préparation du composé **3** à partir de 400 mg du composé **3** et de 122 mg de l'amine N1,N1-diéthyl-N2-méthyl-éthane-1,2-diamine pour conduire à 53 mg (12%) du composé **17.**
LCMS (IE, m/z) : (M+1) 478,59
RMN 1H : dH ppm (400 MHz, DMSO) : 9,66 (1H, s, NH), 8,53-8,54 (1H, d, CHarom), 8,46-8,47 (1H, d, CHarom), 8,09-8,10 (1H, t, CHarom), 7,62-7,64 (1H, d, CHarom), 7,43-7,47 (1H, t, CHarom), 7,35-7,36 (1H, d, CHarom), 7,17-7,20 (1H, dd, CHarom), 6,96-6,98 (1H, m, CHarom), 6,80-6,82 (1H, dd, CHarom), 4,16-4,25 (4H, m, CH₂), 3,87-3,90 (2H, t, CH₂), 3,79-3,81 (2H, m, CH₂), 3,13-3,32 (8H, t, CH₂), 2,27 (3H, s, CH₃), 1,11-1,20 (6H, m, CH₃).

### Composé 18 :

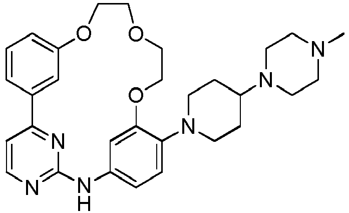

Le composé **18** a été préparé selon le protocole décrit pour la préparation du composé **3** à partir de 400 mg de composé **3** et de 171 mg de l'amine 1-méthyl-4-(pipéridine-4-yl)
pipérazine pour conduire à 63 mg (12%) du composé **18.**
LCMS (IE, m/z) : (M+1) 531,30
RMN 1H : dH ppm (400 MHz, DMSO) : 9,60 (1H, s, NH), 8,51-8,53 (1H, d, CHarom), 8,45 (1H, s, CHarom), 8,10 (1H, s, CHarom), 7,62-7,64 (1H, d, CHarom), 7,42-7,47 (1H, t, CHarom), 7,33-7,34 (1H, d, CHarom), 7,16-7,18 (1H, dd, CHarom), 6,79-6,85 (2H, m, CHarom), 4,20-4,25 (4H, m, CH₂), 3,80-3,84 (4H, m, CH₂), 3,32-3,37 (4H, m, CH₂), 2,20-2,32 (8H, t, CH₂), 2,20-2,27 (1H, t, CH₂), 2,12 (3H, s, CH₃), 1,81-1,83 (2H, m, CH₂), 1,51-1,53 (2H, m, CH₂).

### Composé 19 :

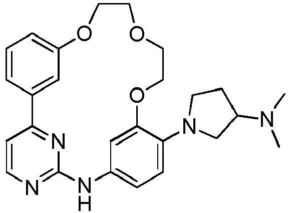

Le composé **19** a été préparé selon le protocole décrit pour la préparation du composé **3** à partir de 400 mg de composé **3** et de 107 mg de l'amine N,N-diméthyl-pyrrolidin-3-diamine pour conduire à 28 mg (6%) du composé **19.**
LCMS (IE, m/z) : (M+1) 462,24
RMN 1H : dH ppm (400 MHz, DMSO) : 9,51 (1H, s, NH), 8,49-8,50 (1H, d, CHarom), 8,41 (1H, s, CHarom), 8,10 (1H, s, CHarom), 7,61-7,63 (1H, d, CHarom), 7,42-7,46 (1H, t, CHarom), 7,30-7,31 (1H, d, CHarom), 7,16-7,18 (1H, d, CHarom), 6,68-6,78 (1H, m, CHarom), 6,65-6,67 (1H, m, CHarom), 4,09-4,24 (4H, m, CH₂), 3,79-3,85 (4H, m, CH₂), 3,19-3,21 (2H, m, CH₂), 3,11-3,15 (1H, t, CH₂), 2,22 (6H, s, CH₃), 1,99-2,07 (2H, m, CH₂), 1,68-1,77 (2H, m, CH₂).

### Composé 20 :

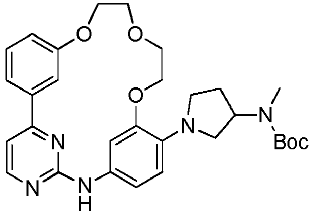

Le composé **20** a été préparé selon le protocole décrit pour la préparation du composé **3** à partir de 200 mg de composé **3** et de 374 mg de l'amine méthyl(pyrrolidin-3-yl)carbamate de tert-butyle pour conduire à 60 mg (22%) de composé **20.**
LCMS (IE, m/z) : (M+1) 548,64

### Composé 21 :

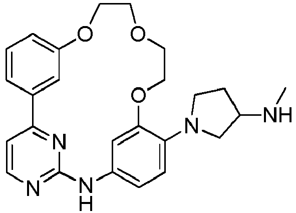

A 60 mg (0.11 mmol) de composé **20** dans 2 mL de dichlorométhane sont ajoutés 25 µl (0,329 mmol) d'acide trifluoroacétique (TFA) à 0°C par petites portions. Le mélange réactionnel est agité à 25°C pendant 12 heures. Le solvant est évaporé, la réaction est hydrolysée par addition d'eau, basifiée et extraite à l'acétate d'éthyle. La phase organique est séchée sur sulfate de sodium, filtrée et concentrée. Le résidu obtenu est purifié par chromatographie sur gel de silice (éluant : acétate d'éthyle/méthanol/ammoniac : 90/8/2) pour conduire à 24 mg (48%) du composé **21** sous la forme d'un solide jaune.
LCMS (IE, m/z) : (M+1) 448,53
RMN 1H : dH ppm (400 MHz, DMSO) : 9,51 (1H, s, NH), 8,50-8,51 (1H, d, CHarom), 8,41-8,42 (1H, d, CHarom), 8,11 (1H, s, CHarom), 7,62-7,64 (1H, d, CHarom), 7,43-7,47 (1H, t, CHarom), 7,30-7,31 (1H, d, CHarom), 7,17-7,19 (1H, d, CHarom), 6,77-6,79 (1H, m, CHarom), 6,63-6,66 (1H, m, CHarom),4,25-4,27 (2H, m, CH₂), 4,13-4,14 (2H, m, CH₂), 3,79-3,86 (4H, m, CH₂), 3,20 (2H, m, CH₂), 3,10-3,21 (3H, m, NH et CH₂), 3,01-3,03 (1H, m, CH), 2,31 (3H, s, CH₃), 2,01-2,05 (1H, m, CH), 1,64-1,68 (1H, m, CH).

### Composé 22 :

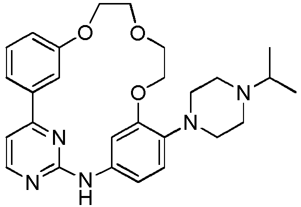

A une solution de 120 mg (0,24 mmol) de composé **13** et de 41 mg (0,71 mmol) de propan-2-one dans 3 mL de 1,2-dichloroéthane sont ajoutés 68 µl d'acide acétique (1,18 mmol) et, par petites fractions, 201 mg (0,95 mmol) de triacétoxyborohydrure de sodium. Le milieu réactionnel est agité pendant 16 heures à température ambiante. Le solvant est alors concentré, le milieu réactionnel extrait à l'acétate d'éthyle et lavé à l'aide d'une solution saturée d'hydroxyde de sodium. Les phases organiques sont regroupées, séchées sur du sulfate de magnésium puis concentrées. Le résidu est purifié par chromatographie sur gel de silice (éluant : dichlorométhane/méthanol/ammoniaque : 95/4/1) pour conduire à 24 mg (21%) du composé **22** sous forme d'un solide jaune.
LCMS (IE, m/z) : (M+1) 476,58
RMN 1H : dH ppm (400 MHz, CDCl₃) : 8,58-8,60 (1H, d, CHarom), 8,42-8,43 (1H, d, CHarom), 8,23-8,24 (1H, t, CHarom), 8,44-8,46 (1H, d, CHarom), 7,36-7,39 (1H, t, CHarom), 7,20 (1H, s, NH), 7,12-7,14 (1H, d, CHarom), 7,05-7,07 (1H, dd, CHarom), 6,88-6,90 (1H, d, CHarom), 6,51-6,54(1H, dt, CHarom), 4,39-4,42 (2H, t, CH₂), 4,29-4,31 (2H, m, CH₂), 3,96-4,00 (2H, m, CH₂), 3,89-3,91 (2H, m, CH₂), 2,62-3,22 (8H, m, CH₂), 2,49-2,58 (1H, m, CH), 1,17 (6H, s, CH₃).

### Composé 23 :

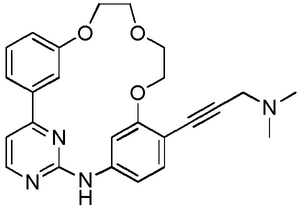

Dans un vial de 10 mL sont mélangés 107 mg (0,25 mmol) de composé **3,** 27 mg (0,058 mmol) de 2-(dicyclohexylphosphino)-2',4',6'-triisopropylbiphényle, 4,79 mg (0,013 mmol) de chlorure de benzonitrile palladium(II), 48 µL (0,450 mmol) de diméthylamino-2-propyne et 244 mg (0,75 mmol) de carbonate de césium dans 0,3 mL de diméthylformamide à température ambiante. On ajoute au mélange réactionnel 81 mg (0,250 mmol) de bromure de tétrabutylammonium. Le milieu réactionnel est porté à 80°C durant 15 heures. Après retour à température ambiante, la réaction est hydrolysée par addition lente d'eau et extraite à l'acétate d'éthyle. La phase organique est séchée sur sulfate de sodium, filtrée et concentrée. Le résidu obtenu est purifié par chromatographie sur gel de silice (éluant : acétate d'éthyle/méthanol/ammoniaque : 95/4/1) pour conduire à 9 mg (9%) de composé **23** sous forme d'un solide jaune.
LCMS (IE, m/z) : (M+1) 431,50
RMN 1H : dH ppm (400 MHz, DMSO) : 10,00 (1H, s, NH), 8,63-8,64 (1H, d, CHarom), 8,59-8,60 (1H, d, CHarom), 8,11 (1H, m, CHarom), 7,64-7,65 (1H, d, CHarom), 7,44-7,48 (2H, m, CHarom), 7,23-7,25 (1H, d, CHarom), 7,18-7,20 (1H, d, CHarom), 6,83-6,85 (1H, dd, CHarom), 4,23-4,28 (4H, m, CH₂), 3,80-3,86 (4H, m, CH₂), 3,42 (2H, m, CH₂), 2,24 (6H, s, CH₃).

### Composé 24 :

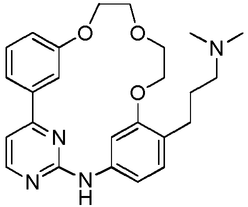

Dans un ballon et sous argon, introduire 40 mg (0,09 mmol) du composé **23** dans 10 mL d'un mélange de THF/MeOH (1/1). Dégazer le milieu sous argon et sous vide. Ajouter 9,8 mg (0,009 mmol) de Pd-C. Dégazer le milieu sous argon et sous vide puis placer un ballon d'hydrogène. Le mélange réactionnel est agité à 25°C durant la nuit puis filtré sur de la silice et rincer avec de l'acétate d'éthyle puis concentré. Le résidu obtenu est purifié par chromatographie sur gel de silice (éluant : acétate d'éthyle/méthanol/ammoniac : 90/8/2) pour conduire à 5,1 mg (12%) du composé **24** sous forme d'un solide jaune.
LCMS (IE, m/z): (M+1) 435,53
RMN 1H : dH ppm (400 MHz, DMSO) : 9,70 (1H, s, NH), 8,54-8,55 (1H, d, CHarom), 8,47-8,48 (1H, d, CHarom), 8,12 (1H, s, CHarom), 7,63-7,64 (1H, d, CHarom), 7,44-7,48 (1H, t, CHarom), 7,37-7,38 (1H, d, CHarom), , 7,17-7,20 (1H, d, CHarom), 7,00-7,03 (1H, dd, CHarom), 6,76-6,78 (1H, dd, CHarom), 4,10-4,24 (4H, m, CH₂), 3,79-3,87 (4H, m, CH₂), 2,18-2,21 (2H, m, CH₂), 2,45 (2H, m, CH₂), 2,11 (6H, s, CH₃), 1,60-1,64 (2H, m, CH₂).

### Composé 25 :

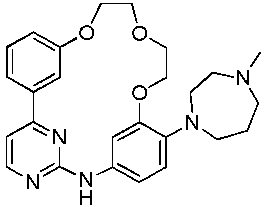

A une solution de 70 mg (0,13 mmol) de composé 11 dans 2 mL de 1,2-dichloroéthane sont ajoutés 37,5 µl (0,269 mmol) de triéthylamine. Agitation pendant 10 minutes puis addition de 29,3 µl (0,403 mmol) d'une solution aqueuse de formaldéhyde et 38,5 µl (0,672 mmol) d'acide acétique, puis 114 mg (0,538 mmol) de triacétoxyborohydrure de sodium. Le milieu réactionnel est agité pendant 24 heures à température ambiante. Le solvant est alors concentré, le milieu réactionnel extrait à l'acétate d'éthyle et lavé à l'aide d'une solution saturée d'hydroxyide de sodium 1N. Les phases organiques sont regroupées, séchées sur du sulfate de magnésium puis concentrées. Le résidu est purifié par chromatographie sur gel de silice (éluant : dichlorométhane/méthanol : 90/10) pour conduire à 29 mg (43%) du composé **25** sous forme d'un solide jaune.
LCMS (IE, m/z) : (M+1) 462,56
RMN 1H : dH ppm (400 MHz, DMSO) : 9,57 (1H, s, NH), 8,50-8,81 (1H, s, CHarom), 8,42-8,43 (1H, d, CHarom), 8,10 (1H, dd, CHarom), 7,62-7,64 (1H, d, CHarom), 7,43-7,47 (1H, dd, CHarom), 7,32-7,35 (1H, d, CHarom), 7,16-7,17 (1H, dd, CHarom), 6,74-6,83 (2H, d, CHarom), 4,17-4,25 (4H, m, CH₂), 3,79-3,84 (4H, m, CH₂), 3,16-3,21 (4H, m, CH₂), 2,55-2,67 (4H, m, CH₂), 2,28 (3H, m, CH₃), 1,83-1,90 (2H, m, CH₂).

### Composé 26 :

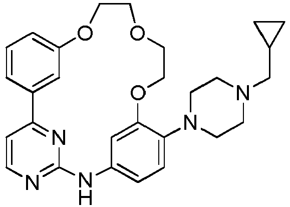

A une solution de 120 mg (0,13 mmol) de composé **13** dans 3 mL de 1,2-dichloroéthane sont ajoutés 61 µl (0,47 mmol) de triéthylamine. Agitation pendant 10 minutes puis addition de 53 µl (0,71 mmol) de cyclopropane carbaldéhyde et de 136 µl (2,37 mmol) d'acide acétique, puis 201 mg (0,94 mmol)) de triacétoxyborohydrure de sodium. Le milieu réactionnel est agité pendant 24 heures à température ambiante. Le solvant est alors concentré, le milieu réactionnel extrait à l'acétate d'éthyle et lavé à l'aide d'une solution saturée d'hydroxide de sodium 1N. Les phases organiques sont regroupées, séchées sur du sulfate de magnésium puis concentrées. Le résidu est purifié par chromatographie sur gel de silice (éluant : dichlorométhane/méthanol/ammoniaque : 90/8/2) pour conduire à 28 mg (24%) du composé **26** sous forme d'un solide jaune.
LCMS (IE, m/z) : (M+1) 488,59
RMN 1H : dH ppm (400 MHz, DMSO) : 9,62 (1H, d, CHarom), 8,51-8,53 (1H, d, CHarom), 8,46-8,47 (1H, d, CHarom), 8,10 (1H, t, CHarom), 7,62-7,64 (1H, d, CHarom), 7,42-7,46 (1H, t, CHarom), 7,34-7,35 (1H, d, CHarom), 7,16-7,18 (1H, dd, CHarom), 6,80-6,86 (2H, m, CHarom), 4,20-4,26 (4H, m, CH₂), 3,78-3,86 (4H, m, CH₂), 2,94 (4H, m, CH₂), 2,56 (4H, m, CH₂), 2,22 (2H, m, CH), 0,85 (1H, s, CH), 0,47 (2H, m, CH₂), 0,09 (2H, s, CH₂).

### Composé 27 :

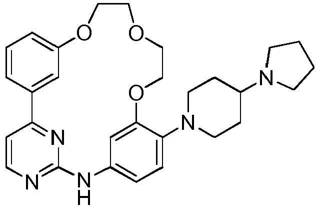

Le composé **27** a été préparé selon le protocole décrit pour la préparation du composé **3** à partir de 400 mg de composé **3** et de 577 mg de l'amine 4-(pyrrolidin-1-yl)pipéridine pour conduire à 63 mg (13%) du composé **27**.
LCMS (IE, m/z) : (M+1) 502,62
RMN 1H : dH ppm (400 MHz, DMSO) : 9,61 (1H, s, NH), 8,51-8,52 (1H, d, CHarom), 8,45-8,46 (1H, d, CHarom), 8,10 (1H, s, CHarom), 7,62-7,64 (1H, d, CHarom), 7,42-7,46 (1H, t, CHarom), 7,33-7,35 (1H, d, CHarom), 7,16-7,18 (1H, dd, CHarom), 6,79-6,85 (2H, m, CHarom), 4,21-4,26 (4H, m, CH₂), 3,79-3,84 (4H, m, CH₂), 3,27-3,32 (4H, m, CH₂), 2,25-2,58 (5H, m, CH et CH₂), 1,89-1,94 (2H, m, CH₂), 1,68 (4H, m, CH₂), 1,46-1,57 (2H, m, CH₂).

### Composé 28 :

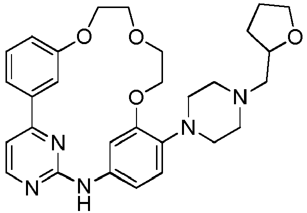

Le composé **28** a été préparé selon le protocole décrit pour la préparation du composé **3** à partir de 400 mg de composé **3** et de 637 mg de l'amine 1-((tétrahydrofuran-2-yl) méthyl)pipérazine pour conduire à 108 mg (22%) du composé **28**.
LCMS (IE, m/z) : (M+1) 518,62
RMN 1H : dH ppm (400 MHz, DMSO) : 9,62 (1H, s, NH), 8,51-8,52 (1H, d, CHarom), 8,45-8,46 (1H, d, CHarom), 8,10 (1H, s, CHarom), 7,62-7,64 (1H, d, CHarom), 7,42-7,46 (1H, t, CHarom), 7,34-7,35 (1H, d, CHarom), 7,16-7,18 (1H, dd, CHarom), 6,80-6,85 (2H, m, CHarom), 4,20-4,26 (4H, m, CH₂), 3,90-3,97 (1H, m, CH), 3,78-3,84 (4H, m, CH₂), 3,71-3,77 (1H, m, CH), 3,58-3,62 (1H, m, CH), 2,92 (4H, m, CH₂), 2,47-2,67 (4H, m, CH₂), 2,38-2,42 (2H, m, CH₂), 1,89-1,97 (1H, m, CH), 1,73-1,83 (2H, m, CH₂), 1,44-1,53 (1H, m, CH).

### Composé 29 :

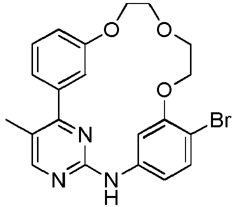

### Etape 1 : Intermédiaire 10

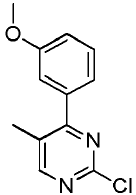

A 1,12 g (6,91 mmol) de 2,4-dichloro-5-méthylpyrimidine dans 50 mL de THF anhydre est additionné 1 g (6,58 mmol) d'acide 3-méthoxyphénylboronique. Le milieu réactionnel est agité à température ambiante durant 10 minutes puis est additionné à température ambiante 1,74 g (16,45 mmol) de carbonate de sodium dissous dans 8 mL d'eau puis 0,38 g (0,329 mmol) de tétrakis(triphénylphosphine)palladium(0). Le milieu réactionnel est agité à 90°C pendant 16 heures. De l'acétate d'éthyle est additionné et la phase organique est lavée avec une solution saturée de chlorure de sodium, séchée sur sulfate de magnésium, filtrée. Le filtrat est évaporé et le résidu purifié par chromatographie sur colonne de silice à l'aide du Companion® (éluant : cyclohexane/acétate d'éthyle 0 à 10% puis cyclohexane/acétate d'éthyle 95/5) pour conduire à 1,2 g (77%) de l'intermédiaire 10 sous forme d'un solide blanc.
LCMS (IE, m/z) : (M+1) 235,68
RMN 1H : dH ppm (400 MHz, DMSO) : 8,72 (1H, s, CHarom), 7,44-7,48 (1H, d, CHarom), 7,21-7,23 (1H, d, CHarom), 7,18 (1H, m, CHarom), 7,10-7,13 (1H, dd, CHarom), 3,83 (3H, s, CH₃), 3,33 (3H, s, CH₃).

### Etape 2 : Intermédiaire 11

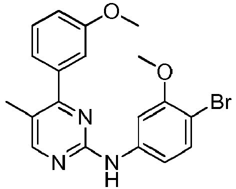

A une solution de 15 mL de n-butanol comprenant 0,6 g (2,56 mmol) de l'intermédiaire 10 et 0,52 g (2,56 mmol) de 4-bromo-3-méthoxyaniline est additionné 0,5 mL d'une solution d'acide chlorhydrique (5N). Le milieu réactionnel est agité à 80°C toute la nuit. Après retour à la température ambiante, le solide formé est filtré, rincé avec du n-butanol pour conduire à 0,79 g (78%) de l'intermédiaire 11 sous forme d'une poudre jaune.
LCMS (IE, m/z) : (M+1) 401,26
RMN 1H : dH ppm (400 MHz, DMSO) : 9,76 (1H, s, NH), 8,46 (1H, d, CHarom), 7,92 (1H, s, CHarom), 7,38-7,46 (2H, m, CHarom), 7,24-7,28 (3H, m, CHarom), 7,06-7,08 (1H, d, CHarom), 3,81 (3H, s, CH₃), 3,87 (3H, s, CH₃), 2,24 (3H, s, CH₃).

### Etape 3 : Intermédiaire 12

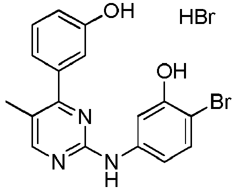

A une solution de 0,25 g (0,62 mmol) de l'intermédiaire 11 dans 7 mL de dichlorométhane est additionné 0,3 mL (3,12 mmol) de tribromo-borane à -78°C. Le milieu réactionnel est alors agité à 45°C pendant 5 h puis toute la nuit à température ambiante. 20 mL de méthanol sont additionnés au mélange réactionnel à 0°C puis on chauffe à 35°C pendant 25 minutes. Le solide formé est filtré puis lavé deux fois avec 20 mL d'éther pour conduire à 0,27 g (99%) de l'intermédiaire 12 sous la forme d'une poudre jaune.
LC-MS (IE m/z) : (M+H⁺) : 372,02 + 374,02
RMN 1H : dH ppm (400 MHz, DMSO) : 9,62 (1H, s, NH), 8,40 (1H, s, CHarom), 7,49-7,50 (1H, m, CHarom), 7,28-7,32 (2H, m, CHarom), 7,18-7,21 (1H, m, CHarom), 7,03-7,08 (2H, m, CHarom), 6,86-6,89 (1H, d, CHarom), 2,20 (3H, s, CH₃).

A une solution sous agitation de 0,25 g (0,67 mmol) de l'intermédiaire 12 dans 19 mL de *N*,*N*-diméthylformamide est ajouté 0,46 g (3,37 mmol) de carbonate de potassium puis

0,15 g (0,67 mmol) de 1-bromo-2-(2-bromoéthoxy)éthane dans 8 mL de *N,N-*diméthylformamide pendant une heure. Le mélange réactionnel est agité à 75°C pendant 20 heures. Après retour à température ambiante, le solvant est évaporé, de l'eau est additionnée et le solide formé est filtré et séché sous vide pour conduire à 0,25 g (64%) du composé **29** sous forme d'une poudre beige.
LCMS (IE, m/z) : (M+1) 443,30
RMN 1H : dH ppm (400 MHz, DMSO) : 9,84 (1H, s, NH), 8,70 (1H, s, CHarom), 8,47 (1H, s, CHarom), 7,59 (1H, s, CHarom), 7,37-7,43 (2H, m, CHarom), 7,29-7,32 (1H, m, CHarom), 7,18-7,20 (1H, d, CHarom), 6,77-6,79 (1H, d, CHarom),4,30 (2H, m, CH₂), 4,09-4,11 (2H, m, CH₂), 3,77-3,85 (4H, m, CH₂), 2,30 (3H, s, CH₃).

### Composé 30 :

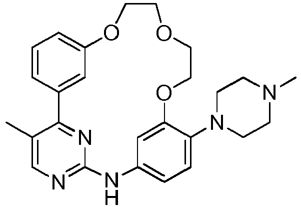

Le composé **30** a été préparé selon le protocole décrit pour la préparation du composé **3** à partir de 124 mg de composé **3** et de 393 mg de l'amine 1-méthylpiperazine pour conduire à 29 mg (22%) du composé **30**.
LCMS (IE, m/z) : (M+1) 462,55
RMN 1H : dH ppm (400 MHz, DMSO) : 9,46 (1H, s, NH), 8,46-8,47 (1H, d, CHarom), 8,39 (1H, s, CHarom), 7,59 (1H, s, CHarom), 7,38-7,42 (1H, t, CHarom), 7,28-7,30 (1H, d, CHarom), 7,14-7,17 (1H, dd, CHarom), 6,73-6,80 (2H, m, CHarom), 4,29-4,31 (2H, m, CH₂), 4,07-4,10 (2H, m, CH₂), 3,80-3,82 (4H, m, CH₂), 2,89 (4H, m, CH₂), 2,42 (4H, m, CH₂), 2,29 (3H, m, CH₃), 2,19 (3H, m, CH₃).

### Composé 31 :

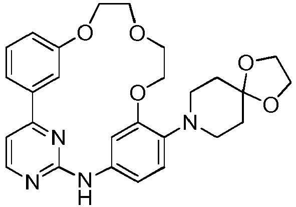

Le composé **31** a été préparé selon le protocole décrit pour la préparation du composé **3** à partir de 400 mg de composé **3** et de 536 mg de l'amine 1,4-dioxa-8-azaspiro[4.5]décane pour conduire à 33 mg (7%) du composé **31**.
LCMS (IE, m/z) : (M+1) 491,55
RMN 1H : dH ppm (400 MHz, DMSO) : 9,63 (1H, s, NH), 8,51-8,52 (1H, d, CHarom), 8,45-8,46 (1H, d, CHarom), 8,10 (1H, s, CHarom), 7,62-7,64 (1H, d, CHarom), 7,43-7,47 (1H, t, CHarom), 7,34-7,35 (1H, d, CHarom), 7,17-7,19 (1H, dd, CHarom), 6,79-6,88 (2H, m, CHarom), 4,22-4,27 (4H, m, CH₂), 3,90 (4H, s, CH₂), 3,79-3,85 (4H, m, CH₂), 2,98 (4H, m, CH₂), 1,73-1,76 (4H, m, CH₂).

### Composé 32 :

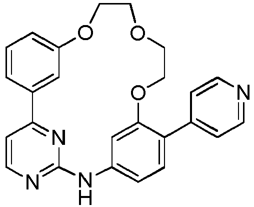

Dans un réacteur micro-ondes sont mélangés 95 mg (0,771 mmol) d'acide pyridin-4-ylboronique dissous dans 10 mL d'un mélange 2/1 dioxane/H₂O et sont additionnés successivement 72 mg (0,17 mmol) de 2-dicyclohexylphosphino-2',6'-diméthoxy-1,1'-biphényle, 64 mg (0,070 mmol) de Pd₂(dba)₃, 297 mg (1,4 mmol) de K₃PO₄ et 300 mg (0,77 mmol) de composé **3**. Le mélange réactionnel est porté à 150°C pendant 20 minutes. Après retour à température ambiante, la réaction est hydrolysée par addition d'eau et extraite à l'acétate d'éthyle. La phase organique est séchée sur sulfate de sodium, filtrée et concentrée. Le résidu obtenu est purifié par chromatographie sur gel de silice (éluant : acétate d'éthyle/méthanol/ ammoniaque : 94/4/2) pour conduire à 96 mg (32%) de composé **32** sous forme d'un solide jaune.
LCMS (IE, m/z) : (M+1) 427,47
RMN 1H : dH ppm (400 MHz, DMSO) : 10,04 (1H, s, NH), 8,71-8,22 (1H, d, CHarom), 8,60-8,62 (1H, d, CHarom), 8,50-8,54 (2H, d, CHarom), 8,14 (1H, m, CHarom), 7,64-7,66 (1H, d, CHarom), 7,55-7,57 (2H, d, CHarom), 7,45-7,49 (2H, m, CHarom), 7,37-7,39 (1H, d, CHarom), 7,18-7,21 (1H, dd, CHarom), 6,98-7,01 (1H, dd, CHarom), 4,22-4,26 (4H, m, CH₂), 3,78-3,84 (4H, m, CH₂).

### Composé 33 :

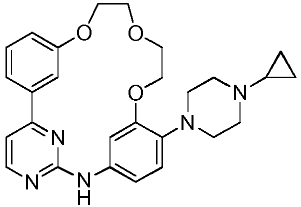

Le composé **33** a été préparé selon le protocole décrit pour la préparation du composé **3** à partir de composé **3** et de l'amine 1-cyclopropylpipérazine.
LCMS (IE, m/z) : (M+1) 474,57
RMN 1H : dH ppm (400 MHz, CDCl3) : 8,57-8,58 (1H, d, CHarom), 8,41-8,43 (1H, d, CHarom), 8,24 (1H, m, CHarom), 7,44-7,46 (1H, d, CHarom), 7,36-7,39 (1H, t, CHarom), 7,19 (1H, s, NH), 7,12-7,14 (1H, d, CHarom), 7,05-7,08 (1H, dd, CHarom), 6,85-6,88 (1H, m, CHarom), 6,51-6,55 (1H, d, CHarom), 4,40-4,43 (2H, t, CH₂), 4,30-4,32 (2H, t, CH₂), 3,97-4,00 (2H, t, CH₂), 3,90-3,92 (2H, t, CH₂), 3,04 (4H, m, CH₂), 2,81 (4H, m, CH₂), 1,61 (1H, m, CH), 0,47 (4H, m, CH₂).

### Composé 34 :

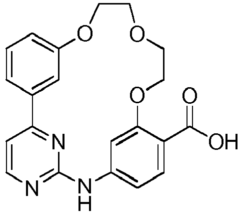

A 400 mg (0,93 mmol) de composé **3** dans 10 mL de tétrahydrofurane est ajouté 1,12 mL (2,8 mmol) d'une solution de butyllithium dans l'hexane (2,5 M) à -78°C. Le milieu réactionnel est agité à -78°C durant 30 minutes puis on fait buller du CO₂ dans la solution pendant 3 heures. Après retour à température ambiante, la réaction est hydrolysée par addition lente d'eau à 0°C puis un mélange de méthanol/acide acétique est ajouté. Les solvants sont évaporés, le solide formé est filtré et séché sous vide pour conduire à 367 mg (100%) du composé **34** sous forme d'une poudre beige.
LCMS (IE, m/z) : (M+1) 394,39

### Composé 35 :

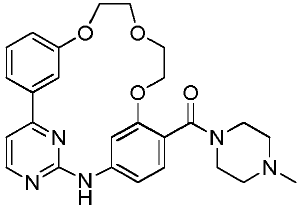

Dans un réacteur micro-ondes sont additionnés successivement 367mg (0,933 mmol) de composé **34**, 815 µl de diisopropyléthylamine (DIEA) (4,66 mmol), 333 µl (1,119 mmol) de T3P® et 112 mg (1,12 mmol) de 1-méthylpipérazine. Le mélange réactionnel est porté à 120°C pendant 120 minutes. Après retour à température ambiante, la réaction est hydrolysée par addition d'eau et extraite au dichlorométhane. La phase organique est séchée sur sulfate de sodium, filtrée et concentrée. Le résidu obtenu est purifié par chromatographie sur gel de silice (éluant : dichlorométhane/méthanol/ammoniaque : 94/4/2) pour conduire à 38 mg (9%) de composé **35** sous forme d'un solide jaune.
LCMS (IE, m/z) : (M+1) 476,57
RMN 1H : dH ppm (400 MHz, CDCl3) : 8,71-8,72 (1H, d, CHarom), 8,47-8,48 (1H, d, CHarom), 8,22 (1H, m, CHarom), 7,45-7,47 (1H, d, CHarom), 7,37-7,41 (1H, t, CHarom), 7,33 (1H, s, NH), 7,18-7,21 (2H, m, CHarom), 7,06-7,09 (1H, dd, CHarom), 6,54-6,57 (1H, dd, CHarom), 4,25-4,38 (4H, m, CH₂), 3,89 (6H, m, CH₂), 3,39 (2H, m, CH₂), 2,35-2,52 (4H, t, CH₂), 2,33 (3H, s, CH₃).

### Composé 36 :

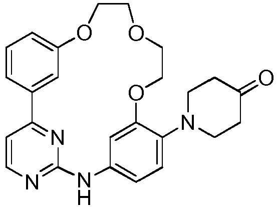

A 70 mg (0,14 mmol) du composé **31** est additionné, au goutte à goutte, 1 mL d'une solution d'acide chlorhydrique dans l'isopropanol (5N). La solution est agitée à 100°C pendant 12 h. Le solide formé est placé en milieu basique et extrait à l'acétate d'éthyle. La phase organique est séchée sur sulfate de sodium, filtrée et concentrée pour conduire à 38 mg (57%) du composé **36** sous la forme d'un solide jaune.
LCMS (IE, m/z) : (M+1) 447,50
RMN 1H : dH ppm (400 MHz, DMSO) 9,67 (1H, s, NH), 8,52-8,54 (1H, d, CHarom), 8,51-8,52 (1H, d, CHarom), 8,11 (1H, s, CHarom), 7,63-7,65 (1H, d, CHarom), 7,43-7,47 (1H, t, CHarom), 7,35-7,37 (1H, d, CHarom), 7,17-7,20 (1H, dd, CHarom), 6,91-6,93 (1H, d, CHarom), 6,80-6,83 (1H, dd, CHarom), 4,24-4,29 (4H, m, CH₂), 3,86-3,89 (2H, t, CH₂), 3,80-3,82 (2H, m, CH₂), 3,23-3,26 (4H, t, CH₂), 2,45 (4H, m, CH₂).

### Composé 37 :

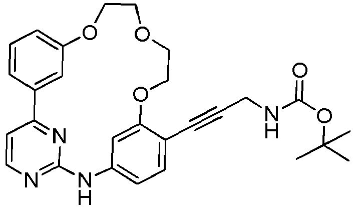

Dans un ballon de 50 mL sont mélangés 1 g (2,335 mmol) de composé **3**, 0,256 g (0,537 mmol) de 2-(dicyclohexylphosphino)-2',4',6'-triisopropylbiphényle, 0,045 g (0,117 mmol) de chlorure de benzonitrile palladium(II), 0,652 g (4,20 mmol) d'ester tert-butylique de l'acide pro-2-yl-carbamique et 2,28 g (7 mmol) de carbonate de césium dans
10 mL de diméthylformamide à température ambiante. On ajoute au mélange réactionnel 0,90 g (2,80 mmol) de bromure de tétrabutylammonium. Le milieu réactionnel est porté à 80°C durant 15 heures. Après retour à température ambiante, la réaction est hydrolysée par addition lente d'eau et extraite à l'acétate d'éthyle. La phase organique est séchée sur sulfate de sodium, filtrée et concentrée. Le résidu obtenu est purifié par chromatographie sur gel de silice (éluant : acétate d'éthyle/cyclohexane : 40/60) pour conduire à 144 mg (12%) de composé 37 sous forme d'un solide jaune.
LCMS (IE, m/z) : (M+1) 503,56
RMN 1H : dH ppm (400 MHz, DMSO) : 10,02 (1H, s, NH), 8,58-8,62 (2H, m, CHarom), 8,09 (1H, s, CHarom), 7,64-7,66 (1H, d, CHarom), 7,44-7,48 (2H, m, CHarom), 7,33 (1H, m, CHarom), 7,17-7,24 (2H, m, CHarom), 6,83-6,86 (1H, m, CHarom), 4,25-4,27 (4H, m, CH₂), 3,96-3,97 (2H, m, CH₂), 3,80-3,86 (4H, m, CH₂), 1,40 (9H, s, CH₃).

### Composé 38 :

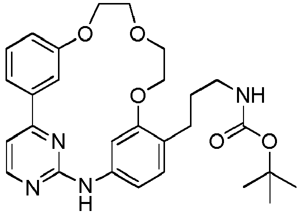

Dans un ballon et sous argon, introduire 144 mg (0,28 mmol) de composé **37** dans 10 mL d'un mélange de THF/MeOH (1/1). Dégazer le milieu sous argon et sous vide. Ajouter

30,5 mg (0,029 mmol) de Pd-C. Dégazer le milieu sous argon et sous vide puis placer un ballon d'hydrogène. Le mélange réactionnel est agité à 25°C durant la nuit puis filtré sur de la silice et rincé avec de l'acétate d'éthyle, le solvant est évaporé et le solide formé est filtré et séché sous vide pour conduire à 132 mg (91%) de composé **38** sous forme d'un solide jaune.
LCMS (IE, m/z) : (M+1) 507,59
RMN 1H : dH ppm (400 MHz, DMSO) : 9,70 (1H, s, NH), 8,54-8,55 (1H, d, CHarom), 8,45-8,47 (1H, d, CHarom), 8,12 (1H, s, CHarom), 7,62-7,64 (1H, d, CHarom), 7,43-7,47 (1H, t, CHarom), 7,36-7,38 (1H, d, CHarom), 7,17-7,18 (1H, dd, CHarom), 7,00-7,02 (1H, dd, CHarom), 6,76-6,80 (2H, m, CHarom), 4,17-4,25 (4H, m, CH₂), 3,79-3,89 (4H, m, CH₂), 2,89-2,93 (2H, m, CH₂), 2,40 (2H, m, CH₂), 1,56-1,67 (2H, m, CH₂), 1,38 (9H, s, CH₃).

### Composé 39 :

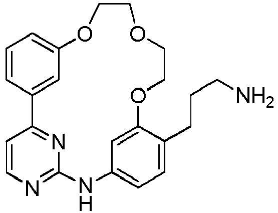

A 132 mg (0,261 mmol) de composé **38** sont additionnés, au goutte à goutte, 2 mL d'une solution d'acide chlorhydrique dans l'isopropanol (5N). La solution est agitée à 40°C pendant 2h45 minutes. Evaporation à sec, le solide formé est trituré dans l'éther puis recristallisé dans l'isopropanol pour obtenir 100 mg (87%) du composé **39** sous la forme d'une poudre jaune.
LCMS (IE, m/z) : (M+1) 407,20
RMN 1H : dH ppm (400 MHz, DMSO) : 9,78 (1H, s, NH), 8,56-8,57 (1H, d, CHarom), 8,50-8,51 (1H, d, CHarom), 8,12 (1H, m, CHarom), 7,64-7,66 (1H, d, CHarom), 7,45-7,49 (1H, t, CHarom), 7,39-7,41 (1H, d, CHarom), 7,18-7,21 (1H, dd, CHarom), 7,04-7,06 (1H, d, CHarom), 6,79-6,81 (1H, dd, CHarom), 4,21-4,25 (4H, m, CH₂), 3,88-3,91 (2H, t, CH₂), 3,80-3,82 (2H, m, CH₂), 2,73-2,79 (2H, m, CH₂), 2,54-2,58 (2H, t, CH₂), 1,76-1,84 (2H, m, CH₂).

### Composé 40 :

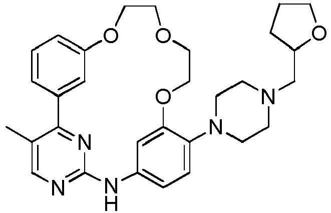

Le composé **40** a été préparé selon le protocole décrit pour la préparation du composé **3** à partir de 115 mg de composé **3** et de 177 mg de l'amine (1-((tétrahydrofuran-2-yl) méthyl)pipérazine) pour conduire à 21 mg (15%) du composé **40**.
LCMS (IE, m/z) : (M+1) 532,64
RMN 1H : dH ppm (400 MHz, DMSO) : 9,46 (1H, s, NH), 8,46-8,47 (1H, d, CHarom), 8,40 (1H, s, CHarom), 7,59 (1H, s, CHarom), 7,39-7,43 (1H, t, CHarom), 7,28-7,30 (1H, d, CHarom), 7,15-7,17 (1H, dd, CHarom), 6,74-6,81 (2H, m, CHarom), 4,29-4,31 (2H, m, CH₂), 4,07-4,12 (2H, m, CH₂), 3,90-3,97 (1H, m, CH), 3,78-3,82 (4H, m, CH₂), 3,70-3,76 (1H, m, CH), 3,57-3,62 (1H, m, CH), 2,89 (4H, m, CH₂), 2,49-2,68 (5H, m, CH₂), 2,38-2,41 (1H, m, CH), 2,29 (3H, m, CH₃), 1,88-1,96 (1H, m, CH), 1,73-1,83 (2H, m, CH₂), 1,43-1,52 (1H, m, CH).

### Composé 41 :

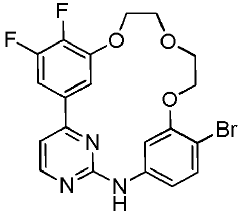

### Etape 1 : Intermédiaire 13

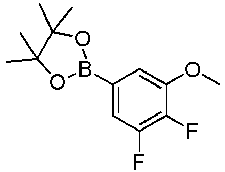

Dans un ballon de 50 mL, introduire 1 g (4,48 mmol) de 5-bromo-2,3-difluoroanisole dans 25 mL de dioxane puis ajouter 1,708 g (6,73 mmol) de bis(pinacolato)diboron, 1,320 g (13,45 mmol) d'acétate de potassium et 0,183 g (0,224 mmol) du complexe bis (diphénylphosphino)ferrocène-palladium(II) dichlorure dichloromethane. Le mélange réactionnel est agité à 80°C durant la nuit. Après retour à température ambiante, la réaction est hydrolysée par addition lente d'eau et extraite à l'acétate d'éthyle. La phase organique est séchée sur sulfate de sodium, filtrée et concentrée. Le résidu obtenu est purifié par chromatographie sur gel de silice (éluant : cyclohexane/acétate d'éthyle : 95/5) pour conduire à 197 mg (15%) de l'intermédiaire 13 sous forme d'un solide jaune.
LCMS (IE, m/z) : (M+1) 271,08
RMN 1H : dH ppm (400 MHz, DMSO) : 7,13-7,17 (2H, m, CHarom), 3,90 (3H, m, CH₃), 1,30 (12H, m, CH₃).

### Etape 2 : Intermédiaire 14

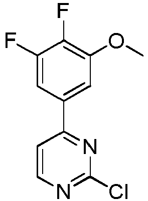

Dans un réacteur micro-ondes sont mélangés 114 mg (0,771 mmol) de 2,4-dichloropyrimidine, 197 mg (0,729 mmol) de l'intermédiaire 13, 42,1 mg (0,036 mmol) de Pd(PPh₃)₄ et 6 mL de tétrahydrofurane. Le mélange réactionnel est porté à 150°C pendant 30 minutes. Après retour à température ambiante, la réaction est hydrolysée par addition d'eau et extraite à l'acétate d'éthyle. La phase organique est séchée sur sulfate de sodium, filtrée et concentrée. Le résidu obtenu est purifié par chromatographie sur gel de silice (éluant : cyclohexane/acétate d'éthyle : 70/30) pour conduire à 112 mg (60%) de l'intermédiaire 14 sous forme d'un solide blanc.
LCMS (IE, m/z) : (M+1) 257,63
RMN 1H : dH ppm (400 MHz, DMSO) : 8,88-8,90 (1H, d, CHarom), 8,27-8,28 (1H, d, CHarom), 7.85-7,90 (1H, m, CHarom), 7,79-7,81 (1H, m, CHarom), 4,02 (3H, m, CH₃).

### Etape 3 : Intermédiaire 15

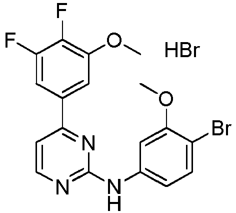

A une solution de 1,25 g (4,87 mmol) de l'intermédiaire 14 et de 0,984 g (4,87 mmol) de 4-bromo-3-méthoxyaniline dans 28 mL de n-butanol est additionné 0,97 mL d'une solution d'acide chlorhydrique (5N). Le milieu réactionnel est agité à 80°C toute la nuit. Après retour à la température ambiante, le solide formé est filtré rincé avec du n-butanol pour conduire à 1,4 g (68%) de l'intermédiaire 15 sous forme d'une poudre jaune.
LCMS (IE, m/z) : (M+1) 423,22
RMN 1H : dH ppm (400 MHz, DMSO) : 9,90 (1H, s, NH), 8,92-8,94 (1H, d, CHarom), 7,82-7,87 (1H, m, CHarom), 7,78-7,80 (1H, d, CHarom), 7,74-7,75 (1H, d, CHarom), 7,57-7,58 (1H, d, CHarom), 7,47-48 (1H, d, CHarom), 7,38-7,41 (1H, dd, CHarom), 4,01 (3H, s, CH₃), 3,86 (3H, s, CH₃).

### Etape 4 : Intermédiaire 16

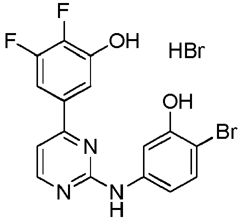

A une solution de 1,4 g (3,32 mmol) de l'intermédiaire 15 dans 36 mL de dichlorométhane est additionné 1,56 mL (16,58 mmol) de tribromo-borane à -78°C. Le milieu réactionnel est alors agité à 45°C pendant 4 h puis toute la nuit à température ambiante. 20 mL de méthanol sont additionnés au mélange réactionnel à 0°C puis on chauffe à 35°C pendant 25 minutes. Le solide formé est filtré puis lavé deux fois avec 20 mL d'éther pour conduire à 1,54 g (98%) de l'intermédiaire 16 sous la forme d'une poudre jaune.
LC-MS (IE m/z) : (M+H⁺) : 395.17 + 397,2
RMN 1H : dH ppm (400 MHz, DMSO) : 9,75 (1H, s, NH), 8,55-8,56 (1H, d, CHarom), 7,61-7,63 (2H, m, CHarom), 7,49-7,50 (1H, d, CHarom), 7,35-7,37 (2H, m, CHarom), 7,23-7,26 (1H, dd, CHarom).

### Etape 5 : Composé 41

A une solution sous agitation de 0,7 g (1,47 mmol) de l'intermédiaire 16 dans 70 mL de *N*,*N*-diméthylformamide est ajouté 0,34 g (1,47 mmol) de carbonate de potassium puis 0,32 g (1,47 mmol) de 1-bromo-2-(2-bromoéthoxy)éthane dans 35 mL de *N,N-*diméthylformamide pendant une heure. Le mélange réactionnel est agité à 75°C pendant 20 heures. Après retour à température ambiante, le solvant est évaporé, de l'eau est additionnée et le solide formé est filtré et séché sous vide pour conduire à 0,53 g (78%) du composé **41** sous forme d'une poudre beige.
LCMS (IE, m/z) : (M+1) 465,26
RMN 1H : dH ppm (400 MHz, DMSO) : 9,98 (1H, s, NH), 8,60-8,60 (1H, d, CHarom), 8,54 (1H, s, CHarom), 8,05-8,07 (1H, d, CHarom), 7,81-7,85 (1H, m, CHarom), 7,43-7,47 (2H, m, CHarom), 6,83-6,85 (1H, dd, CHarom), 4,41 (2H, m, CH₂), 4,23 (2H, m, CH₂), 3,81 (4H, m, CH₂).

### Composé 42 :

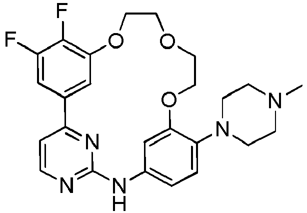

Le composé **42** a été préparé selon le protocole décrit pour la préparation du composé **3** à partir de 272 mg de composé **41** et de 117 mg de l'amine 1-méthylpipérazine pour conduire à 14 mg du composé **42** avec un rendement de 8%.
LCMS (IE, m/z) : (M+1) 484,51
RMN 1H : dH ppm (400 MHz, DMSO) : 9,66 (1H, s, NH), 8,53-8,55 (1H, d, CHarom), 8,34-8,35 (1H, d, CHarom), 8,07-8,09 (1H, d, CHarom), 7,82-7,87 (1H, m, CHarom), 7,39-7,40 (1H, d, CHarom), 6,82-6,87 (2H, m, CHarom),4,37-4,41 (2H, m, CH₂), 4,21-4,23 (2H, m, CH₂), 3,75-3,81 (4H, m, CH₂), 2,95 (4H, m, CH₂), 2,46 (4H, m, CH₂), 2,21 (3H, m, CH₃).

### Composé 43 :

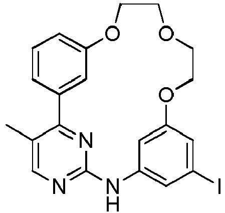

### Etape 1 : Intermédiaire 17

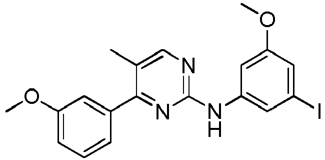

A une solution de 0,44 g (1,79 mmol) de l'intermédiaire 10 et de 0,446 g (1,790 mmol) de 3-iodo-5-méthoxyaniline dans 10 mL de n-Butanol est additionné 0,35 mL d'une solution d'acide chlorhydrique (5N). Le milieu réactionnel est agité à 80°C toute la nuit. Après retour à la température ambiante, la réaction est hydrolysée par addition lente d'une solution de NaHCO₃ et extraite à l'acétate d'éthyle. La phase organique est séchée sur sulfate de sodium, filtrée et concentrée. Le résidu obtenu est purifié par chromatographie sur gel de silice (éluant : cyclohexane/acétate d'éthyl : 90/10) pour conduire à 441 mg (55 %) de l'intermédiaire 17 sous forme d'un solide blanc.
LCMS (IE, m/z) : (M+1) 448,27

### Etape 2 : Intermédiaire 18

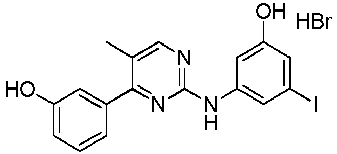

A une solution de 441 mg (0,98 mmol) de l'intermédiaire 17 dans 11 mL de dichlorométhane est additionné 0,47 mL (4,94 mmol) de tribromo-borane à -78°C. Le milieu réactionnel est alors agité à 45°C pendant 5 heures puis toute la nuit à température ambiante. 20 mL de méthanol sont additionnés au mélange réactionnel à 0°C qui est chauffé à 35°C pendant 25 minutes. Le solide formé est filtré puis lavé deux fois avec 20 mL d'éther pour conduire à 0,50 g (99 %) de l'intermédiaire 18 sous la forme d'une poudre jaune.
LCMS (IE, m/z) : (M+1) 420,01
RMN 1H : dH ppm (400 MHz, DMSO) : 9,58 (1H, s, NH), 8,42 (1H, s, CHarom), 7,70 (1H, s, CHarom), 7,29-7,33 (2H, m, CHarom), 7,04-7,09 (2H, m, CHarom), 6,87-6,90 (1H, d, CHarom), 6,69 (1H, d, CHarom), 2.21 (3H, s, CH₃).

### Etape 3 : Composé 43

A une solution sous agitation de 0,54 g (1,09 mmol) de l'intermédiaire 18 dans 55 mL de *N*,*N*-diméthylformamide est ajouté 0,75 g (5,47 mmol) de carbonate de potassium puis

0,137 mL (1,09 mmol) de 1-bromo-2-(2-bromoéthoxy)éthane dans 23 mL de *N,N-*diméthylformamide pendant une heure. Le mélange réactionnel est agité à 80°C pendant 20 heures. Après retour à température ambiante, le solvant est évaporé, de l'eau est additionnée et le solide formé est filtré et séché sous vide pour conduire à 0,25 g (64 %) du composé **43** sous forme d'une poudre beige.
LCMS (IE, m/z) : (M+1) 490,30
RMN 1H : dH ppm (400 MHz, DMSO) : 9,74 (1H, s, NH), 8,62 (1H, s, CHarom), 8,47 (1H, s, CHarom), 7,74 (1H, s, CHarom), 7,41-7,42 (2H, m, CHarom), 7,20 (1H, s, CHarom), 7,09-7,14 (1H, m, CHarom), 6,82 (1H, s, CHarom), 4,30-4,33 (2H, t, CH₂), 4,00-4,02 (2H, t, CH₂), 3,82-3,86 (4H, m, CH₂), 2,38 (3H, s, CH₃).

### Composé 44 :

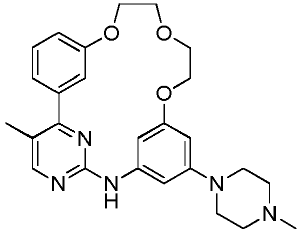

Le composé **44** a été préparé selon le protocole décrit pour la préparation du composé **3** à partir de 490 mg de composé **43** et de 401 mg de l'amine 1-méthylpipérazine pour conduire à 156 mg (34%) du composé **44.**
LCMS (IE, m/z) : (M+1) 462,5
RMN 1H : dH ppm (400 MHz, DMSO) : 9,36 (1H, s, NH), 8,42 (1H, s, CHarom), 8,07 (1H, s, CHarom), 7,76 (1H, s, CHarom), 7,40-7,41 (1H, d, CHarom), 7,08-7,12 (1H, m, CHarom), 6,41 (1H, s, CHarom), 6,10 (1H, s, CHarom), 4,30-4,32 (2H, t, CH₂), 3,99-4,00 (2H, t, CH₂), 3,81-3,86 (4H, m, CH₂), 3,79 (4H, m, CH₂), 2,44 (4H, m, CH₂), 2,36 (3H, m, CH₃), 2,22 (3H, m, CH₃).

### Composé 45 :

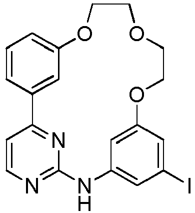

### Etape 1 : 2-chloro-4-(3-méthoxyphényl)pyrimidine (Intermédiaire 19)

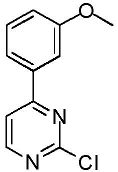

A 11,32 g (76 mmol) de 2,4-dichloropyrimidine dans 550 mL de THF anhydre sont additionnés 11 g (72,4 mmol) d'acide 3-méthoxyphényl-boronique. Le milieu réactionnel est agité à température ambiante durant 10 minutes puis sont additionnés à température ambiante 19,1 g de carbonate de sodium dissous dans 80 mL d'eau puis 4,18 g (3,62 mmol) de tétrakis(triphénylphosphine)palladium(0). Le milieu réactionnel est agité à 90°C pendant 16 heures. De l'acétate d'éthyle est additionné et la phase organique est lavée avec une solution saturée de chlorure de sodium, séchée sur sulfate de magnésium, filtrée. Le filtrat est évaporé et le résidu purifié par chromatographie sur colonne de silice à l'aide du Companion® (éluant : heptane/acétate d'éthyle 0 à 10% puis dichlorométhane/acétate d'éthyle 10%) pour conduire à 15,23 g (95%) de 2-chloro-4-(3-méthoxyphényl)pyrimidine sous forme d'un solide blanc.
LCMS (IE, m/z) : (M+1) 221,65
RMN 1H : dH ppm (400 MHz, DMSO) : 8,82-8,84 (1H, d, CHarom), 8,17-8,19 (1H, d, CHarom), 7,77-7,79 (1H, d, CHarom), 7,70-7,71 (1H, d, CHarom), 7,47-7,51 (1H, d, CHarom), 7,17-7,20 (1H, t, CHarom), 3,86 (3H, s, CH₃).

### Etape 2 : Intermédiaire 20

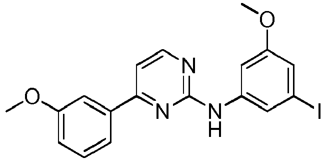

A une solution de 0,5 g (2,26 mmol) de l'intermédiaire 19 et de 0,56 g (2,26 mmol) de 3-iodo-5-méthoxyaniline dans 13 mL de n-butanol est additionné 0,45 mL d'une solution d'acide chlorhydrique (5N). Le milieu réactionnel est agité à 80°C toute la nuit. Après retour à la température ambiante, la réaction est hydrolysée par addition lente d'une solution de NaHCO₃ et extraite à l'acétate d'éthyle. La phase organique est séchée sur sulfate de sodium, filtrée et concentrée. Le résidu obtenu est purifié par chromatographie sur gel de silice (éluant : cyclohexane/acétate d'éthyle : 80/20) pour conduire à 693 mg (70%) de l'intermédiaire 20 sous forme d'un solide jaune.
LCMS (IE, m/z) : (M+1) 434,24

### Etape 3 : Intermédiaire 21

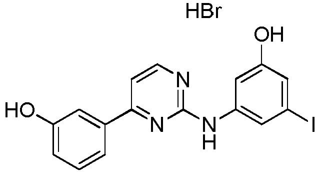

A une solution de 693 mg (1,6 mmol) de l'intermédiaire 20 dans 18 mL de dichlorométhane est additionné 0,75 mL (1,6 mmol) de tribromo-borane à -78°C. Le milieu réactionnel est alors agité à 45°C pendant 5 h puis toute la nuit à température ambiante. 20 mL de méthanol sont additionnés au mélange réactionnel à 0°C puis on chauffe à 35°C pendant 25 minutes. Le solide formé est filtré puis lavé deux fois avec 20 mL d'éther pour conduire à 0,76 g (98%) de l'intermédiaire 21 sous la forme d'une poudre jaune.
LCMS (IE, m/z) : (M+1) 406,18
RMN 1H : dH ppm (400 MHz, DMSO) : 9,69 (1H, s, NH), 8,54-8,55 (1H, d, CHarom), 7,79 (1H, s, CHarom), 7,58-7,60 (1H, d, CHarom), 7,52 (1H, m, CHarom), 7,32-7,36 (3H, m, CHarom), 6,94-6,96 (1H, dd, CHarom), 6,75 (1H, t, CH₃).

### Etape 4 : Composé 45

A une solution sous agitation de 0,76 g (1,56 mmol) de l'intermédiaire 21 dans 78 mL de *N*,*N*-diméthylformamide est ajouté 1,08 g (7,82 mmol) de carbonate de potassium puis
0,19 mL (1,56 mmol) de 1-bromo-2-(2-bromoéthoxy)éthane dans 34 mL de *N,N-*diméthylformamide pendant une heure. Le mélange réactionnel est agité à 80°C pendant 20 heures. Après retour à température ambiante, le solvant est évaporé, de l'eau est additionnée et le solide formé est filtré et séché sous vide pour conduire à 0,30 g (40%) du composé **45** sous forme d'une poudre beige.
LCMS (IE, m/z) : (M+1) 476,28

### Composé 46 :

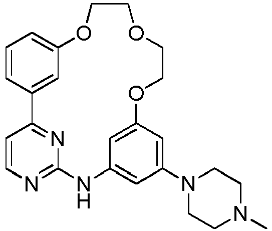

Le composé **46** a été préparé selon le protocole décrit pour la préparation du composé **3** à partir de 300 mg de composé **45** et de 253 mg de l'amine 1-méthylpipérazine pour conduire à 115 mg du composé **46** avec un rendement de 41%.
LCMS (IE, m/z) : (M+1) 448,52
RMN 1H : dH ppm (400 MHz, DMSO) : 9,54 (1H, s, NH), 8,54-8,55 (1H, d, CHarom), 8,27 (1H, s, CHarom), 8,14 (1H, s, CHarom), 7,70-72 (1H, d, CHarom), 7,44-7,46 (1H, d, CHarom), 7,40-7,42 (1H, d, CHarom), 7,08-7,11 (1H, dd, CHarom), 6,44 (1H, s, CHarom), 6,16 (1H, s, CHarom), 4,28-4,31 (2H, t, CH₂), 4,02-4,05 (2H, t, CH₂), 3,87-3,93 (4H, m, CH₂), 3,16 (4H, m, CH₂), 2,45 (4H, m, CH₂), 2,23 (3H, m, CH₃)

### 2. Activité biologique des composés selon l'invention

Les abréviations suivantes ont été utilisées :
- ATP: : Adénosine-5'-triphosphate
- IMDM: : Iscove's Modified Dulbecco's Médium
- PSFG: : Penicilline Streptomycine FunGizone
- RPMI: : Roswell Park Mémorial Institute médium
- SVF: : Sérum de veau foetal

### Mesure des activités d'inhibition in vitro des composés selon l'invention :

Les enzymes recombinantes FLT3 (#PV3182), JAK2 (#PV4210) et JAK3 (#PV3855) ont été achetées chez Life Technologies. Les protéines FLT3-ITD (#0778-0000-1) et FLT3^{D835Y} (#14-610) ont respectivement été achetées chez Proquinase et chez Merck Millipore. L'ensemble des essais ont été réalisés en plaques 384-puits. Le principe de ces tests de liaison est basé sur la méthodologie LanthaScreen® TR-FRET de Life Technologies.

Essais FLT3. Le mélange réactionnel de 15 µl de volume total contient les composés suivants : 15nM de FLT3, FLT3-ITD ou FLT3D^{835Y}, 3nM de kinase traceur 236 (Life Technologies, #PV5592) et 6nM d'anticorps LanthaScreen® anti-GST couplé à un chélate d'europium (Life Technologies, #PV5594) pour FLT3-ITD et FLT3D^{835Y} ou 6nM d'anticorps LanthaScreen® anti-His couplé à un chélate d'europium (Life Technologies, #PV5596) pour FLT3.

Essais JAKs. Le mélange réactionnel de 15 µl de volume total contient les composés suivants : 15nM de JAK2 ou JAK3, 150nM de kinase traceur 236 (Life Technologies, #PV5592) pour JAK2 ou 3nM pour JAK3 et 6nM d'anticorps LanthaScreen® anti-GST couplé à un chélate d'europium (Life Technologies, #PV5594) pour les deux enzymes. Les composés sont évalués à 8 concentrations différentes préparées en réalisant des dilutions à partir d'une solution stock de départ à 10mM dans le diméthylsulfoxyde (DMSO) (Sigma, #D8418). La concentration finale en DMSO dans le test est de 1%. La réaction est réalisée à 25°C pendant 1 heure et détectée sur le lecteur EnVision® (Perkin Elmer) selon les recommandations du fournisseur Life Technologies.

Les résultats sont présentés (Tableau 1) sous forme de valeurs de concentration en composé permettant d'inhiber 50% de l'activité kinase, IC₅₀ (µM), générées à l'aide du logiciel Prism (GraphPad).

**Tableau 1**

| **Composé/enzyme** | **FLT3 IC₅₀ (µM)** | **FLT3-ITD IC₅₀ (µM)** | **FLT3^{D835Y} IC₅₀ (µM)** | **JAK2 IC₅₀ (µM)** | **JAK3 IC₅₀ (µM)** |
|---|---|---|---|---|---|
| **4** | 0,0255 | 0,0145 | 0,035 | 0,0077 | 0,05 |
| **46** | 0,0047 | 0,0016 | 0,0062 | 0,060 | 0,10 |
| **6** | 0,11 | 0,12 | 0,0412 | 0,0052 | 0,05 |

### Mesure in vitro de l'activité antiproliférative des composés selon l'invention :

### Lignées cellulaires.

Les caractéristiques des lignées cellulaires utilisées sont les suivantes (Tableau 2).

**Tableau 2**

| **Lignées cellulaires** | **Fournisseur** | **Origine tumorale** | **Oncogèn e exprimé** | **Milieu de culture de base** | **Ensemencement (Densité cellulaire / puits)** |
|---|---|---|---|---|---|
| MV4-11 | DSMZ | Leucémie myéloïde aiguë | FLT3-ITD | IMDM, 10% SVF, PSFG | 0,4 10⁵ cellules/puits (100µl) |
| MOLM-13 | DSMZ | Leucémie myéloïde aiguë | FLT3-ITD | RPMI 1640, 15% SVF, PSFG | 0,3 10⁵ cellules/puits (100µl) |

### Mesure de l'activité antiproliférative.

Les lignées cellulaires MV4-11 et MOLM-13 sont cultivées dans le milieu de culture précisé dans le tableau 2 ci-dessus et selon les recommandations du fournisseur. Les essais sont réalisés en plaques de 96 puits. Les cellules sont dédoublées à J0. A J1, elles sont ensemencées et traitées avec les composés à différentes concentrations et incubées pendant 72h à 37°C et 5% de CO₂. La dilution des composés à partir de solutions stock dans le DMSO (Sigma, #D8418) a été faite de manière semi-logarithmique pour une concentration finale dans le milieu de culture de 0,1%. Au jour 4, la viabilité cellulaire est évaluée en dosant l'ATP libéré par les cellules vivantes à l'aide du kit ATPLite® (Perkin Elmer, #6016947). Les valeurs d'EC₅₀ (concentration en composé nécessaire pour obtenir 50% de l'effet maximum) sont calculées à l'aide d'un logiciel d'ajustement de courbe. Les résultats sous forme de valeurs d'EC₅₀ (en M) sont présentés dans le tableau 3.

**Tableau 3**

| **Composé/lignée** | **MV411** | **MOLM13** |
|---|---|---|
| **4** | 5,73E-08 | 1,89E-08 |
| **46** | 1,80E-09 | 3,90E-09 |
| **6** | 2,46E-07 | 1,45E-07 |

## Revendications

1. Composé de formule générale **(I)** suivante : 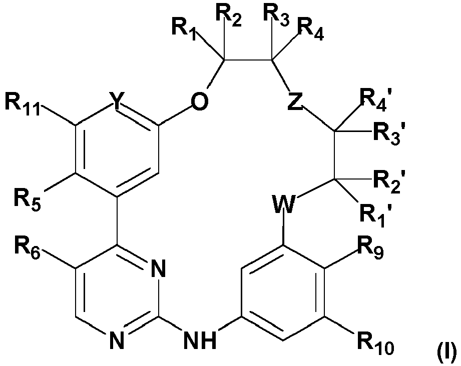 ou un sel et/ou un solvate pharmaceutiquement acceptable de celui-ci,
dans laquelle :
- **W** représente un atome d'oxygène ou de soufre,
- **Y** représente un atome d'azote ou un groupe C**Ry** avec **Ry** représentant un atome d'hydrogène, un atome d'halogène, un groupe (C₁C₆)alkyle, (C₁-C₆)halogénoalkyle, (C₁-C₆)alcoxy, (C₁-C₆)halogénoalcoxy, OH, CN, NO₂, N**R₁₂R₁₃,** CO₂H ou CO₂((C₁-C₆)alkyle),
- **Z** représente un groupe (C**R_{Q1}R_{Q2}**)ₙ**Q**(C**R_{Q3}R_{Q4}**)ₘ, avec **n** et **m** représentant, indépendamment l'un de l'autre, un nombre entier compris entre 0 et 3,
- **Q** représente O, S, S(O) ou S(O)₂,
- **R_{Q1}**, **R_{Q2}**, **R_{Q3}** et **R_{Q4}** représentent, indépendamment les uns des autres, un atome d'hydrogène ou un groupe (C₁-C₆)alkyle,
- **R₁, R₂, R₁'** et **R₂'** représentent, indépendamment les uns des autres, un atome d'hydrogène ou un groupe (C₁-C₆)alkyle,
- **R₃, R₄, R₃'** et **R₄'** représentent, indépendamment les uns des autres, un atome d'hydrogène, un groupe (C₁-C₆)alkyle ou OH ou **R₃** et **R₄** et/ou **R₃'** et **R₄'** forment ensemble, avec l'atome de carbonne qui les porte, un carbocycle ou hétérocycle monocyclique éventuellement substitué,
- **R₅** et **R**₆ représentent, indépendamment l'un de l'autre, un atome d'hydrogène, un atome d'halogène, un groupe (C₁-C₆)alkyle, (C₁-C₆)halogénoalkyle, (C₁-C₆)alcoxy, (C₁-C₆)halogénoalcoxy, (C₁-C₆)thioalcoxy, (C₁-C₆)halogénothioalcoxy, OH, SH, CN, NO₂, ou **NR₇R₈,**
- **R₉** et **R₁₀** représentent, indépendamment l'un de l'autre, un atome d'hydrogène, un atome d'halogène, un groupe (C₁-C₆)alkyle éventuellement substitué, (C₂-C₆)alcényle éventuellement substitué, (C₂-C₆)alcynyle éventuellement substitué, (C₁-C₆)alcoxy éventuellement substitué, (C₁-C₆)thioalcoxy éventuellement substitué, CN, NO₂, OH, SH, N**R₁₄R₁₅**, CO₂**R₅₄**, CON**R₅₅R₅₆**, un carbocycle éventuellement substitué ou un hétérocycle éventuellement substitué,
- **R₁₁** représente un atome d'hydrogène, un atome d'halogène, ou un groupe (C₁-C₆)alkyle, (C₁-C₆)halogénoalkyle, (C₁-C₆)alcoxy ou (C₁-C₆)halogénoalcoxy, et
- **R₇, R₈, R₁₂, R₁₃, R₁₄, R₁₅, R₅₄, R₅₅** et **R₅₆** représentent, indépendamment les uns des autres, un atome d'hydrogène ou un groupe (C₁-C₆)alkyle éventuellement substitué, (C₂-C₆)alcényle éventuellement substitué, ou (C₂-C₆)alcynyle éventuellement substitué, ou **R₇** et **R₈, R₁₂** et **R₁₃, R₁₄** et **R₁₅** et/ou **R₅₅** et **R₅₆,** indépendamment les uns des autres, forment avec l'atome d'azote qui les portent un hétérocycle azoté éventuellement substitué.

2. Composé selon la revendication 1, **caractérisé en ce que Z** représente un atome d'oxygène et **R₁, R₂, R₃, R₄, R₁', R₂', R₃'** et **R₄'** représentent chacun un atome d'hydrogène.

3. Composé selon l'une quelconque des revendications 1 et 2, **caractérisé en ce que Y** représente un groupe C**Ry** avec **Ry** représentant un atome d'hydrogène ou un atome d'halogène tel que F.

4. Composé selon l'une quelconque des revendications 1 à 3, **caractérisé en ce que R₁₁** représente un atome d'hydrogène ou un atome d'halogène tel que F.

5. Composé selon l'une quelconque des revendications 1 à 4, **caractérisé en ce que R₅** et **R₆** représentent, indépendamment l'un de l'autre, un atome d'hydrogène ou un groupe (C₁-C₆)alkyle.

6. Composé selon l'une quelconque des revendications 1 à 5, **caractérisé en ce que R₉** et **R₁₀** représentent, indépendamment l'un de l'autre, un atome d'hydrogène, un atome d'halogène, CO₂**R₅₄**, CON**R₅₅R₅₆**, ou un groupe (C₁-C₆)alkyle, (C₂-C₆)alcényle, (C₂-C₆)alcynyle, (C₁-C₆)alcoxy, (C₁-C₆)thioalcoxy, (C₁-C₆)alkyl-amino, di((C₁-C₆)alkyl)amino ou hétérocycle, ledit groupe étant éventuellement substitué par un ou plusieurs substituants choisis parmi :
- un atome d'halogène,
- un groupe (C₁-C₆)alkyle éventuellement substitué par un ou plusieurs groupes choisis parmi un atome d'halogène, O**R₁₆,** S**R₁₇,** N**R₁₈R₁₉,** un carbocycle et un hétérocycle,
- les groupes oxo (=O), CN, NO₂, O**R₂₀,** S**R₂₁,** N**R₂₂R₂₃,** C(O)**R₂₄,** CO₂**R₂₅,** OC(O)**R₂₆,** S(O)**R₂₇,** SO₂**R₂₈,** N**R₂₉**C(O)**R₃₀,** C(O)N**R₃₁R₃₂,** N**R₃₃**CO₂**R₃₄,** OC(O)N**R₃₅R₃₆,** N**R₃₇**CON**R₃₈R₃₉** et OCO₂**R₄₀,**
- un carbocycle éventuellement substitué par un ou plusieurs groupes choisis parmi un atome d'halogène, un groupe (C₁-C₆)alkyle, oxo(=O), O**R₄₁,** S**R₄₂** et N**R₄₃R₄₄,**
- un hétérocycle éventuellement substitué par un ou plusieurs groupes choisis parmi un atome d'halogène, un groupe (C₁-C₆)alkyle, oxo(=O), O**R₄₅,** S**R₄₆** et N**R₄₇R₄₈,** et
- un groupe -O(CH₂)ₙO- avec n représentant un nombre entier compris entre 1 et 5, notamment compris entre 2 et 3,
où :
▪ **R₁₆** à **R₄₈** représentent, indépendamment les uns des autres, un atome d'hydrogène, un groupe (C₁-C₆)alkyle, aryle, aryl-(C₁-C₆)alkyle, hétérocycle ou hétérocycle-(C₁-C₆)alkyle,
le noyau aryle de ces groupes étant éventuellement substitué par un ou plusieurs groupes choisis parmi un atome d'halogène et un groupe (C₁-C₆)alkyle, et
le noyau hétérocycle de ces groupes étant éventuellement substitué par un ou plusieurs groupes choisis parmi un atome d'halogène, un groupe (C₁-C₆)alkyle, et oxo(=O), ou
▪ **R₂₂** et **R₂₃, R₃₁** et **R₃₂, R₃₅** et **R₃₆, R₃₈** et **R₃₉, R₄₃** et **R₄₄,** et/ou **R₄₇** et **R₄₈** forment ensemble, avec l'atome d'azote qui les porte, un hétérocycle azoté éventuellement substitué par un ou plusieurs groupes choisis parmi un atome d'halogène, un groupe (C₁-C₆)alkyle, et oxo(=O).

7. Composé selon l'une quelconque des revendications 1 à 6, **caractérisé en ce que R₉** et **R₁₀** représentent, indépendamment l'un de l'autre :
- un atome d'hydrogène ou d'halogène,
- un groupe CO₂**R₅₄** avec **R₅₄** représentant un atome d'hydrogène ou un groupe (C₁-C₆)alkyle, notamment un atome d'hydrogène,
- un groupe CON**R₅₅R₅₆** avec **R₅₅** et **R₅₆** formant avec l'atome d'azote qui les portent un hétérocycle azoté à 5, 6 ou 7 chaînons, comprenant 1 ou 2 hétéroatomes choisis parmi N et O dont au moins un atome d'azote, de préférence saturé, éventuellement substitué par un ou plusieurs substituants choisis parmi un atome d'halogène, (C₁-C₆)alkyle, oxo(=O), O**R₂₀,** N**R₂₂R₂₃,** CO₂**R₂₅,** C(O)N**R₃₁R₃₂,** N**R₃₃**CO₂**R₃₄,** OC(O)N**R₃₅R₃₆,** N**R₃₇**CON**R₃₈R₃₉** et OCO₂**R₄₀** ; notamment choisis parmi un atome d'halogène, (C₁-C₆)alkyle, oxo(=O), O**R₂₀,** N**R₂₂R₂₃,** CO₂**R₂₅** et C(O)N**R₃₁R₃₂,**
- un groupe -**Z₁**-(CH₂)**ₘ-R₄₉** dans lequel **Z₁** représente une liaison simple, CH₂-CH₂, CH=CH, C=C, O, S ou N**R₅₀,** notamment une liaison simple, CH₂-CH₂, C=C, O ou N**R₅₀ ; m** représente un nombre entier compris entre 1 et 6, notamment compris entre 1 et 4 ; **R₅₀** représente un atome d'hydrogène ou un groupe (C₁C₆)alkyle ; et **R₄₉** représente un atome d'halogène, O**R₂₀,** N**R₂₂R₂₃,** C(O)**R₂₄,** CO₂**R₂₅,** OC(O)**R₂₆,** N**R₂₉**C(O)**R₃₀,** C(O)N**R₃₁R₃₂,** N**R₃₃**CO₂**R₃₄,** OC(O)N**R₃₅R₃₆,** N**R₃₇**CON**R₃₈R₃₉** ou OCO₂**R₄₀** ; en particulier O**R₂₀,** N**R₂₂R₂₃,** CO₂**R₂₅,** C(O)N**R₃₁R₃₂,** N**R₃₃**CO₂**R₃₄,** OC(O)N**R₃₅R₃₆,** N**R₃₇**CON**R₃₈R₃₉** ou OCO₂**R₄₀** ; notamment N**R₂₂R₂₃,** N**R₃₃**CO₂**R₃₄,** ou N**R₃₇**CON**R₃₈R₃₉,** ou
- un hétérocycle à 5, 6 ou 7 chaînons, notamment à 6 ou 7 chaînons, comprenant 1 ou 2 hétéroatomes choisis parmi N et O et comprenant avantageusement au moins un atome d'azote, de préférence saturé ou aromatique, éventuellement substitué par un ou plusieurs substituants choisis parmi :
• un atome d'halogène,
• un groupe (C₁-C₆)alkyle éventuellement substitué par un ou plusieurs groupes choisis parmi un atome d'halogène, O**R₁₆**, N**R₁₈R₁₉,** un carbocycle monocyclique en C₃ à C₆ et un hétérocycle monocyclique à 3 à 6 chaînons comprenant 1 ou 2 hétéroatomes choisis parmi N et O,
• les groupes oxo (=O), O**R₂₀,** N**R₂₂R₂₃,** C(O)**R₂₄,** CO₂**R₂₅,** OC(O)**R₂₆,** N**R₂₉**C(O)**R₃₀,** C(O)N**R₃₁R₃₂,** N**R₃₃**CO₂**R₃₄,** OC(O)N**R₃₅R₃₆,** N**R₃₇**CON**R₃₈R₃₉** ou OCO₂**R₄₀** ; et plus particulièrement les groupes oxo (=O), O**R₂₀,** N**R₂₂R₂₃,** CO₂**R₂₅,** C(O)N**R₃₁R₃₂,** N**R₃₃**CO₂**R₃₄,** OC(O)N**R₃₅R₃₆,** N**R₃₇**CON**R₃₈R₃₉** ou OCO₂**R₄₀,**
• un carbocycle en C₃ à C₆ éventuellement substitué par un ou plusieurs groupes choisis parmi un atome d'halogène, un groupe (C₁-C₆)alkyle, oxo (=O), O**R₄₁** et N**R₄₃R₄₄,**
• un hétérocycle à 3 à 6 chaînons, comprenant 1 ou 2 hétéroatomes choisis parmi N et O, notamment saturé ou insaturé, de préférence saturé, éventuellement substitué par un ou plusieurs groupes choisis parmi un atome d'halogène, un groupe (C₁-C₆)alkyle, oxo (=O), O**R₄₅** et N**R₄₇R₄₈,** et
• un groupe -O(CH₂)ₙO- avec n représentant un nombre entier égal à 2 ou 3,
où :
▪ **R₁₆, R₁₈** à **R₂₀, R₂₂** à **R₂₆, R₂₉** à **R₄₀, R₄₅** et **R₄₇** à **R₄₈** représentent, indépendamment les uns des autres, un atome d'hydrogène, un groupe (C₁-C₆)alkyle, aryle, ou aryl-(C₁-C₆)alkyle, notamment un atome d'hydrogène ou un groupe (C₁-C₆)alkyle,
le noyau aryle de ces groupes étant un groupe phényle et étant éventuellement substitué par un ou plusieurs groupes choisis parmi un atome d'halogène et un groupe (C₁-C₆)alkyle, ou
▪ **R₂₂** et **R₂₃, R₃₁** et **R₃₂, R₃₅** et **R₃₆, R₃₈** et **R₃₉, R₄₃** et **R₄₄,** et/ou **R₄₇** et **R₄₈** forment ensemble, avec l'atome d'azote qui les porte, un hétérocycle azoté à 5 ou 6 chaînons, de préférence saturé, comprenant éventuellement 1 hétéroatome en plus de l'atome d'azote choisi parmi N et O, tel qu'un cycle pipérazine, pipéridine, morpholine ou pyrrolidine, l'hétérocycle étant éventuellement substitué par un ou plusieurs groupes choisis parmi un atome d'halogène, un groupe (C₁-C₆)alkyle, et oxo(=O).

8. Composé selon la revendication 1, **caractérisé en ce qu'**il est choisi parmi les composés suivants : 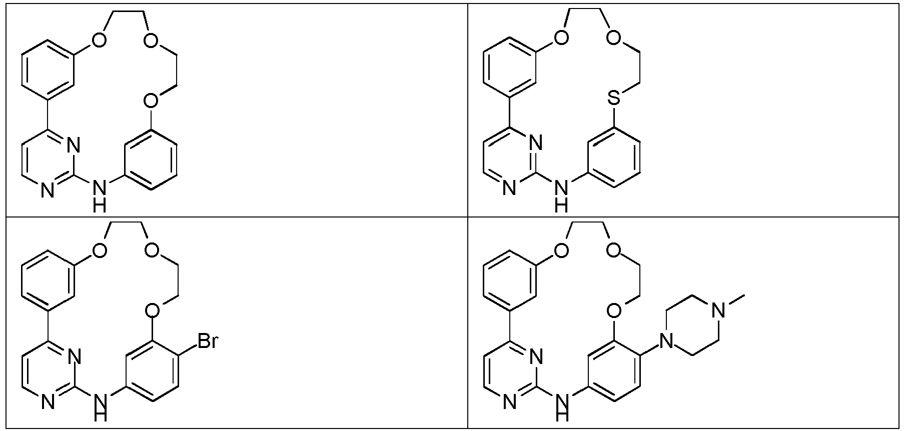 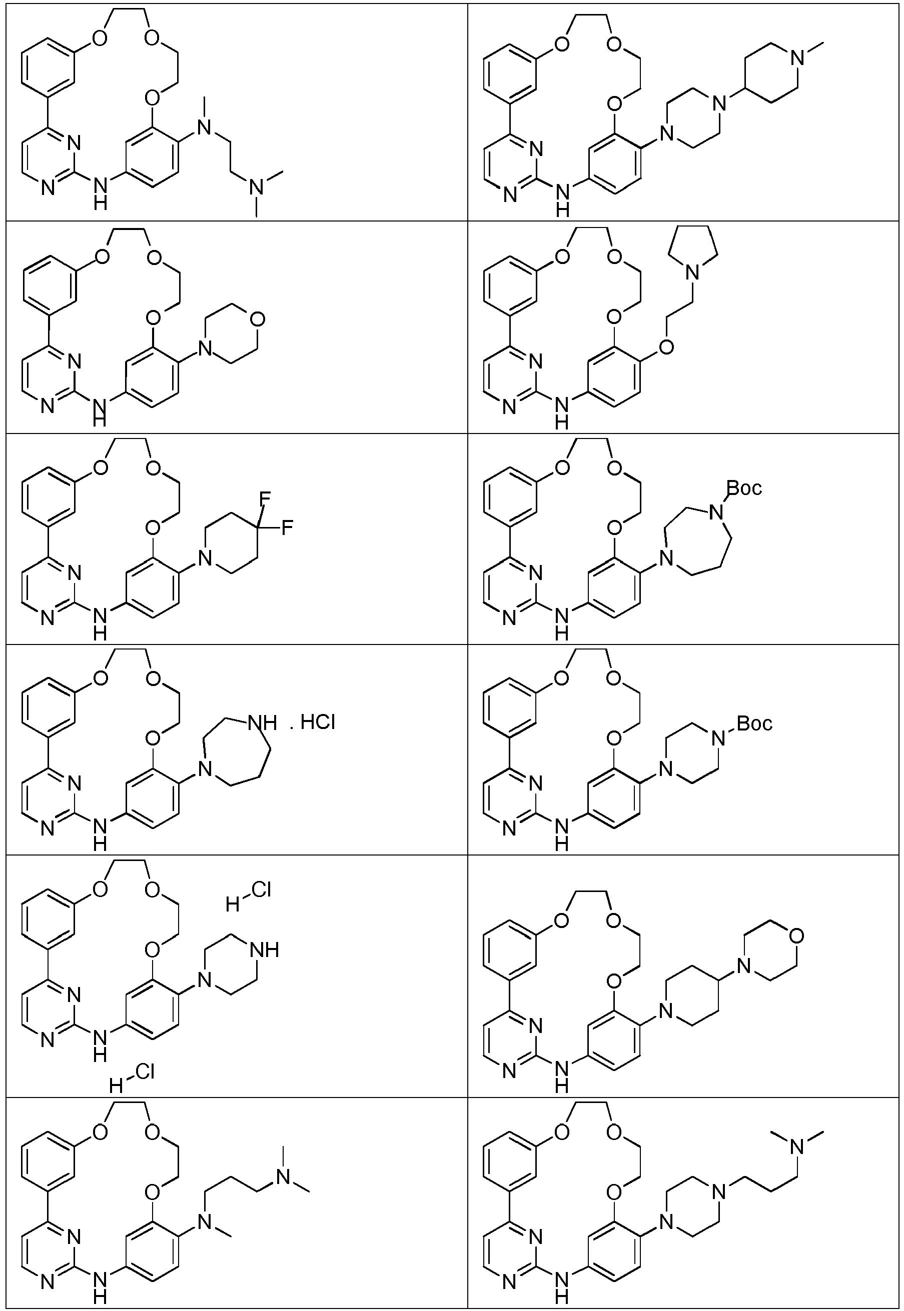 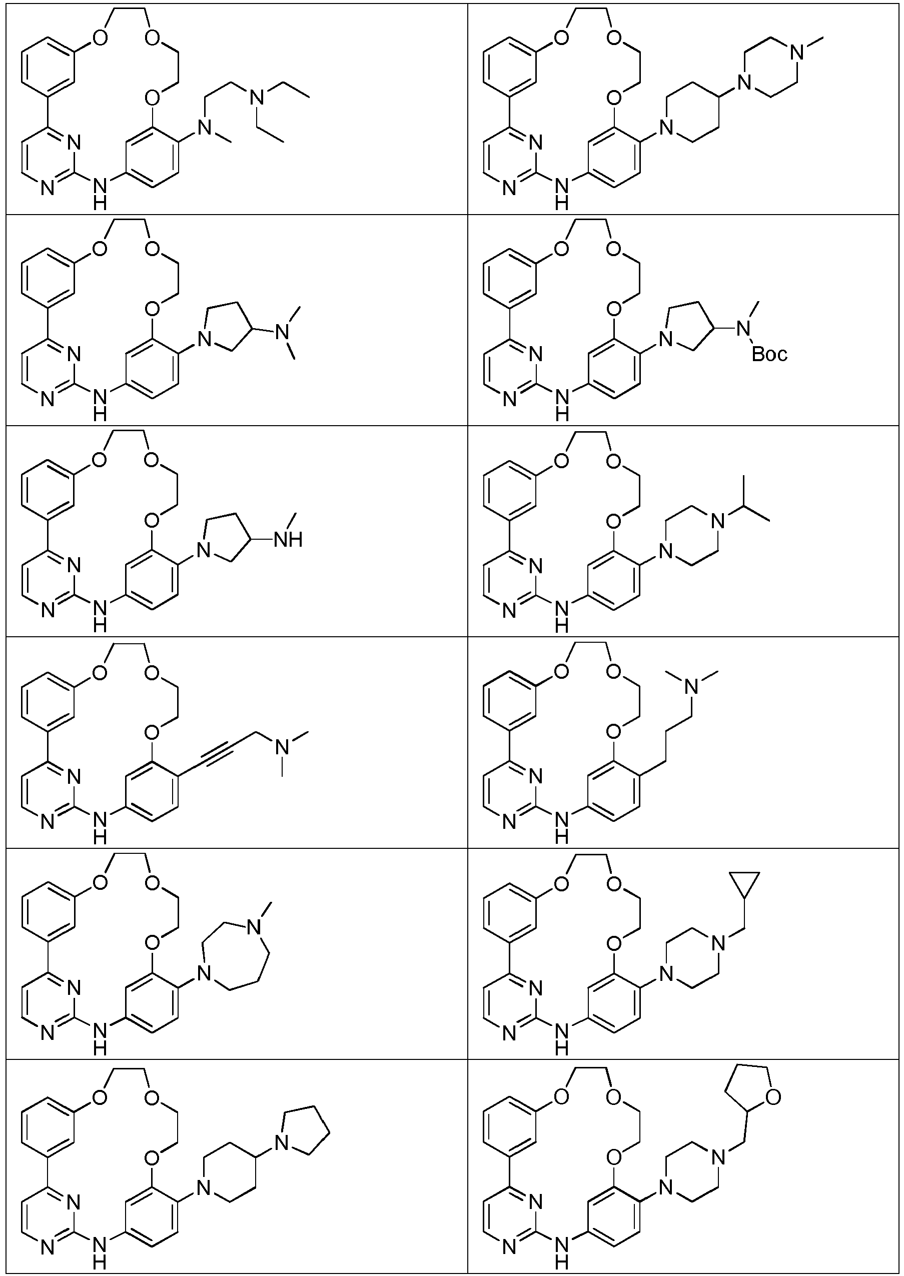 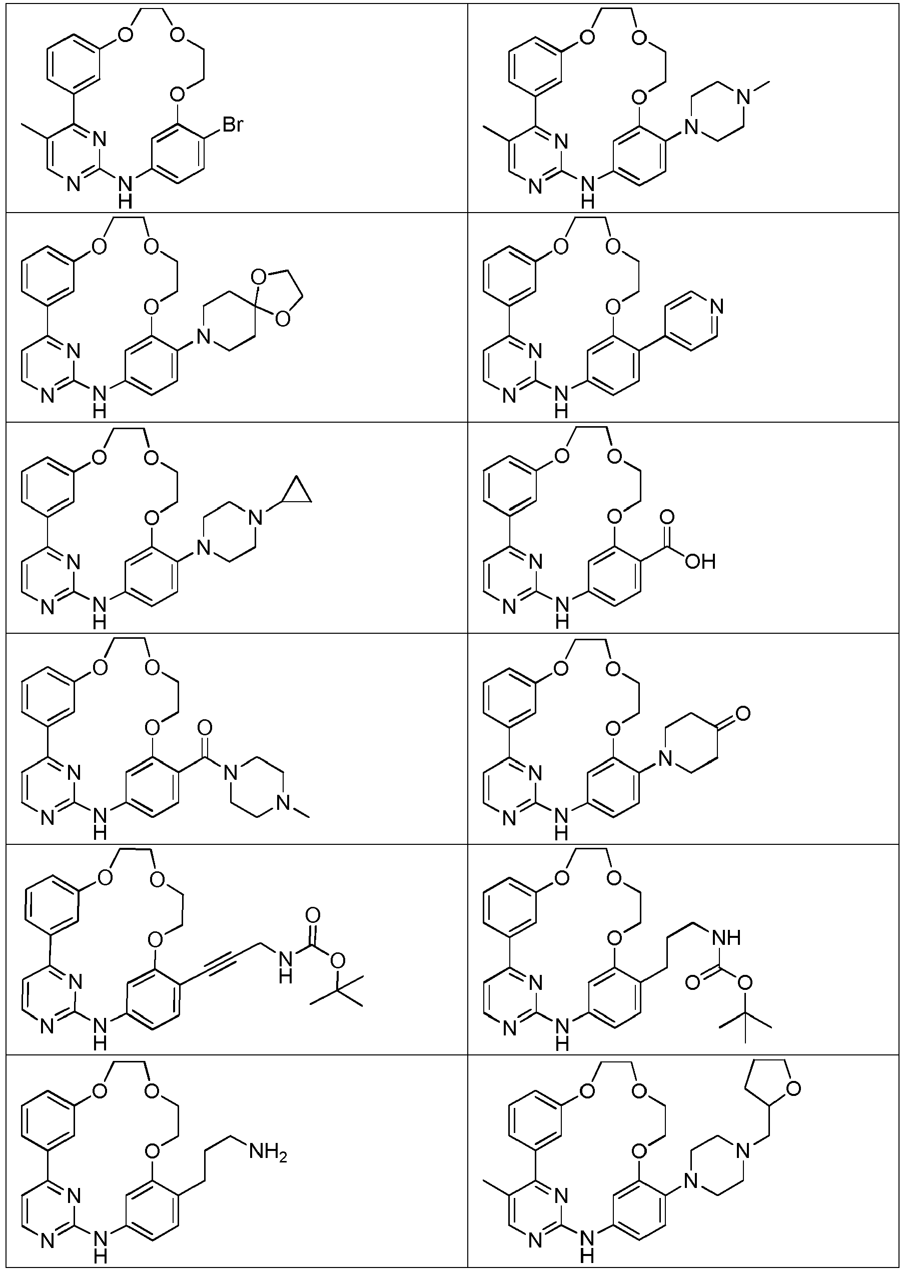 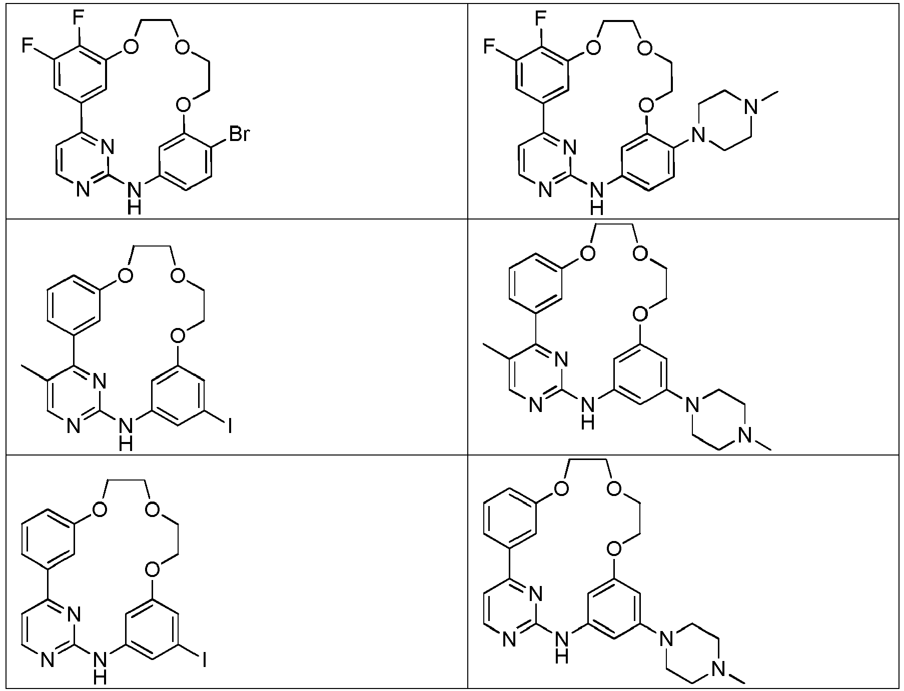 et leurs sels et/ou solvates pharmaceutiquement acceptables.

9. Composé selon l'une quelconque des revendications 1 à 8 pour son utilisation comme médicament.

10. Composé selon l'une quelconque des revendications 1 à 8 pour son utilisation dans le traitement du cancer.

11. Composition pharmaceutique comprenant au moins un composé selon l'une quelconque des revendications 1 à 8 et au moins un excipient pharmaceutiquement acceptable.

12. Procédé de préparation d'un composé de formule **(I)** selon l'une quelconque des revendicaitons 1 à 8 comprenant la réaction de couplage entre :
un composé de formule **(II)** suivante : 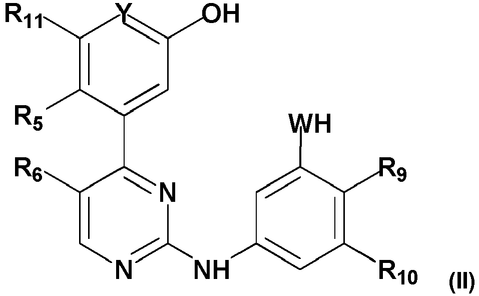 pour laquelle **W**, **Y**, **R₅**, **R₆**, **R₉**, **R₁₀** et **R₁₁** sont tels que définis à la revendication 1, et un composé de formule **(III)** suivante : 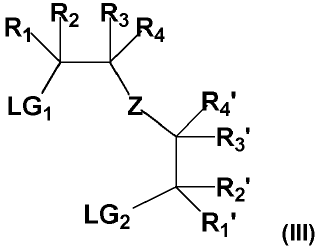 pour laquelle **Z, R₁, R₂, R₃, R₄, R₁', R₂', R₃'** et **R₄'** sont tels que définis à la revendication 1, et **LG₁** et **LG₂** représentent chacun, indépendamment l'un de l'autre, un groupe partant.

13. Procédé de préparation d'un composé selon l'une quelconque des revendications 1 à 8 comprenant la réaction de cyclisation d'un composé de formule **(VIa)** ou **(VIb)** suivante : 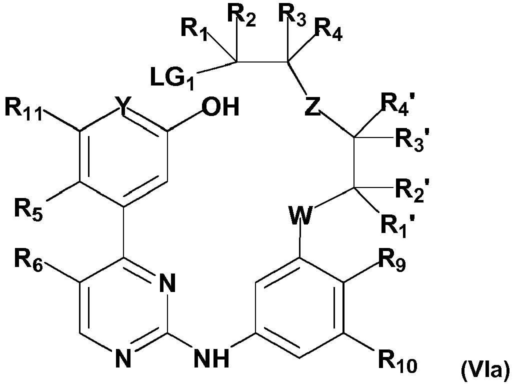 ou 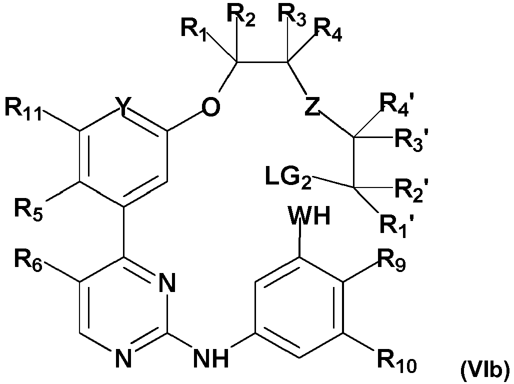 pour lesquelles **W, Y, Z, R₁** à **R₆, R₉** à **R₁₁** et **R₁'** à **R₄'** sont tels que définis à la revendication 1, et **LG₁** et **LG₂** représentent chacun, indépendamment l'un de l'autre, un groupe partant, tel qu'un atome d'halogène et en particulier un atome de brome.

14. Procédé de préparation d'un composé selon l'une quelconque des revendications 1 à 8, pour lequel **R₉** et/ou **R₁₀** représente un groupe (C₁-C₆)alcoxy éventuellement substitué, (C₁-C₆)thioalcoxy éventuellement substitué ou N**R₁₄R₁₅** ou un hétérocycle éventuellement substitué comprenant un hétéroatome directement lié au noyau phényle, comprenant le couplage entre un composé de formule **(IVa)** ou **(IVb)** suivante : 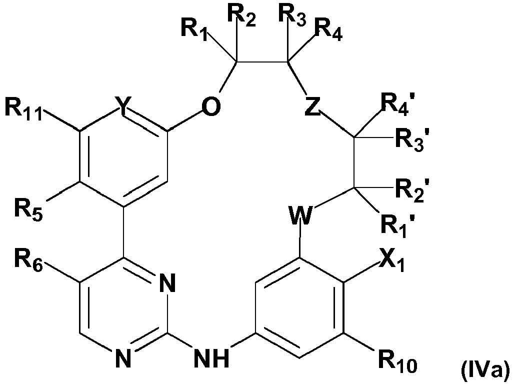 ou 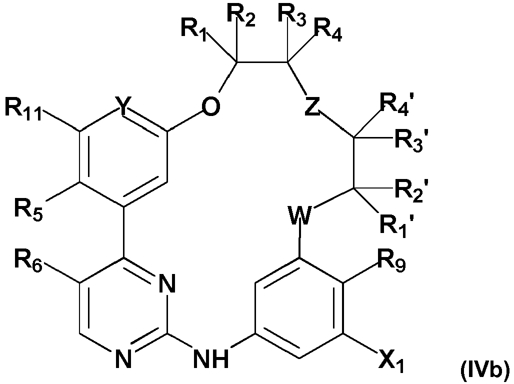 pour lesquelles **W**, **Y, Z, R₁** à **R₆, R₉** à **R₁₁** et **R₁'** à **R₄'** sont tels que définis à la revendication 1 et **X₁** représente un atome d'halogène tel que Br, Cl ou I,
et respectivement un composé de formule **R₉**H ou **R₁₀**H pour lesquels **R₉** et **R₁₀** sont tels que définis ci-dessus.

15. Procédé de préparation d'un composé selon l'une quelconque des revendications 1 à 8, pour lequel **R₉** et/ou **R₁₀** représente un groupe (C₁-C₆)alkyle éventuellement substitué, (C₂-C₆)alcényle éventuellement substitué ou (C₂-C₆)alcynyle éventuellement substitué, un carbocycle éventuellement substitué ou un hétérocycle éventuellement substitué lié au noyau phényle au moyen d'un atome de carbone, comprenant le couplage entre un composé de formule **(Va)** ou **(Vb)** suivante : 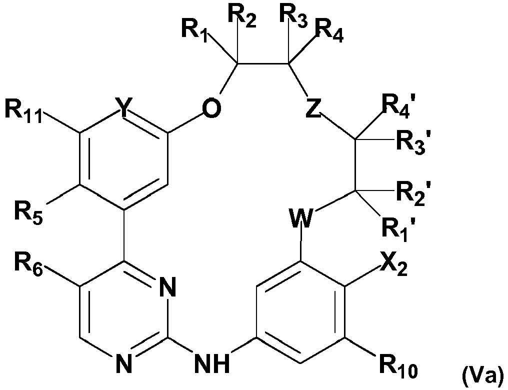 ou 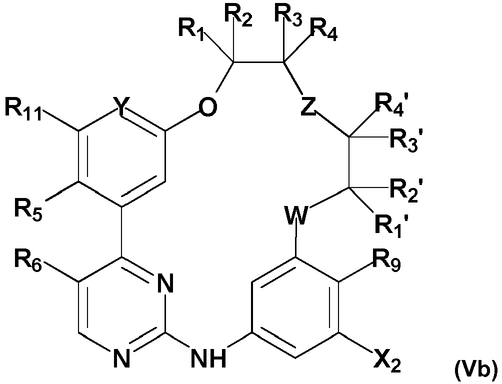 pour lesquelles **W**, **Y, Z, R₁** à **R₆, R₉** à **R₁₁** et **R₁'** à **R₄'** sont tels que définis à la revendication 1 et **X₂** représente Br, CI, I ou OTf(OSO₂CF₃),
et respectivement un composé de formule **R₉**-B**R₅₂R₅₃** ou **R₁₀-**B**R₅₂R₅₃** pour lesquels **R₉** et **R₁₀** sont tels que définis ci-dessus et **R₅₂** et **R₅₃** représentent, indépendamment l'un de l'autre, un groupe OH, (C₁-C₆)alkyle ou (C₁-C₆)alcoxy ou **R₅₂** et **R₅₃** forment ensemble une chaîne-**X₃**- ou -O-**X₃**-O- pour laquelle **X₃** représente un groupe divalent hydrocarboné comprenant 2 à 15, notamment 2 à 10, atomes de carbone.

16. Procédé de préparation d'un composé selon la revendication 7, pour lequel **R₉** et/ou **R₁₀** représente -**Z₁**-(CH₂)**ₘ-R₄₉** avec **Z₁** représentant CH₂-CH₂, CH=CH ou C=C, comprenant les étapes suivantes :
(1) couplage de Sonogashira entre un composé de formule **(Va)** ou **(Vb)** tel que défini à la revendication 15,
et un composé de formule HC≡C-(CH₂)**ₘ-R₄₉** pour lequel **m** et **R₄₉** sont tels que définis à la revendication 7,
pour donner un composé de formule **(I)** pour lequel **R₉** ou **R₁₀** représente -C≡C-(CH₂)**ₘ-R₄₉,** et
(2) éventuellement réduction de la fonction alcyne du composé de formule **(I)** obtenu à l'étape précédente pour donner un composé de formule **(I)** pour lequel **R₉** ou **R₁₀** représente -CH=CH-(CH₂)**ₘ-R₄₉** ou -(CH₂)_{**m**+2}**-R₄₉.**

17. Procédé de préparation d'un composé selon l'une quelconque des revendications 1 à 8, pour lequel **R₉** et/ou **R₁₀** représente un groupe CO₂**R₅₄** ou CON**R₅₅R₅₆,** qui comprend au moins l'une des étapes suivantes :
(a) pour obtenir un composé de formule **(I)** pour lequel **R₉** et/ou **R₁₀** représente un groupe CO₂H, la réaction d'un composé de formule **(I)** pour lequel **R₉** et/ou **R₁₀** représente un atome d'halogène avec du CO₂ ;
(b) pour obtenir un composé de formule **(I)** pour lequel **R₉** et/ou **R₁₀** représente un groupe CO₂**R₅₄** avec **R₅₄** ≠ H, la réaction de substitution d'un composé de formule **(I)** pour lequel **R₉** et/ou **R₁₀** représente un groupe CO₂H, éventuellement obtenu selon l'étape (a), éventuellement sous une forme activée, avec un alcool de formule **R₅₄**OH ;
(c) pour obtenir un composé de formule **(I)** pour lequel **R₉** et/ou **R₁₀** représente un groupe CON**R₅₅R₅₆,** la réaction de substitution d'un composé de formule **(I)** pour lequel **R₉** et/ou **R₁₀** représente un groupe CO₂H, éventuellement obtenu selon l'étape (a), éventuellement sous une forme activée, avec une amine de formule HN**R₅₅R₅₆.**

## Patentansprüche

1. Verbindung der folgenden allgemeinen Formel **(I)**: 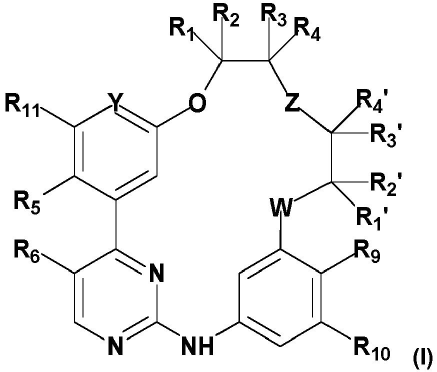 oder ein pharmazeutisch annehmbares Salz und/oder Solvat davon,
wobei:
- **W** ein Sauerstoff- oder Schwefelatom darstellt,
- **Y** ein Stickstoffatom oder eine Gruppe C**Ry** darstellt, mit **Ry** darstellend ein Wasserstoffatom, ein Halogenatom, eine Gruppe (C₁-C₆)Alkyl, (C₁-C₆)Halogenoalkyl, (C₁-C₆)Alcoxy, (C₁-C₆)Halogenoalcoxy, OH, CN, NO₂, N**R₁₂R₁₃,** CO₂H oder CO₂((C₁-C₆)alkyl),
- **Z** eine Gruppe (C**R_{Q1}R_{Q2}**)ₙ**Q**(C**R_{Q3}R_{Q4}**)ₘ darstellt, mit **n** und **m** unabhängig voneinander darstellend eine ganze Zahl im Bereich zwischen 0 und 3,
- **Q** O, S, S(O) oder S(O)₂ darstellt,
- **R_{Q1}**, **R_{Q2}**, **R_{Q3}** und **R_{Q4}** unabhängig voneinander ein Wasserstoffatom oder eine Gruppe (C₁-C₆)Alkyl darstellen,
- **R₁**, **R₂**, **R₁'** und **R₂'** unabhängig voneinander ein Wasserstoffatom oder eine Gruppe (C₁-C₆)Alkyl darstellen,
- **R₃**, **R₄, R₃'** und **R₄'** unabhängig voneinander ein Wasserstoffatom, eine Gruppe (C₁-C₆)Alkyl oder OH darstellen oder **R₃** und **R₄** und/oder **R₃'** und **R₄'** zusammen, mit dem Kohlenstoffatom, das sie trägt, einen monocyclischen Carbocyclus oder Heterocyclus, eventuell substituiert, bilden,
- **R₅** und **R₆,** unabhängig voneinander, ein Wasserstoffatom, ein Halogenatom, eine Gruppe (C₁-C₆)Alkyl, (C₁-C₆)Halogenoalkyl, (C₁-C₆)Alcoxy, (C₁-C₆)Halogenoalcoxy, (C₁-C₆)Thioalcoxy, (C₁-C₆)Halogenothioalcoxy, OH, SH, CN, NO₂ oder N**R₇R₈** darstellen,
- **R₉** und **R₁₀** unabhängig voneinander ein Wasserstoffatom, ein Halogenatom, eine Gruppe (C₁-C₆)Alkyl, eventuell substituiert, (C₂-C₆)Alcenyl, eventuell substituiert, (C₂-C₆)Alcynyl, eventuell substituiert, (C₁-C₆)Alcoxy, eventuell substituiert, (C₁-C₆)Thioalcoxy, eventuell substituiert, CN, NO₂, OH, SH, N**R₁₄R₁₅**, CO₂**R₅₄**, CON**R₅₅R₅₆**, einen Carbocyclus, eventuell substituiert, oder einen Heterocyclus, eventuell substituiert, darstellen,
- **R₁₁** ein Wasserstoffatom, ein Halogenatom, oder eine Gruppe (C₁-C₆)Alkyl, (C₁-C₆)Halogenoalkyl, (C₁-C₆)Alcoxy oder (C₁-C₆)Halogenoalcoxy darstellt, und
- **R₇, R₈**, **R₁₂**, **R₁₃, R₁₄, R₁₅, R₅₄, R₅₅** und **R₅₆** unabhängig voneinander ein Wasserstoffatom oder eine Gruppe (C₁-C₆)Alkyl, eventuell substituiert, (C₂-C₆)Alcenyl, eventuell substituiert, oder (C₂-C₆)Alcynyl, eventuell substituiert, darstellen, oder **R₇** und **R₈**, **R₁₂** und **R₁₃, R₁₄** und **R₁₅** und/oder **R₅₅** und **R₅₆**, unabhängig voneinander, mit dem Stickstoffatom, dass sie tragen, einen stickstoffhaltigen Heterocyclus, eventuell substituiert, bilden.

2. Verbindung nach Anspruch 1, **dadurch gekennzeichnet, dass Z** ein Sauerstoffatom darstellt und **R₁, R₂**, **R₃**, **R₄, R₁', R₂', R₃'** und **R₄'** jeweils ein Wasserstoffatom darstellen.

3. Verbindung nach einem der Ansprüche 1 und 2, **dadurch gekennzeichnet, dass Y** eine Gruppe C**Ry** darstellt, mit **Ry** darstellend ein Wasserstoffatom oder ein Halogenatom wie F.

4. Verbindung nach einem der Ansprüche 1 bis 3, **dadurch gekennzeichnet, dass R₁₁** ein Wasserstoffatom oder ein Halogenatom darstellt, wie F, darstellt.

5. Verbindung nach einem der Ansprüche 1 bis 4, **dadurch gekennzeichnet, dass R₅** und **R₆** unabhängig voneinander ein Wasserstoffatom oder eine Gruppe (C₁-C₆)Alkyl darstellen.

6. Verbindung nach einem der Ansprüche 1 bis 5, **dadurch gekennzeichnet, dass R₉** und **R₁₀** unabhängig voneinander ein Wasserstoffatom, ein Halogenatom, CO₂**R₅₄**, CON**R₅₅R₅₆**, oder eine Gruppe (C₁-C₆)Alkyl, (C₂-C₆)Alcenyl, (C₂-C₆)Alcynyl, (C₁-C₆)Alcoxy, (C₁-C₆)Thioalcoxy, (C₁-C₆)Alkylamino, Di((C₁-C₆)alkyl)amino-oder Heterocyclus darstellen, wobei die Gruppe eventuell substituiert ist durch einen oder mehrere Substituenten, ausgewählt aus:
- einem Halogenatom,
- einer Gruppe (C₁-C₆)Alkyl, eventuell substituiert durch eine oder mehrere Gruppen, ausgewählt aus einem Halogenatom, O**R₁₆,** S**R₁₇,** N**R₁₈R₁₉,** einem Carbocyclus und einem Heterocyclus,
- den Gruppen Oxo (=O), CN, NO₂, O**R₂₀,** S**R₂₁**, N**R₂₂R₂₃,** C(O)**R₂₄**, CO₂**R₂₅,** OC(O)**R₂₆**S(O)**R₂₇**, SO₂**R₂₈,** N**R₂₉**C(O)**R₃₀,** C(O)N**R₃₁R₃₂,** N**R₃₃**CO₂**R₃₄,** OC(O)N**R₃₅R₃₆,** N**R₃₇**CON**R₃₈R₃₉** und OCO₂**R₄₀,**
- einem Carbocyclus, eventuell substituiert durch eine oder mehrere Gruppen, ausgewählt aus einem Halogenatom, einer Gruppe (C₁-C₆)Alkyl, Oxo (=O), O**R₄₁,** S**R₄₂** und N**R₄₃R₄₄,**
- einem Heterocyclus, eventuell substituiert durch eine oder mehrere Gruppen, ausgewählt aus einem Halogenatom, einer Gruppe (C₁-C₆)Alkyl, Oxo(=O), O**R₄₅,** S**R₄₆** und N**R₄₇R₄₈**, und
- einer Gruppe -O(CH₂)ₙO-, mit n darstellend eine ganze Zahl im Bereich zwischen 1 und 5, insbesondere zwischen 2 und 3,
wobei:
▪ **R₁₆** bis **R₄₈** unabhängig voneinander ein Wasserstoffatom, eine Gruppe (C₁-C₆)Alkyl, Aryl, Aryl-(C₁-C₆)alkyl, Heterocyclus oder Heterocyclus-(C₁-C₆)Alkyl darstellen,
wobei der Arylkern dieser Gruppen eventuell substituiert ist durch eine oder mehrere Gruppen, ausgewählt aus einem Halogenatom, und einer Gruppe (C₁-C₆)Alkyl, und
wobei der Heterocycluskern dieser Gruppen eventuell substituiert ist durch eine oder mehrere Gruppen, ausgewählt aus einem Halogenatom, einer Gruppe (C₁-C₆)Alkyl und Oxo (=O), oder
▪ **R₂₂** und **R₂₃, R₃₁** und **R₃₂, R₃₅** und **R₃₆, R₃₈** und **R₃₉, R₄₃** und **R₄₄,** und/oder **R₄₇** und **R₄₈** zusammen mit dem Stickstoffatom, der sie trägt, einen stickstoffhaltigen Heterocyclus bilden, eventuell substituiert durch eine oder mehrere Gruppen, ausgewählt aus einem Halogenatom, einer Gruppe (C₁-C₆)Alkyl und Oxo (=O).

7. Verbindung nach einem der Ansprüche 1 bis 6, **dadurch gekennzeichnet, dass R₉** und **R₁₀** unabhängig voneinander Folgendes darstellen:
- ein Wasserstoff- oder Halogenatom,
- eine Gruppe CO₂**R₅₄** mit **R₅₄** darstellend ein Wasserstoffatom oder eine Gruppe (C₁-C₆)Alkyl, insbesondere ein Wasserstoffatom,
- eine Gruppe CON**R₅₅R₅₆**, mit **R₅₅** und **R₅₆** bildend mit dem Stickstoffatom, das sie tragen, einen stickstoffhaltigen Heterocyclus mit 5, 6 oder 7 Kettengliedern, umfassend 1 oder 2 Heteroatome, ausgewählt aus N und O, darunter mindestens ein Stickstoffatom, vorzugsweise gesättigt, eventuell substituiert durch einen oder mehrere Substituenten, ausgewählt aus einem Halogenatom, (C₁-C₆)Alkyl, Oxo (=O), O**R₂₀,** N**R₂₂R₂₃,** CO₂**R₂₅,**C(O)N**R₃₁R₃₂,** N**R₃₃**CO₂**R₃₄,** OC(O)N**R₃₅R₃₆,** N**R₃₇**CON**R₃₈R₃₉** und OCO₂**R₄₀;** insbesondere ausgewählt aus einem Halogenatom, (C₁-C₆)Akyl, Oxo(=O), O**R₂₀,** N**R₂₂R₂₃,** CO₂**R₂₅** und C(O)N**R₃₁R₃₂,**
- eine Gruppe -**Z₁**-(CH₂)**ₘ**-**R₄₉**, wobei **Z₁** eine einfache Bindung darstellt, CH₂-CH₂, CH=CH, C=C, O, S oder N**R₅₀** insbesondere eine einfache Bindung, CH₂-CH₂, C=C, O oder N**R₅₀; m** eine ganze Zahl im Bereich zwischen 1 und 6 darstellt, insbesondere im Bereich zwischen 1 und 4; **R₅₀** ein Wasserstoffatom oder eine Gruppe (C₁-C₆)Alkyl darstellt; und **R₄₉** ein Halogenatom, O**R₂₀,** N**R₂₂R₂₃,** C(O)**R₂₄**, CO₂**R₂₅,** OC(O)**R₂₆,** N**R₂₉**C(O)**R₃₀,** C(O)N**R₃₁R₃₂,** N**R₃₃**CO₂**R₃₄,** OC(O)N**R₃₅R₃₆,** N**R₃₇**CON**R₃₈R₃₉** oder OCO₂**R₄₀** darstellt; besonders O**R₂₀,** N**R₂₂R₂₃,** CO₂**R₂₅,** C(O)N**R₃₁R₃₂,** N**R₃₃**CO₂**R₃₄,** OC(O)N**R₃₅R₃₆,** N**R₃₇**CON**R₃₈R₃₉** oder OCO₂**R₄₀;** insbesondere N**R₂₂R₂₃,** N**R₃₃**CO₂**R₃₄** oder N**R₃₇**CON**R₃₈R₃₉**, oder
- einen Heterocyclus mit 5, 6 oder 7 Kettengliedern, insbesondere mit 6 oder 7 Kettengliedern, umfassend 1 oder 2 Heteroatome, ausgewählt aus N und O und umfassend vorteilhafterweise mindestens ein Stickstoffatom, vorzugsweise gesättigt oder aromatisch, eventuell substituiert durch einen oder mehrere Substituenten, ausgewählt aus:
• einem Halogenatom,
• einer Gruppe (C₁-C₆)Alkyl, eventuell substituiert durch eine oder mehrere Gruppen, ausgewählt aus einem Halogenatom, O**R₁₆,** N**R₁₈R₁₉,** einem monocyclischen Carbocyclus mit C₃ bis C₆ und einem monocyclischen Heterocyclus mit 3 bis 6 Kettengliedern, umfassend 1 oder 2 Heteroatome, ausgewählt aus N und O,
• den Gruppen Oxo(=O), O**R₂₀,** N**R₂₂R₂₃,** C(O)**R₂₄**, CO₂**R₂₅,** OC(O)**R₂₆,** N**R₂₉**C(O)**R₃₀,** C(O)N**R₃₁R₃₂,** N**R₃₃**CO₂**R₃₄,** OC(O)N**R₃₅R₃₆,** N**R₃₇**CON**R₃₈R₃₉** oder OCO₂**R₄₀;** und ganz besonders den Gruppen Oxo (=O), O**R₂₀,** N**R₂₂R₂₃,** CO₂**R₂₅,**C(O)N**R₃₁R₃₂,** N**R₃₃**CO₂**R₃₄,** OC(O)N**R₃₅R₃₆,** N**R₃₇**CON**R₃₈R₃₉** oder OCO₂**R₄₀,**
• einem Carbocyclus mit C₃ bis C₆, eventuell substituiert durch eine oder mehrere Gruppen, ausgewählt aus einem Halogenatom, einer Gruppe (C₁-C6)Alkyl, Oxo (=O)-, O**R₄₁** und N**R₄₃R₄₄,**
• einem Heterocyclus mit 3 bis 6 Kettengliedern, umfassend 1 oder 2 Heteroatome, ausgewählt aus N und O, insbesondere gesättigt oder ungesättigt, vorzugsweise gesättigt, eventuell substituiert durch eine oder mehrere Gruppen, ausgewählt aus einem Halogenatom, einer Gruppe (C₁-C6)Alkyl, Oxo(=O), O**R₄₅** und N**R₄₇R₄₈** und
• einer Gruppe -O(CH₂)ₙO-, mit n darstellend eine ganze Zahl gleich 2 oder 3,
wobei:
▪ **R₁₆, R₁₈** bis **R₂₀, R₂₂** bis **R₂₆, R₂₉** bis **R₄₀, R₄₅** und **R₄₇** bis **R₄₈** unabhängig voneinander ein Wasserstoffatom, eine Gruppe (C₁-C₆)Alkyl, Aryl oder Aryl-(C₁-C₆)alkyl darstellen, insbesondere ein Wasserstoffatom oder eine Gruppe (C₁-C₆)Alkyl,
wobei der Arylkern dieser Gruppen eine Phenylgruppe ist, und eventuell substituiert ist durch eine oder mehrere Gruppen, ausgewählt aus einem Halogenatom, und einer Gruppe (C₁-C₆)Alkyl, oder
▪ **R₂₂** und **R₂₃, R₃₁** und **R₃₂, R₃₅** und **R₃₆, R₃₈** und **R₃₉, R₄₃** und **R₄₄,** und/oder **R₄₇** und **R₄₈** zusammen mit dem Stickstoffatom, das sie trägt, einen stickstoffhaltigen Heterocyclus mit 5 oder 6 Kettengliedern bilden, vorzugsweise gesättigt, umfassend eventuell 1 Heteroatom, zusätzlich zum Stickstoffatom, ausgewählt aus N und O, wie eine Piperazin-, Piperidin-, Morpholin- oder Pyrrolidincyclus, wobei der Heterocyclus eventuell substituiert ist durch eine oder mehrere Gruppen, ausgewählt aus einem Halogenatom, einer Gruppe (C₁-C₆)Alkyl und Oxo (=O).

8. Verbindung nach Anspruch 1, **dadurch gekennzeichnet, dass** sie ausgewählt ist ausfolgenden Verbindungen: 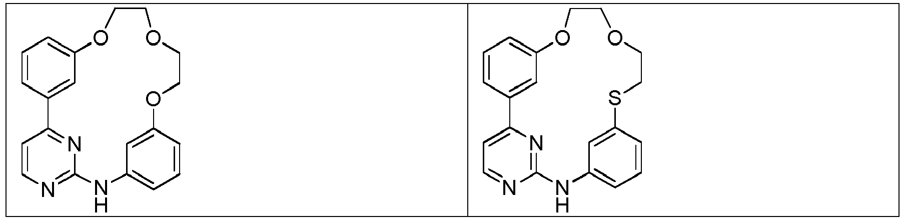 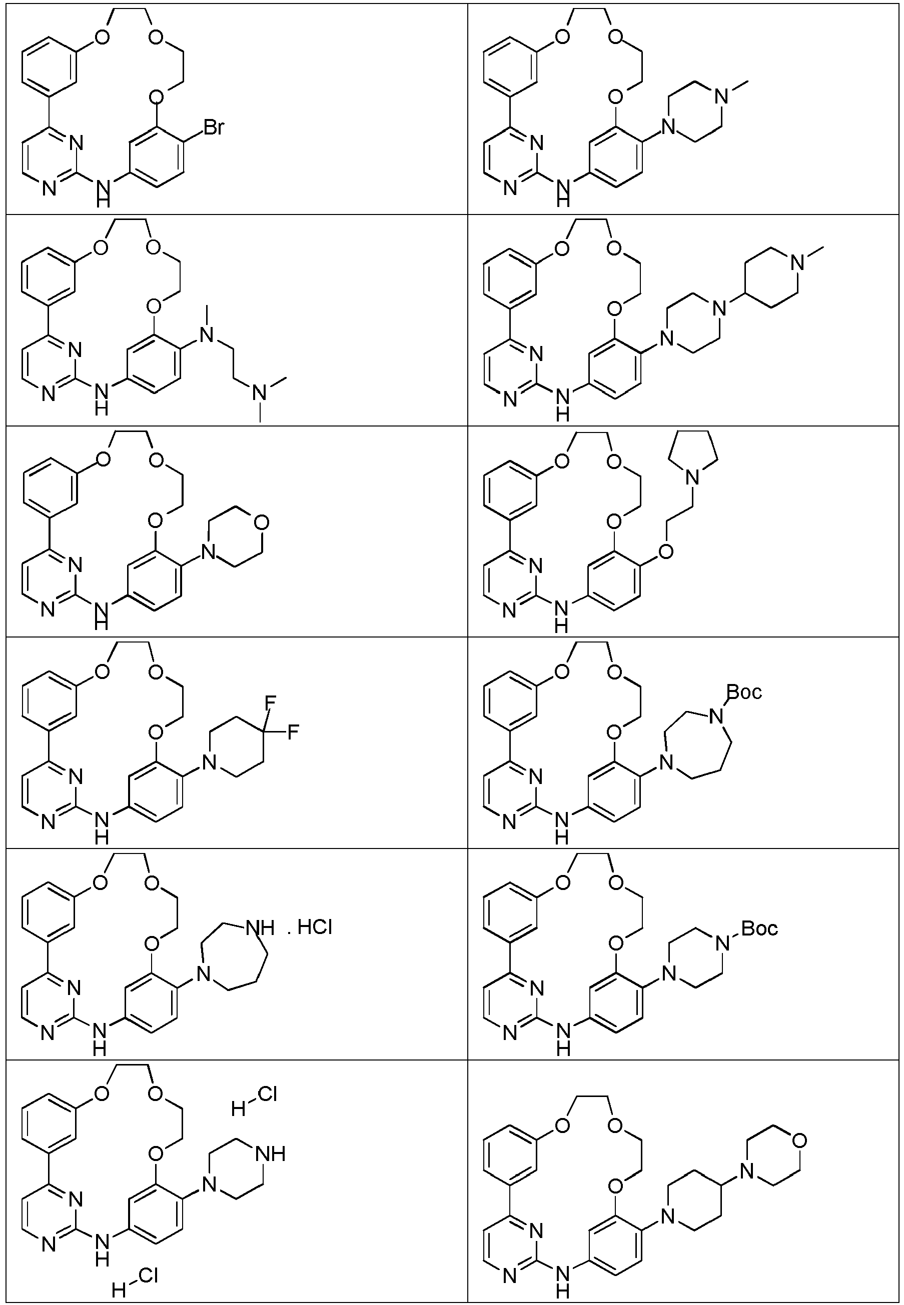 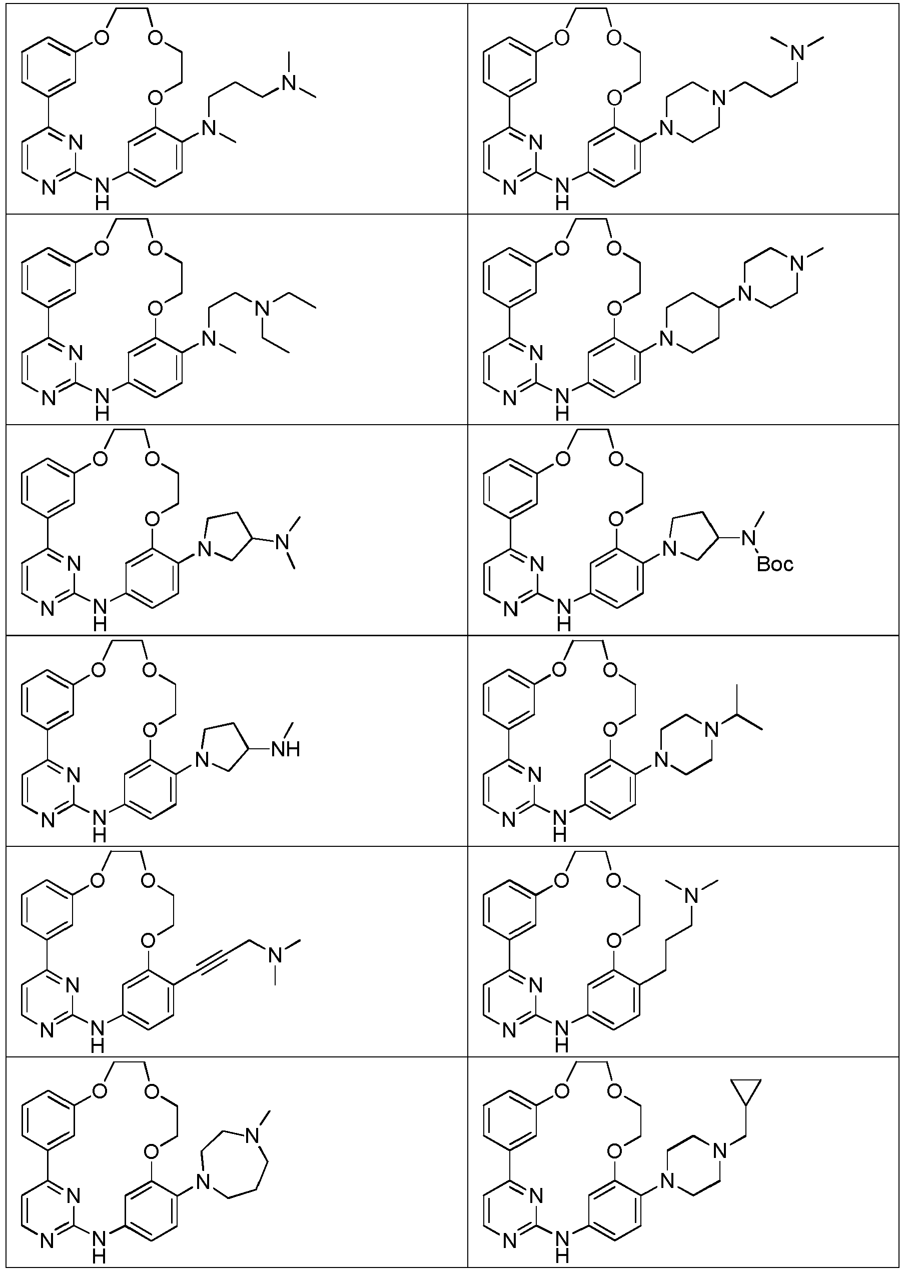 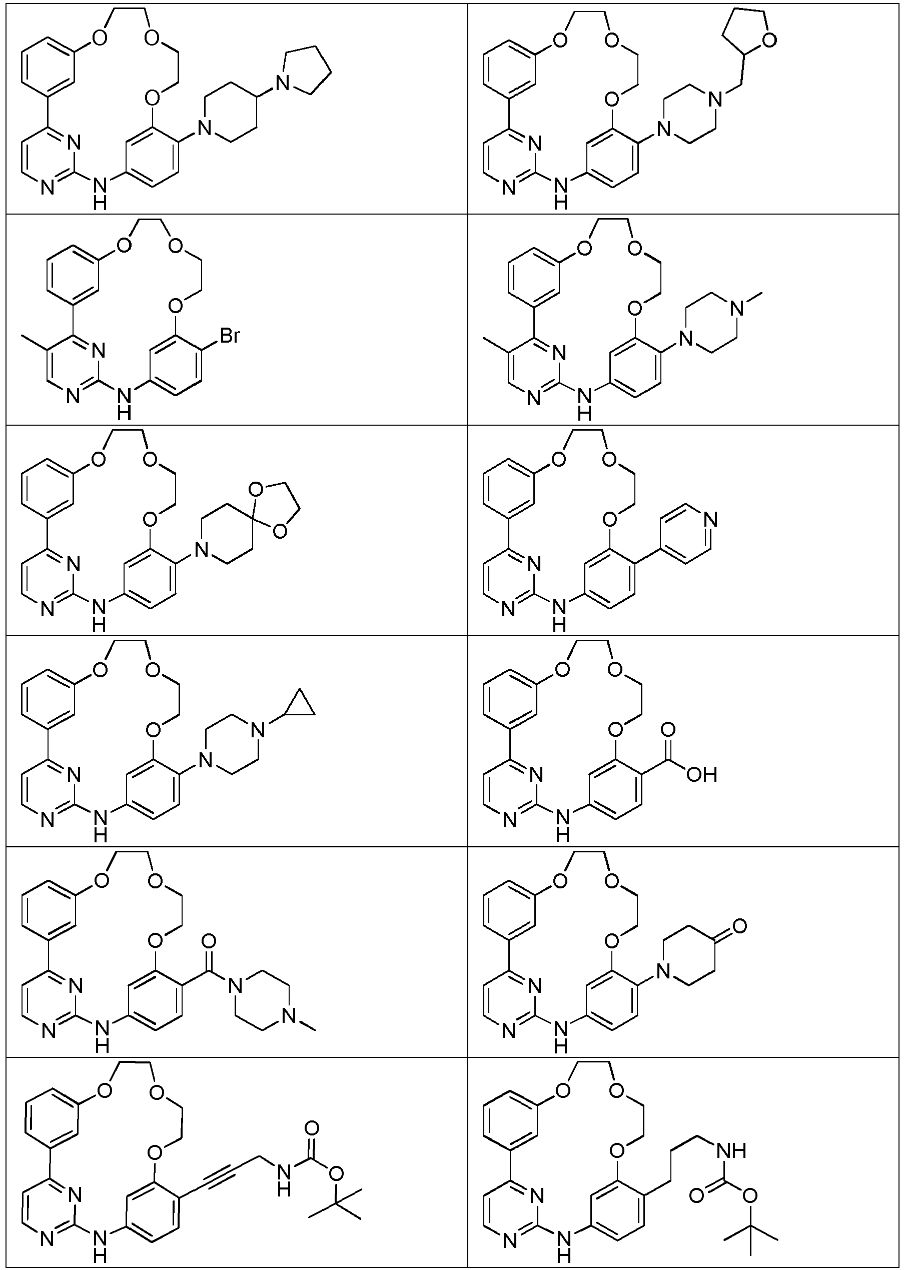 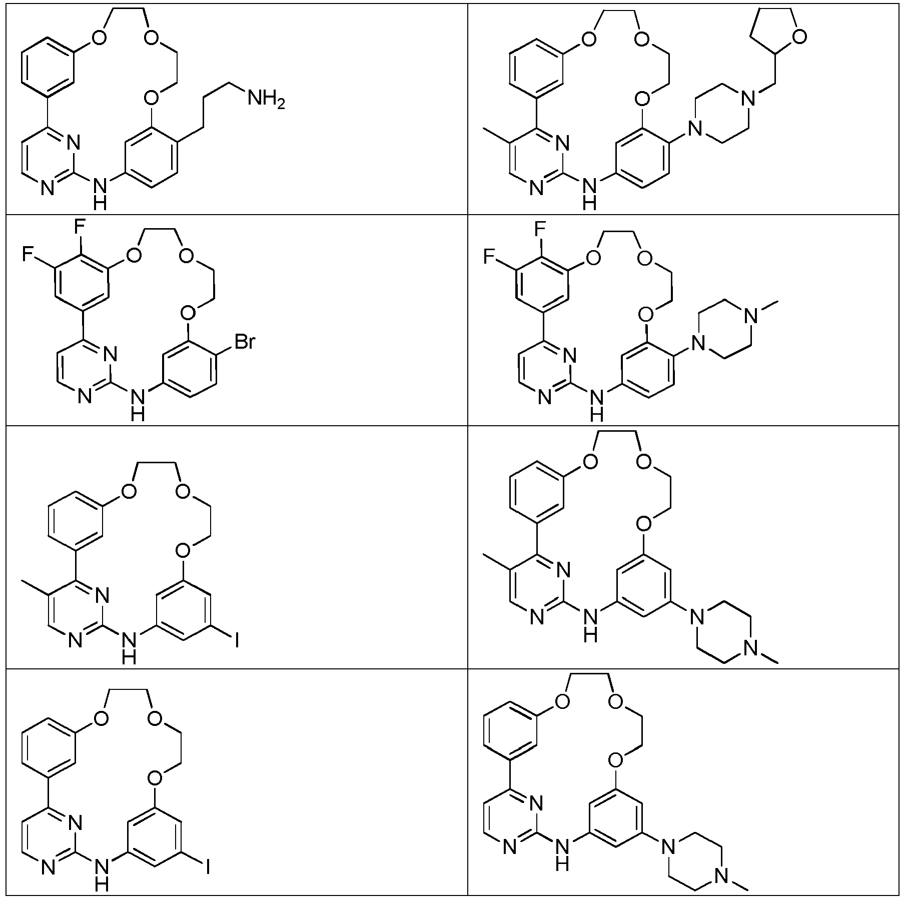 und ihren pharmazeutisch annehmbaren Salzen und/oder Solvaten.

9. Verbindung nach einem der Ansprüche 1 bis 8 für ihre Verwendung als Arzneimittel.

10. Verbindung nach einem der Ansprüche 1 bis 8 für ihre Verwendung bei der Behandlung von Krebs.

11. Pharmazeutische Zusammensetzung, umfassend mindestens eine Verbindung nach einem der Ansprüche 1 bis 8 und mindestens einen pharmazeutisch annehmbaren Hilfsstoff.

12. Verfahren zur Herstellung einer Verbindung der Formel **(I)** nach einem der Ansprüche 1 bis 8, umfassend die Kopplungsreaktion zwischen:
einer Verbindung der folgenden Formel **(II):** 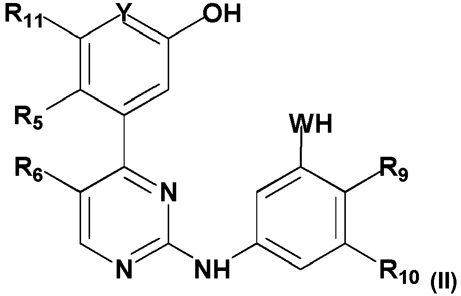 für die **W, Y, R₅, R₆, R₉, R₁₀** und **R₁₁** wie in Anspruch 1 definiert sind, und einer Verbindung der folgenden Formel **(III):** 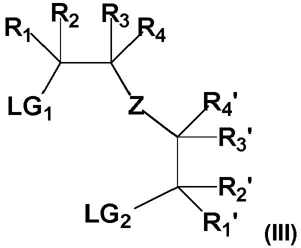 für die **Z, R₁, R₂**, **R₃**, **R₄, R₁', R₂', R₃'** und **R₄'** wie in Anspruch 1 definiert sind, und **LG₁** und **LG₂** jeweils unabhängig voneinander eine Abgangsgruppe darstellen.

13. Verfahren zur Herstellung einer Verbindung nach einem der Ansprüche 1 bis 8, umfassend eine Cyclisierungsreaktion einer Verbindung mit folgenden Formel **(VIa)** oder **(VIb):** 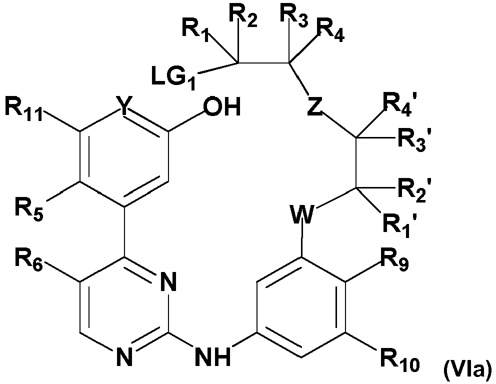 oder 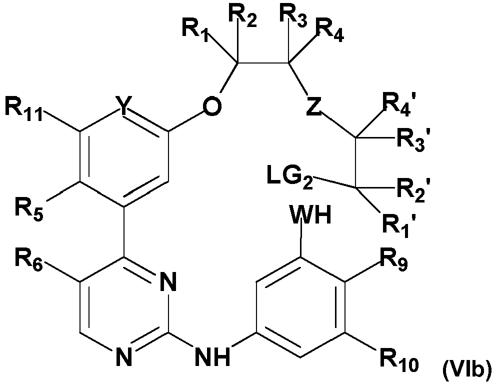 für die **W, Y, Z, R₁** bis **R₆, R₉** bis **R₁₁** und **R₁'** bis **R₄'** wie in Anspruch 1 definiert sind, und **LG₁** und **LG₂** jeweils unabhängig voneinander eine Abgangsgruppe darstellen, wie ein Halogenatom und insbesondere ein Bromatom.

14. Verfahren zur Herstellung einer Verbindung nach einem der Ansprüche 1 bis 8, für das **R₉** und/oder **R₁₀** eine Gruppe (C₁-C₆)Alcoxy darstellt, eventuell substituiert, (C₁-C₆)Thioalcoxy, eventuell substituiert, oder N**R₁₄R₁₅** oder einen Heterocyclus, eventuell substituiert, umfassend ein Heteroatom, das direkt mit dem Phenylkern verbunden ist, umfassend die Kopplung zwischen einer Verbindung der folgenden Formel **(IVa)** oder **(IVb)**: 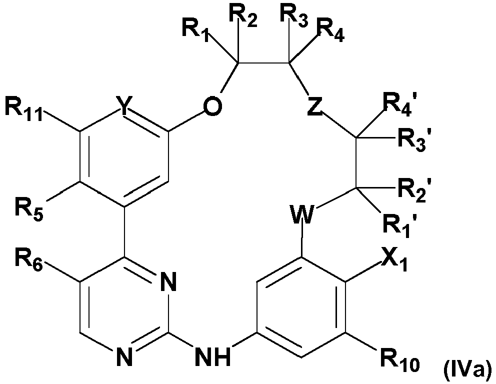 oder 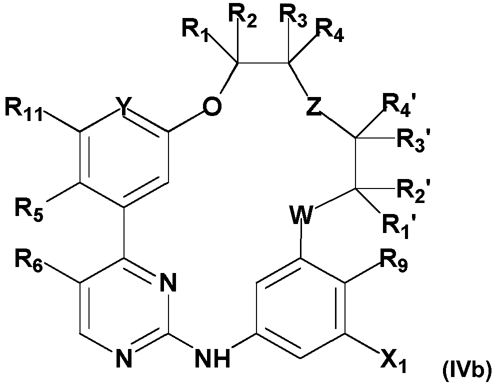 für die **W, Y, Z, R₁** bis **R₆, R₉** bis **R₁₁** und **R₁'** bis **R₄'** wie in Anspruch 1 definiert sind und **X₁** ein Halogenatom darstellt, wie Br, Cl oder I
und jeweils eine Verbindung der Formel **R₉H** oder **R₁₀**H, für die **R₉** und **R₁₀** wie oben definiert sind.

15. Verfahren zur Herstellung einer Verbindung nach einem der Ansprüche 1 bis 8, für die **R₉** und/oder **R₁₀** eine Gruppe (C₁-C₆)Alkyl, eventuell substituiert, (C₂-C₆)Alcenyl, eventuell substituiert, (C₂-C₆)Alcynyl, eventuell substituiert, einen Carbocyclus, eventuell substituiert, oder einen Heterocyclus, eventuell substituiert, darstellt, verbunden mit dem Phenylkern mit Hilfe eines Kohlenstoffatoms, umfassend die Kopplung zwischen einer Verbindung der folgenden Formel **(Va)** oder **(Vb):** 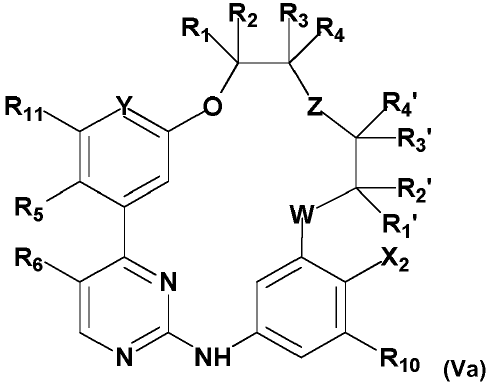 oder 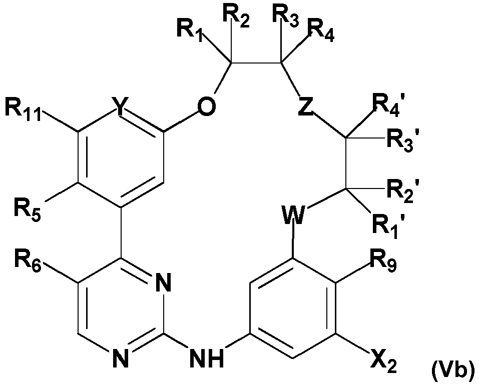 für die **W, Y, Z, R₁** bis **R₆, R₉** bis **R₁₁** und **R₁'** bis **R₄'** wie in Anspruch 1 definiert sind und **X₂** Br, Cl, I oder OTf(OSO₂CF₃) darstellt,
und jeweils eine Verbindung der Formel **R₉-**B**R₅₂R₅₃** oder **R₁₀**-B**R₅₂R₅₃,** für die **R₉** und **R₁₀** wie oben definiert sind und **R₅₂** und **R₅₃** unabhängig voneinander eine Gruppe OH, (C₁-C₆)Alkyl oder (C₁-C₆)Alcoxy darstellen oder **R₅₂** und **R₅₃** zusammen eine Kette -**X₃**- oder -O-**X₃**-O- bilden, für die **X₃** eine divalente Kohlenwasserstoffgruppe darstellt, umfassend 2 bis 15, insbesondere 2 bis 10 Kohlenstoffatome.

16. Verfahren zur Herstellung einer Verbindung nach Anspruch 7, für das **R₉** und/oder **R₁₀**-**Z₁**-(CH₂)**ₘ**-**R₄₉** darstellt, mit **Z₁** darstellend CH₂-CH₂, CH=CH oder C=C, umfassend die folgenden Schritte:
(1) Sonogashira-Koppeln zwischen einer Verbindung der Formel **(Va)** oder **(Vb),** wie in Anspruch 15 definiert,
und eine Verbindung der Formel HC=C-(CH₂)ₘ-**R₄₉,** für die **m** und **R₄₉** wie in Anspruch 7 definiert sind,
um eine Verbindung der Formel **(I)** zu ergeben, für die **R₉** oder **R₁₀**-C≡C-(CH₂)**ₘ**-**R₄₉** darstellt, und
(2) eventuell Reduzieren der Alcynfunktion der Verbindung der Formel **(I),** erhalten im vorhergehenden Schritt, um eine Verbindung der Formel **(I)** zu ergeben, für die **R₉** oder **R₁₀**-CH=CH-(CH₂)**ₘ**-**R₄₉** oder -(CH₂)_{**m**+2}-**R₄₉** darstellt.

17. Verfahren zur Herstellung einer Verbindung nach einem der Ansprüche 1 bis 8, für die **R₉** und/oder **R₁₀** eine Gruppe CO₂**R₅₄** oder CON**R₅₅R₅₆** darstellt, die mindestens einen der folgenden Schritte umfasst:
(a) um eine Verbindung der folgenden Formel **(I)** zu erhalten, für die **R₉** und/oder **R₁₀** eine Gruppe CO₂H darstellt, die Reaktion einer Verbindung der Formel **(I),** für die **R₉** und/oder **R₁₀** ein Halogenatom mit CO₂ darstellt;
(b) um eine Verbindung der folgenden Formel **(I)** zu erhalten, für die **R₉** und/oder **R₁₀** eine Gruppe CO₂**R₅₄** mit **R₅₄** ≠ H darstellt, die Substitutionsreaktion einer Verbindung der Formel **(I),** für die **R₉** und/oder **R₁₀** eine Gruppe CO₂H darstellt, eventuell erhalten gemäß Schritt (a), eventuell in einer aktivierten Form, mit einem Alkohol der Formel **R₅₄**OH;
(c) um eine Verbindung der Formel **(I)** zu erhalten, für die **R₉** und/oder **R₁₀** eine Gruppe CON**R₅₅R₅₆** darstellt, die Substitutionsreaktion einer Verbindung der Formel **(I),** für die **R₉** und/oder **R₁₀** eine Gruppe CO₂H darstellt, eventuell erhalten gemäß Schritt (a), eventuell in einer aktivierten Form, mit einem Amin der Formel HN**R₅₅R₅₆**.

## Claims

1. A compound of the following general formula **(I)**: 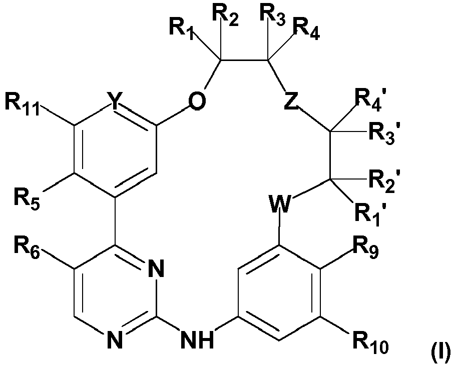 or a pharmaceutically acceptable salt and/or a solvate thereof,
wherein:
- **W** represents an oxygen or sulphur atom,
- **Y** represents a nitrogen atom or a C**Ry** group with **Ry** representing a hydrogen atom, a halogen atom, a (C₁-C₆)alkyl, (C₁-C₆)halogenoalkyl, (C₁-C₆)alkoxy, (C₁-C₆)halogenoalkoxy, OH, CN, NO₂, N**R₁₂R₁₃,** CO₂H or CO₂((C₁-C₆)alkyl) group,
- **Z** represents a (C**R_{Q1}R_{Q2}**)ₙ**Q**(C**R_{Q3}R_{Q4}**)ₘ group, with **n** and **m** representing, independently of one another, an integer between 0 and 3,
- **Q** represents O, S, S(O) or S(O)₂,
- **R_{Q1}, R_{Q2}, R_{Q3}** and **R_{Q4}** represent, independently of each other, a hydrogen atom or a (C₁-C₆)alkyl group,
- **R₁, R₂**, **R₁'** and **R₂'** represent, independently of each other, a hydrogen atom or a (C₁-C₆)alkyl group,
- **R₃**, **R₄, R₃'** and **R₄'** represent, independently of each other, a hydrogen atom, a (C₁-C₆)alkyl group or OH or **R₃** and **R₄** and/or **R₃'** and **R₄'** form together, with the carbon atom carrying them, an optionally substituted monocyclic carbocycle or heterocycle,
- **R₅** and **R₆** represent, independently of one another, a hydrogen atom, a halogen atom, a (C₁-C₆)alkyl, (C₁-C₆)halogenoalkyl, (C₁-C₆)alkoxy, (C₁-C₆)halogenoalkoxy, (C₁-C₆)thioalkoxy, (C₁-C₆)halogenothioalkoxy, OH, SH, CN, NO₂, or N**R₇R₈** group,
- **R₉** and **R₁₀** represent, independently of one another, a hydrogen atom, a halogen atom, an optionally substituted (C₁-C₆)alkyl, optionally substituted (C₂-C₆)alkenyl, optionally substituted (C₂-C₆)alkynyl, optionally substituted (C₁-C₆)alkoxy, optionally substituted (C₁-C₆)thioalkoxy, CN, NO₂, OH, SH, N**R₁₄R₁₅,** CO₂**R₅₄**,CON**R₅₅R₅₆**, an optionally substituted carbocycle or an optionally substituted heterocycle group,
- **R₁₁** represents a hydrogen atom, a halogen atom, or a (C₁-C₆)alkyl, (C₁-C₆)halogenoalkyl, (C₁-C₆)alkoxy or (C₁-C₆)halogenoalkoxy group, and
- **R₇, R₈, R₁₂, R₁₃, R₁₄, R₁₅, R₅₄, R₅₅** and **R₅₆** represent, independently of each other, a hydrogen atom or an optionally substituted (C₁-C₆)alkyl, optionally substituted (C₂-C6)alkenyl, or optionally substituted (C₂-C₆)alkynyl group, or **R₇** and **R₈, R₁₂** and **R₁₃, R₁₄** and **R₁₅** and/or **R₅₅** and **R₅₆**, independently of each other, form with the nitrogen atom carrying them an optionally substituted nitrogen-containing heterocycle.

2. The compound according to claim 1, **characterised in that Z** represents an oxygen atom and **R₁, R₂**, **R₃**, **R₄, R₁', R₂', R₃'** and **R₄'** each represent a hydrogen atom.

3. The compound according to any one of claims 1 and 2, **characterised in that Y** represents a C**Ry** group with **Ry** representing a hydrogen atom or a halogen atom such as F.

4. The compound according to any one of claims 1 to 3, **characterised in that R₁₁** represents a hydrogen atom or a halogen atom such as F.

5. The compound according to any one of claims 1 to 4, **characterised in that R₅** and **R₆** represent, independently of one another, a hydrogen atom or a (C₁-C₆)alkyl group.

6. The compound according to any one of claims 1 to 5, **characterised in that R₉** and **R₁₀** represent, independently of one another, a hydrogen atom, a halogen atom, CO₂**R₅₄**, CON**R₅₅R₅₆**, or a (C₁-C₆)alkyl, (C₂-C₆)alkenyl, (C₂-C₆)alkynyl, (C₁-C₆)alkoxy, (C₁-C₆)thioalkoxy, (C₁-C₆)alkyl-amino, di((C₁-C₆)alkyl)amino or heterocycle group, said group being optionally substituted with one or several substituents chosen from:
- a halogen atom,
- a (C₁-C₆)alkyl group optionally substituted with one or several groups chosen from a halogen atom, O**R₁₆,** S**R₁₇**, N**R₁₈R₁₉,** a carbocycle and a heterocycle,
- the oxo (=O), CN, NO₂, O**R₂₀,** S**R₂₁**, N**R₂₂R₂₃,** C(O)**R₂₄,** CO₂**R₂₅,**OC(O)**R₂₆,**S(O)**R₂₇,** SO₂**R₂₈,** N**R₂₉**C(O)**R₃₀,** C(O)N**R₃₁R₃₂,** N**R₃₃**CO₂**R₃₄,** OC(O)N**R₃₅R₃₆,** N**R₃₇**CON**R₃₈R₃₉** and OCO₂**R₄₀** groups,
- a carbocycle optionally substituted with one or several groups chosen from a halogen atom, a (C₁-C₆)alkyl, oxo (=O), O**R₄₁,** S**R₄₂** and N**R₄₃R₄₄** group,
- a heterocycle optionally substituted with one or several groups chosen from a halogen atom, a (C₁-C₆)alkyl, oxo (=O), O**R₄₅,** S**R₄₆** and N**R₄₇R₄₈** group, and
- a -O(CH₂)ₙO- group with **n** representing an integer between 1 and 5, in particular between 2 and 3,
where:
▪ **R₁₆** to **R₄₈** represent, independently of each other, a hydrogen atom, a (C₁-C₆)alkyl, aryl, aryl-(C₁-C₆)alkyl, heterocycle or heterocycle-(C₁-C₆)alkyl group,
the aryl ring of these groups being optionally substituted with one or several groups chosen from a halogen atom and a (C₁-C₆)alkyl group, and
the heterocycle ring of these groups being optionally substituted with one or several groups chosen from a halogen atom, a (C₁-C₆)alkyl group, and oxo(=O), or
▪ **R₂₂** and **R₂₃, R₃₁** and **R₃₂, R₃₅** and **R₃₆, R₃₈** and **R₃₉, R₄₃** and **R₄₄,** and/or **R₄₇** and **R₄₈** form together, with the nitrogen atom carrying them, a nitrogen-containing heterocycle optionally substituted with one or several groups chosen from a halogen atom, a (C₁-C₆)alkyl group, and oxo(=O).

7. The compound according to any one of claims 1 to 6, **characterised in that R₉** and **R₁₀** represent, independently of one another:
- a hydrogen or halogen atom,
- a CO₂**R₅₄** group with **R₅₄** representing a hydrogen atom or a (C₁-C₆)alkyl group, in particular a hydrogen atom,
- a CON**R₅₅R₅₆** group with **R₅₅** and **R₅₆** forming with the nitrogen atom carrying them a nitrogen-containing 5-, 6- or 7-membered heterocycle, comprising 1 or 2 heteroatoms chosen from N and O of which at least one nitrogen atom, more preferably saturated, optionally substituted with one or several substituents chosen from a halogen atom, (C₁-C₆)alkyl, oxo (=O), O**R₂₀,** N**R₂₂R₂₃,** CO₂**R₂₅,**C(O)N**R₃₁R₃₂,** N**R₃₃**CO₂**R₃₄,** OC(O)N**R₃₅R₃₆,** N**R₃₇**CON**R₃₈R₃₉** and OCO₂**R₄₀;** in particular chosen from a halogen atom, (C₁-C₆)alkyl, oxo (=O), O**R₂₀,** N**R₂₂R₂₃,** CO₂**R₂₅** and C(O)N**R₃₁R₃₂,**
- a -**Z₁**-(CH₂)**ₘ**-**R₄₉** group wherein **Z₁** represents a single bond, CH₂-CH₂, CH=CH, C=C, O, S or N**R₅₀,** in particular a single bond, CH₂-CH₂, C=C, O or N**R_{50;} m** represents an integer between 1 and 6, in particular between 1 and 4; **R₅₀** represents a hydrogen atom or a (C₁-C₆)alkyl group; and **R₄₉** represents a halogen atom, O**R₂₀,** N**R₂₂R₂₃,** C(O)**R₂₄,** CO₂**R₂₅,** OC(O)**R₂₆,** N**R₂₉**C(O)**R₃₀,** C(O)N**R₃₁R₃₂,** N**R₃₃**CO₂**R₃₄,** OC(O)N**R₃₅R₃₆,** N**R₃₇**CON**R₃₈R₃₉** or OCO₂**R₄₀;** in particular O**R₂₀,** N**R₂₂R₂₃,** CO₂**R₂₅,** C(O)N**R₃₁R₃₂,** N**R₃₃**CO₂**R₃₄,** OC(O)N**R₃₅R₃₆,** N**R₃₇**CON**R₃₈R₃₉** or OCO₂**R₄₀;** in particular N**R₂₂R₂₃,** N**R₃₃**CO₂**R₃₄,** or N**R₃₇**CON**R₃₈R₃₉,** or
- a 5-, 6- or 7-membered heterocycle, in particular 6- or 7-membered, comprising 1 or 2 heteroatoms chosen from N and O and comprising advantageously at least one nitrogen atom, more preferably saturated or aromatic, optionally substituted with one or several substituents chosen from:
• a halogen atom,
• a (C₁-C₆)alkyl group optionally substituted with one or several groups chosen from a halogen atom, O**R₁₆,** N**R₁₈R₁₉,** a C₃ to C₆ monocyclic carbocycle and monocyclic 3- to 6-membered heterocycle comprising 1 or 2 heteroatoms chosen from N and O,
• the oxo (=O), O**R₂₀,** N**R₂₂R₂₃,** C(O)**R₂₄,** CO₂**R₂₅,**OC(O)**R₂₆,**N**R₂₉**C(O)**R₃₀**, C(O)N**R₃₁R₃₂,** N**R₃₃**CO₂**R₃₄,** OC(O)N**R₃₅R₃₆,** N**R₃₇**CON**R₃₈R₃₉** or OCO₂**R₄₀** groups; and more particularly the oxo(=O), O**R₂₀,** N**R₂₂R₂₃,** CO₂**R₂₅,** C(O)N**R₃₁R₃₂,** N**R₃₃**CO₂**R₃₄,** OC(O)N**R₃₅R₃₆,** N**R₃₇**CON**R₃₈R₃₉** or OCO₂**R₄₀** groups,
• a C₃ to C₆ carbocycle optionally substituted with one or several groups chosen from a halogen atom, a (C₁-C₆)alkyl group, oxo(=O), O**R₄₁** and N**R₄₃R₄₄,**
• a 3- to 6-membered heterocycle, comprising 1 or 2 heteroatoms chosen from N and O, in particular saturated or unsaturated, more preferably saturated, optionally substituted with one or several groups chosen from a halogen atom, a (C₁-C₆)alkyl group, oxo (=O), O**R₄₅** and N**R₄₇R₄₈,** and
• a -O(CH₂)ₙO- group with n representing an integer equal to 2 or 3,
where:
▪ **R₁₆, R₁₈** to **R₂₀, R₂₂** to **R₂₆, R₂₉** to **R₄₀, R₄₅** and **R₄₇** to **R₄₈** represent, independently of each other, a hydrogen atom, a (C₁-C₆)alkyl, aryl, or aryl-(C₁-C₆)alkyl group, in particular a hydrogen atom or a (C₁-C₆)alkyl group,
the aryl ring of these groups being a phenyl group and being optionally substituted with one or several groups chosen from a halogen atom and a (C₁-C₆)alkyl group, or
▪ **R₂₂** and **R₂₃, R₃₁** and **R₃₂, R₃₅** and **R₃₆, R₃₈** and **R₃₉, R₄₃** and **R₄₄,** and/or **R₄₇** and **R₄₈** form together, with the nitrogen atom carrying them, a nitrogen-containing 5- or 6-member heterocycle, more preferably saturated, optionally comprising 1 heteroatom in addition to the nitrogen atom chosen from N and O, such as a piperazine, piperidine, morpholine or pyrrolidine cycle, the heterocycle being optionally substituted with one or several groups chosen from a halogen atom, a (C₁-C₆)alkyl group, and oxo(=O).

8. The compound according to claim 1, **characterised in that** it is chosen from the following compounds: 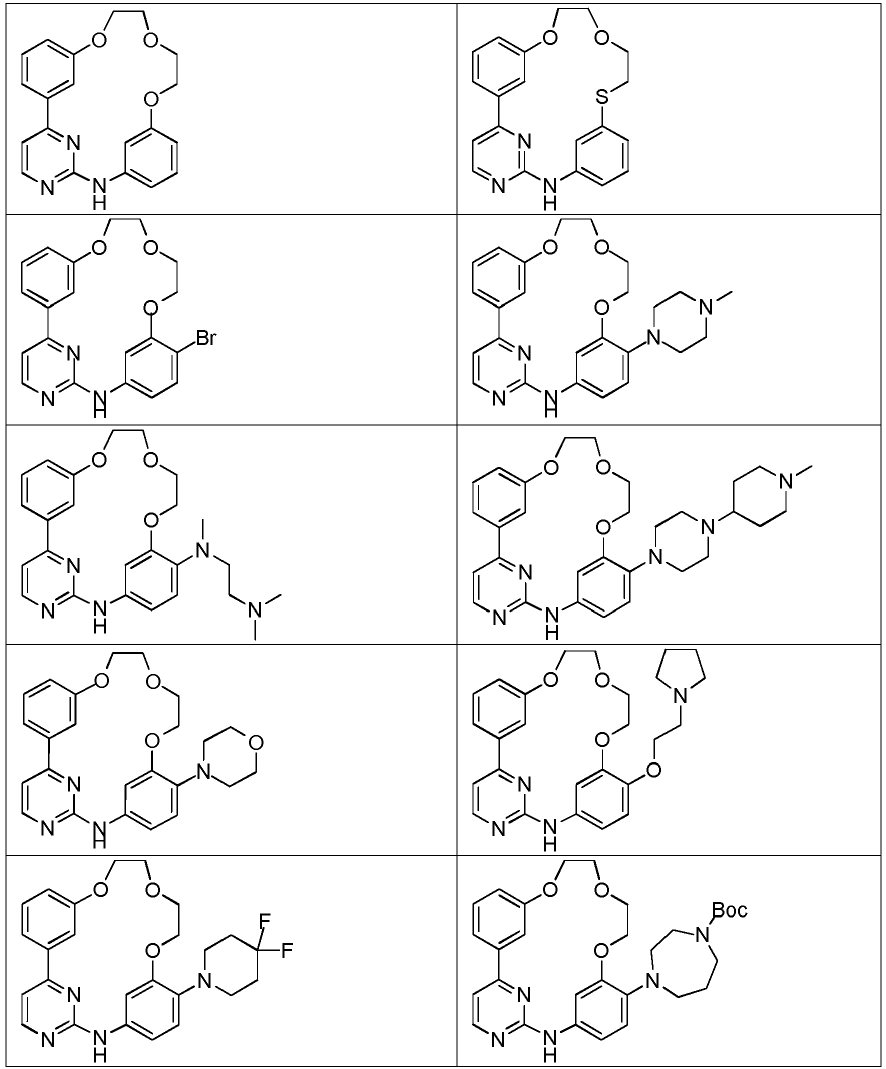 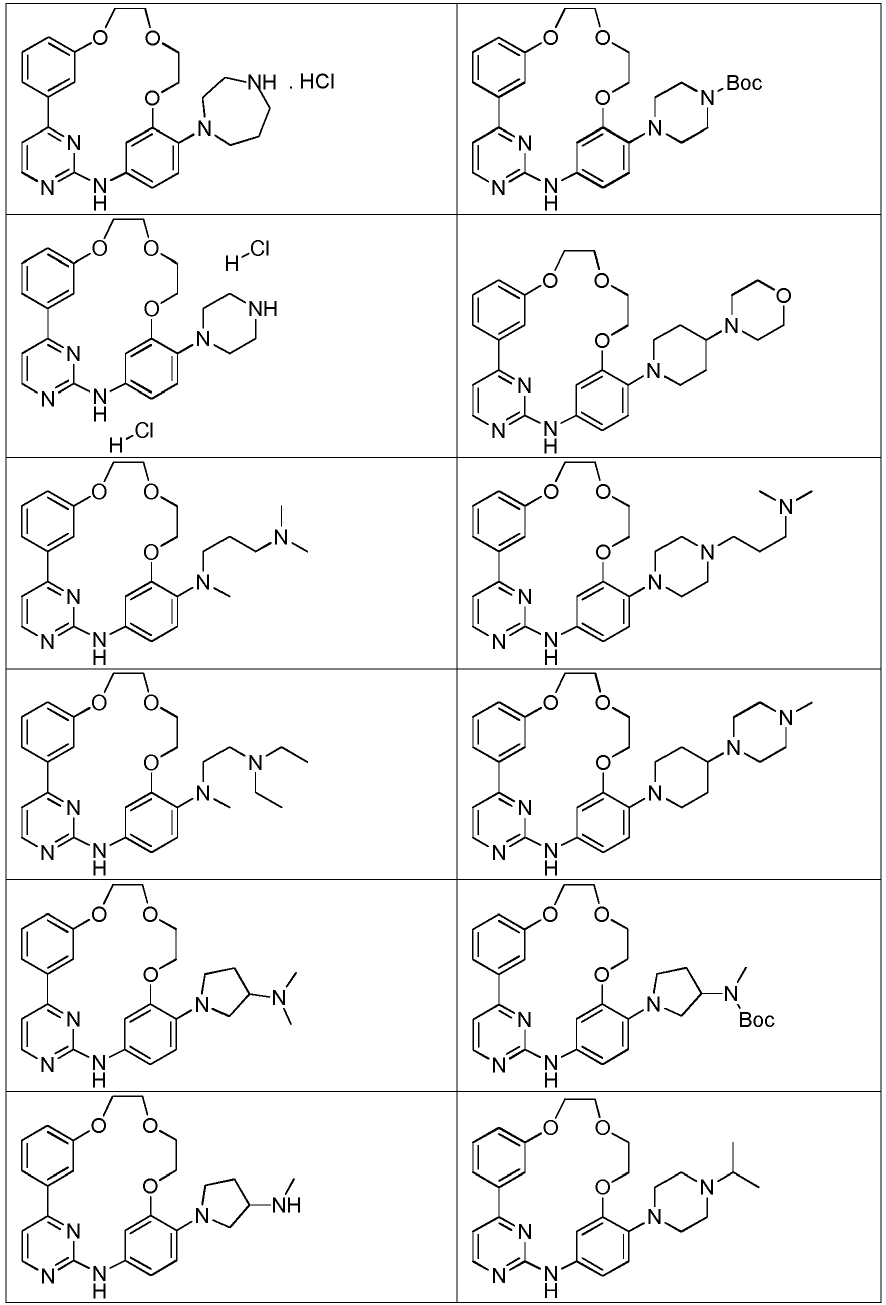 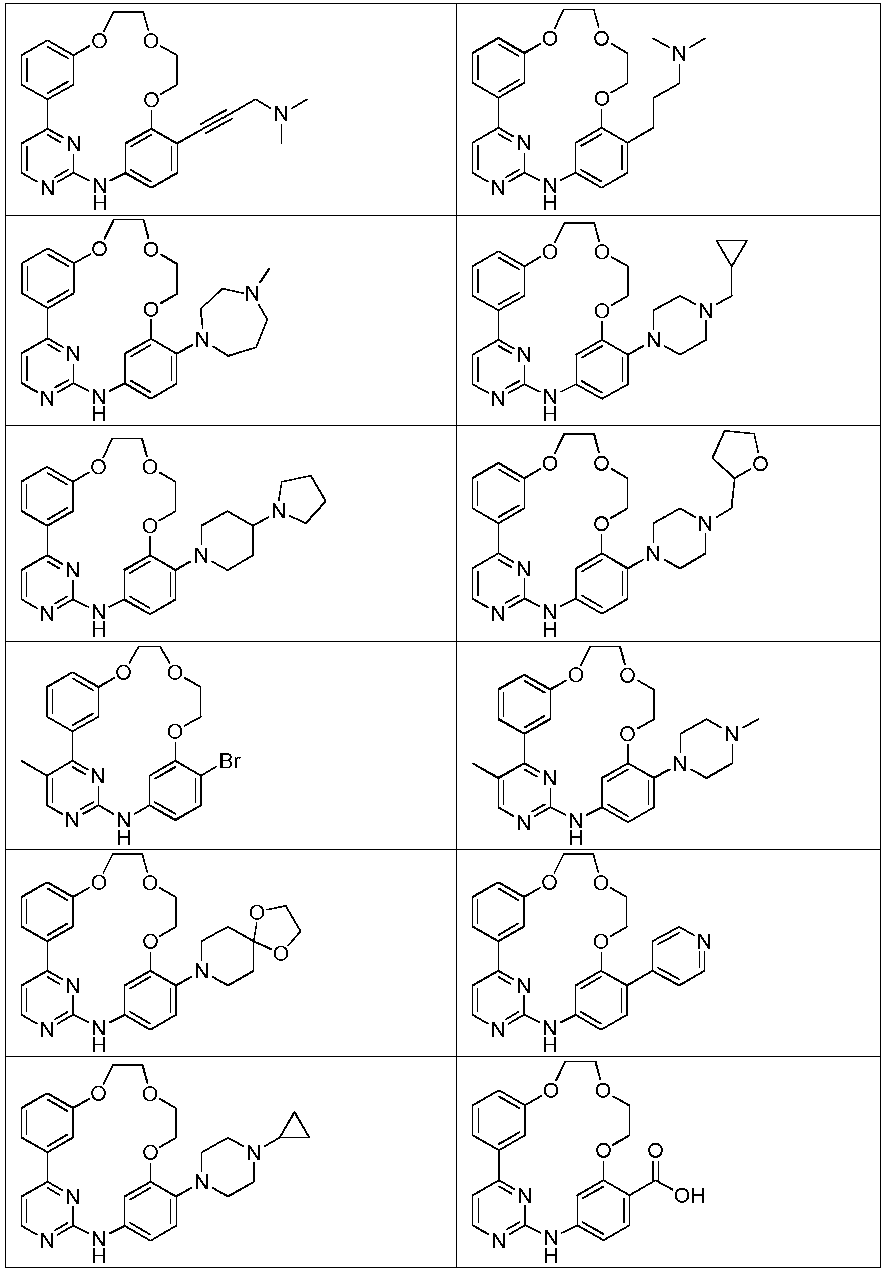 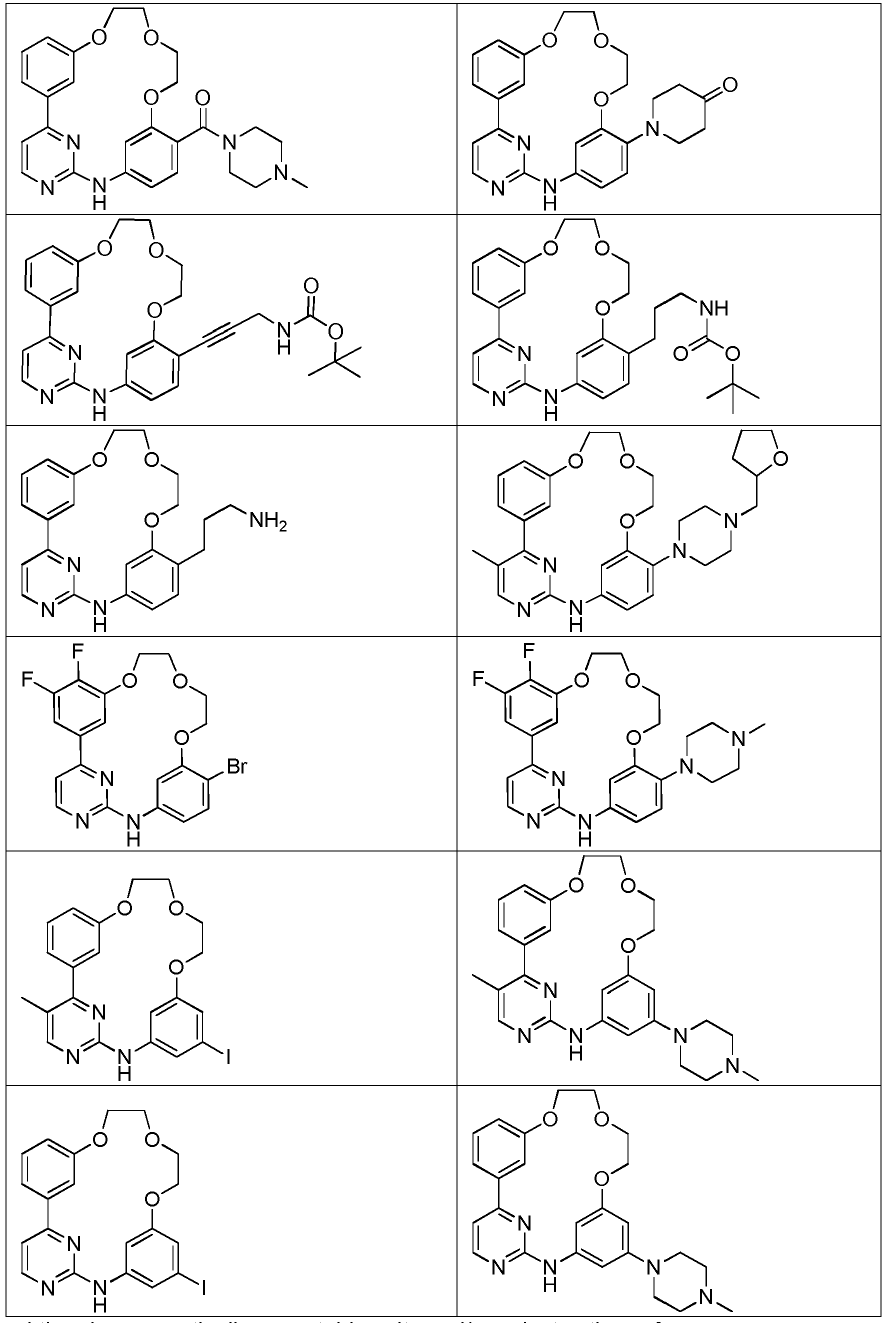 and the pharmaceutically acceptable salts and/or solvates thereof.

9. The compound according to any one of claims 1 to 8 for use as a drug.

10. The compound according to any one of claims 1 to 8 for use in the treatment of cancer.

11. A pharmaceutical composition comprising at least one compound according to any one of claims 1 to 8 and at least one pharmaceutically acceptable excipient.

12. A method for preparing a compound of formula **(I)** according to any one of claims 1 to 8 comprising the coupling reaction between:
a compound of the following formula **(II)**: 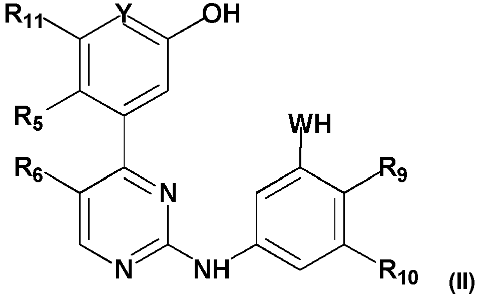 for which **W, Y, R₅, R₆,** R₉, **R₁₀** and **R₁₁** are as defined in claim 1, and a compound of the following formula (III): 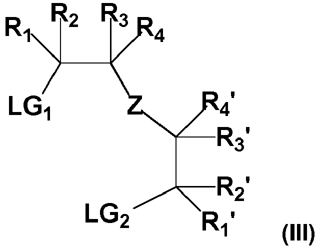 for which **Z, R₁, R₂**, **R₃**, **R₄, R₁', R₂', R₃'** and **R₄'** are as defined in claim 1, and **LG₁** and **LG₂** each represent, independently of one another, a leaving group.

13. A method for preparing a compound according to any one of claims 1 to 8 comprising the ring-closure reaction of a compound of the following formula **(VIa)** or **(VIb)**: 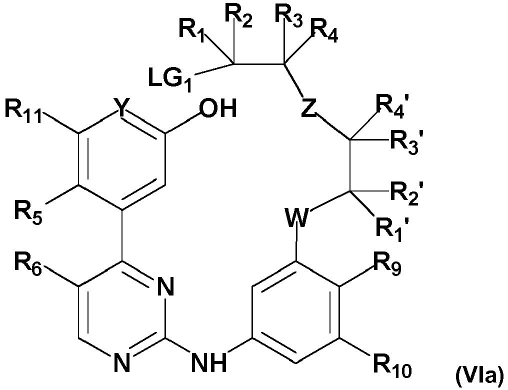 or 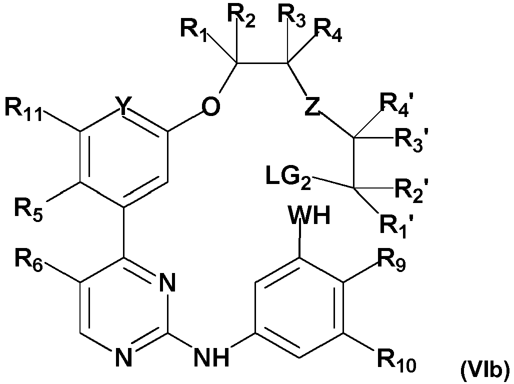 for which **W, Y, Z, R₁** to **R₆, R₉** to **R₁₁** and **R₁'** to **R₄'** are as defined in claim 1, and **LG₁** and **LG₂** each represent, independently of one another, a leaving group, such as a halogen atom and in particular a bromine atom.

14. A method for preparing a compound according to any one of claims 1 to 8, for which **R₉** and/or **R₁₀** represents an optionally substituted (C₁-C₆)alkoxy group, optionally substituted (C₁-C₆)thioalkoxy or N**R₁₄R₁₅** or an optionally substituted heterocycle comprising a heteroatom directly bonded to the phenyl ring, comprising the coupling between a compound of the following formula **(IVa)** or **(IVb)**: 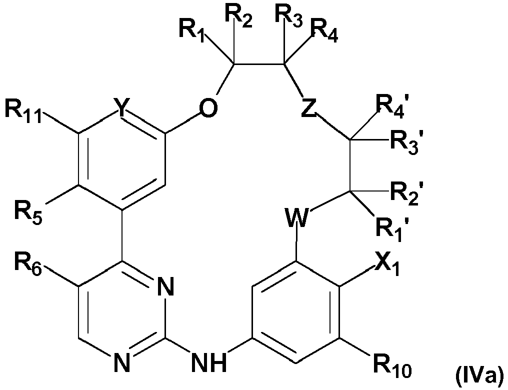 or 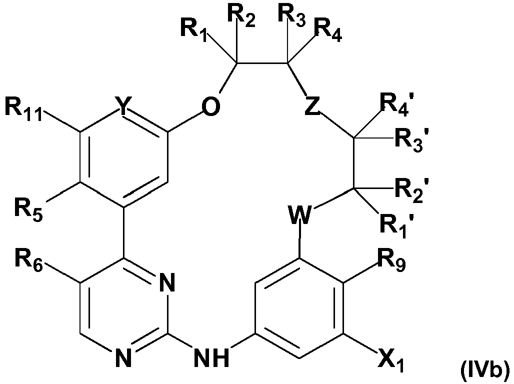 for which **W, Y, Z, R₁** to **R₆, R₉** to **R₁₁** and **R₁'** to **R₄'** are as defined in claim 1 and **X₁** represents a halogen atom such as Br, Cl or I,
and respectively a compound of formula **R₉**H or **R₁₀**H for which **R₉** and **R₁₀** are as defined hereinabove.

15. A method for preparing a compound according to any one of claims 1 to 8, for which **R₉** and/or **R₁₀** represents an optionally substituted (C₁-C₆)alkyl, optionally substituted (C₂-C₆)alkenyl or optionally substituted (C₂-C₆)alkynyl, an optionally substituted carbocycle or an optionally substituted heterocycle group bonded to the phenyl ring by means of a carbon atom, comprising the coupling between a compound of the following formula **(Va)** or **(Vb)**: 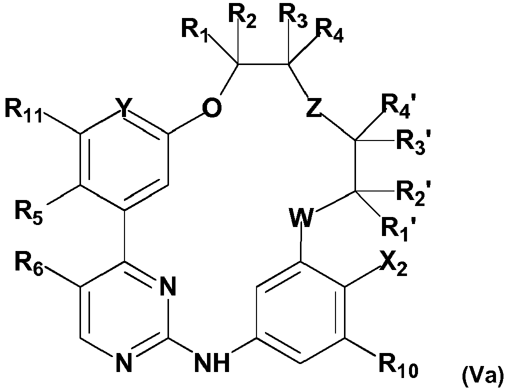 or 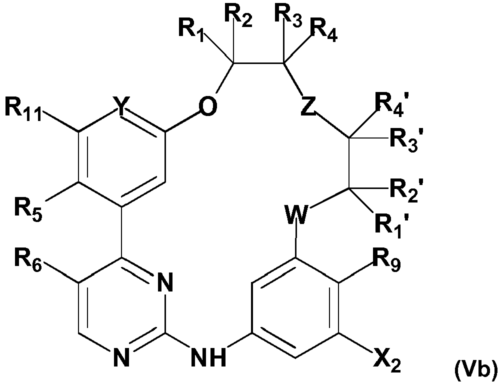 for which **W, Y, Z, R₁** to **R₆, R₉** to **R₁₁** and **R₁'** to **R₄'** are as defined in claim 1 and **X₂** represents Br, Cl, I or OTf (OSO₂CF₃),
and respectively a compound of formula **R₉-**B**R₅₂R₅₃** or **R₁₀-**B**R₅₂R₅₃** for which **R₉** and **R₁₀** are as defined hereinabove and **R₅₂** and **R₅₃** represent, independently of one another, a OH, (C₁-C₆)alkyl or (C₁-C₆)alkoxy group or **R₅₂** and **R₅₃** form together a -**X₃**- or -O-**X₃**-O-chain for which **X₃** represents a divalent hydrocarbon group comprising 2 to 15, in particular 2 to 10, carbon atoms.

16. A method for preparing a compound according to claim 7, for which **R₉** and/or **R₁₀** represents -**Z₁**-(CH₂)**ₘ**-**R₄₉** with **Z₁** representing CH₂-CH₂, CH=CH or C=C, comprising the following steps:
(1) Sonogashira coupling between a compound of formula **(Va)** or **(Vb)** as defined in claim 15,
and a compound of formula HC=C-(CH₂)**ₘ**-**R₄₉** for which **m** and **R₄₉** are as defined in claim 7,
in order to yield a compound of formula **(I)** for which **R₉** or **R₁₀** represents -C=C-(CH₂)**ₘ**-**R₄₉,** and
(2) optionally reduction of the alkyne function of the compound of formula **(I)** obtained in the preceding step in order to yield a compound of formula **(I)** for which **R₉** or **R₁₀** represents -CH=CH-(CH₂)**ₘ**-**R₄₉** or -(CH₂)ₘ₊₂-**R₄₉.**

17. A method for preparing a compound according to any one of claims 1 to 8, for which **R₉** and/or **R₁₀** represents a CO₂**R₅₄** or CON**R₅₅R₅₆** group, which comprises at least one of the following steps:
(a) in order to yield a compound of formula **(I)** for which **R₉** and/or **R₁₀** represents a CO₂H group, the reaction of a compound of formula **(I)** for which **R₉** and/or **R₁₀** represents a halogen atom with CO₂;
(b) in order to yield a compound of formula **(I)** for which **R₉** and/or **R₁₀** represents a CO₂**R₅₄** group with **R₅₄** ≠ H, the substitution reaction of a compound of formula **(I)** for which **R₉** and/or **R₁₀** represents a CO₂H group, optionally obtained according to the step (a), optionally under an activated form, with an alcohol of formula **R₅₄**OH;
(c) in order to yield a compound of formula **(I)** for which **R₉** and/or **R₁₀** represents a CON**R₅₅R₅₆** group, the substitution reaction of a compound of formula **(I)** for which **R₉** and/or **R₁₀** represents a CO₂H group, optionally obtained according to the step (a), optionally in an activated form, with an amine of formula HN**R₅₅R₅₆.**
